# EUROPEAN PATENT APPLICATION

(11) **EP 1 803 810 A1**
(43) Date of publication of application: **04.07.2007**
(21) Application number: 06026259.9
(22) Date of filing: 23.02.2001
(51) Int. Cl.: C12N 9/64, C12N 15/57, A61K 38/16, A61P 35/00, A61P 37/00, A61P 27/00, A61P 17/02, C07K 14/705

(54) **Integrin antagonists**

(30) Priority: 25.02.2000 US 184865 P
(62) Divisional of application: 01920133.4
(71) Applicant: IMMUNEX CORPORATION, Thousand Oaks, CA 91320-1799 (US)
(72) Inventor: Fanslow, William C., III, Normandy Park, WA 98166 (US); Cerretti, Douglas Pat, Seattle, WA 98133 (US); Poindexter, Kurt Matthew, Seattle, WA 98103 (US); Black, Roy Alvin, Seatlle, WA 98115 (US)
(74) Representative: Lee, Nicholas John

(57) **Abstract**

The present invention provides methods and compositions for inhibiting the biological activity of integrins, for inhibiting endothelial cell migration, and for inhibiting angiogenesis. In particular, the invention provides compositions comprising ADAM disintegrin domains and methods for using said compositions. In preferred embodiments the methods and compositions of the invention are used to inhibit angiogenesis and to treat diseases or conditions mediated by angiogenesis.

## Description

### CROSS REFERENCE TO RELATED APPLICATIONS

This application claims the benefit of pending U.S. provisional application Serial No. 60/184,865, filed 25 February 2000, the contents of which are incorporated herein by reference.

### FIELD OF THE INVENTION

This invention relates to methods and compositions that are useful for antagonizing the interaction between integrins and their ligands. In particular, the invention relates to the use of ADAM disintegrin domains for antagonizing the interaction between integrins and their ligands.

### BACKGROUND OF THE INVENTION

### A. Integrins and Disintegrins

Integrins are a family of cell surface proteins that mediate adhesion between cells (cell-cell adhesion) and between cells and extracellular matrix proteins (cell-ECM adhesion). Integrins are heterodimeric structures composed of noncovalently bound α and β subunits. In humans, at least fifteen different α subunits and eight different β subunits combine to form integrins with diverse biological activities and ligand specificities. Integrins play important roles in biological processes including embryonic development, platelet aggregation, immune reactions, tissue repair and remodeling, bone resorption, and tumor invasion and metastasis. Integrins are, therefore, important targets for therapeutic intervention in human disease.

The disintegrins are a family of low molecular weight, soluble, cysteine-rich peptides which have been isolated from snake venom (reviewed in Niewiarowski et al., Seminars in Hematology 31(4):289, 1994). The snake venom disintegrins typically contain an RGD (Arg-Gly-Asp, SEQ ID NO:19) motif. The RGD motif is recognized by many integrins, and is present in several integrin ligands including fibronectin, vitronectin, and von Willebrand factor. Disintegrins disrupt normal adhesion processes by inhibiting the binding of cell surface integrins to their ligands.

Disintegrin-like domains have been identified in cellular proteins from both invertebrates and vertebrates (see, e.g., Westcamp and Blobel, Proc. Natl. Acad. Sci. USA 91:2748, 1994; Wolfsberg et al., Dev. Biol. 169:378, 1995; Alfandari et al., Dev. Biol. 182:314, 1997), including the ADAM family of transmembrane proteins.

### B. ADAMs

The ADAMs, which have also been called MDCs, are a family of type I transmembrane cysteine-rich glycoproteins (Weskamp et al., Proc. Natl. Acad. Sci. USA, 91:2748, 1994; Wolfsberg et al., Dev. Biol. 169:378, 1995). The multidomain structure of the ADAMs typically includes an amino-terminal metalloprotease domain, a disintegrin domain, a cysteine-rich region (the region between the disintegrin domain and the transmembrane domain), a transmembrane region, and a cytoplasmic domain. At least 30 ADAM family members have been identified, in a variety of animal species. The structure of the ADAMs suggests that they may be involved in a variety of biological processes, including cell adhesion, cell fusion, signal transduction, and proteolysis. Members of the ADAM family have, in fact, been shown to play roles in sperm-egg binding and fusion, myotube formation, neurogenesis, and proteolysis.

ADAM-15, also called MDC-15 or metargidin, is the only ADAM identified to date which contains an RGD motif within its disintegrin domain. Zhang et al. (J. Biol. Chem. 273(13):7345, 1998) have reported that the isolated disintegrin domain of ADAM-15, expressed in E. coli as a glutathione S-transferase fusion protein, specifically interacts with αᵥβ₃ integrin and that the interaction is mediated by the RGD tripeptide sequence. The recombinant fusion protein did not interact with other integrins tested, including α_{IIb}β₃ and α₅β₁. Nath et al. (J. Cell Science 112:579, 1999) have reported that the entire ADAM-15 extracellular domain, expressed as an Fc fusion protein in COS cells, interacts with αᵥβ₃ and α₅β₁ integrins on hematopoietic cells and that the interaction is mediated by the RGD tripeptide sequence. Zhang et al. and Nath et al. commented that the RGD-dependent interaction between ADAM-15 and αᵥβ₃ integrin suggests a role in processes such as malignancy and angiogenesis.

### C. Angiogenesis

Angiogenesis, the generation of new blood vessels, is a spatially and temporally regulated process in which endothelial and smooth muscle cells proliferate, migrate, and assemble into tubes, in response to endogenous positive and negative regulatory molecules. Angiogenesis plays important roles in both normal and pathological physiology.

Under normal physiological conditions, angiogenesis is involved in fetal and embryonic development, wound healing, organ regeneration, and female reproductive remodeling processes including formation of the endometrium, corpus luteum, and placenta. Angiogenesis is stringently regulated under normal conditions, especially in adult animals, and perturbation of the regulatory controls can lead to pathological angiogenesis.

Pathological angiogenesis has been implicated in the manifestation and/or progression of inflammatory diseases, certain eye disorders, and cancer. In particular, several lines of evidence support the concept that angiogenesis is essential for the growth and persistence of solid tumors and their metastases (see, e.g., Folkman, N. Engl. J. Med. 285:1182, 1971; Folkman et al., Nature 339:58, 1989; Kim et al., Nature 362:841, 1993; Hori et al., Cancer Res., 51:6180, 1991; Zetter, Annu. Rev. Med. 49:407, 1998). The formation of new blood vessels provides a growing tumor with oxygen, nutrients, waste removal, and a conduit by which invasive cells can enter the circulatory system and establish distant metastases. Various classes of angiogenesis inhibitors are presently being developed and tested for the prevention (e.g., treatment of premalignant conditions), intervention (e.g., treatment of small tumors), and regression (e.g., treatment of large tumors) of cancers (see, e.g., Bergers et al., Science 284:808, 1999) and other forms of pathological angiogenesis. Because many steps in the angiogenic process, including endothelial cell migration, proliferation, and morphogenesis require vascular cell adhesion, certain integrin antagonists have been tested as anti-angiogenic agents.

Several integrins are expressed on the surface of cultured endothelial and smooth muscle cells, including αᵥβ₃ integrin. The αᵥβ₃ integrin is an endothelial cell receptor for von Willebrand factor, fibrin, fibrinogen, and fibronectin, and a marker of angiogenic vascular tissue. Brooks et al. have reported that monoclonal antibodies to αᵥβ₃ integrin, as well as cyclic peptide inhibitors, disrupt angiogenesis and that αᵥβ₃ antibodies promote tumor regression (Science 264:569, 1994; Cell 79:1157, 1994). These results suggest that αᵥβ₃ integrin is a useful therapeutic target for diseases characterized by pathological angiogenesis.

There is great need for additional compositions and methods of antagonizing the interaction between integrins and their ligands. In particular, there is great need for additional compositions and methods of inhibiting angiogenesis for the prevention, abrogation, and mitigation of disease processes that are dependent upon pathological angiogenesis.

### SUMMARY OF THE INVENTION

The present invention is based upon the discovery that ADAM disintegrin domains are useful for inhibiting the biological activity of integrins and for inhibiting endothelial cell migration and angiogenesis, including the unexpected discovery that these inhibitory activities reside in ADAM disintegrin domains that lack an RGD motif.

The invention is directed to methods of antagonizing the binding of an integrin to its ligands, and thereby inhibiting the biological activity of the integrin, comprising contacting the integrin with an effective amount of an ADAM disintegrin domain polypeptide. The invention is further directed to methods of inhibiting endothelial cell migration and methods of inhibiting angiogenesis comprising administering an effective amount of an ADAM disintegrin domain polypeptide. In some embodiments the ADAM disintegrin domain polypeptide is in the form of a multimer, preferably a leucine zipper multimer or Fc polypeptide. In some embodiments the ADAM disintegrin domain is from a human ADAM, and preferably from ADAM-8, ADAM-9, ADAM-10, ADAM-15, ADAM-17, ADAM-20, ADAM-21, ADAM-22, ADAM-23, or ADAM-29. The ADAM disintegrin domain is preferably produced in a recombinant cell, and is preferably present in a composition comprising a pharmaceutically acceptable carrier.

In some preferred embodiments the ADAM disintegrin domain polypeptide comprises an amino acid sequence selected from the group consisting of: amino acids 23-264 of SEQ ID NO:2, amino acids 23-303 of SEQ ID NO:4, amino acids 23-235 of SEQ ID NO:6. amino acids 23-292 of SEQ ID NO:8, amino acids 23-216 of SEQ ID NO:10, amino acids 23-305 of SEQ ID NO:12, amino acids 23-293 of SEQ ID NO: 14, amino acids 23-312 of SEQ ID NO: 16, amino acids 23-310 of SEQ ID NO: 18, and amino acids 23-298 of SEQ ID NO:22. In some more preferred embodiments the ADAM disintegrin domain polypeptide comprises an amino acid sequence selected from the group consisting of: amino acids 34-91 of SEQ ID NO:2, amino acids 34-92 of SEQ ID NO:4, amino acids 34-99 of SEQ ID NO:6, amino acids 34-92 of SEQ ID NO:8, amino acids 34-93 of SEQ ID NO: 10, amino acids 34-91 of SEQ ID NO:12, amino acids 34-91 of SEQ ID NO:14, amino acids 34-92 of SEQ ID NO: 16, amino acids 34-91 of SEQ ID NO: 18, and amino acids 34-91 of SEQ ID NO:22. In some most preferred embodiments the ADAM disintegrin domain polypeptide comprises an amino acid sequence selected from the group consisting of: amino acids 78-91 of SEQ ID NO:2, amino acids 79-92 of SEQ ID NO:4, amino acids 87-99 of SEQ ID NO:6, amino acids 79-92 of SEQ ID NO:8, amino acids 79-93 of SEQ ID NO:10, amino acids 78-91 of SEQ ID NO:12, amino acids 78-91 of SEQ ID NO:14, amino acids 79-92 of SEQ ID NO: 16, amino acids 78-91 of SEQ ID NO:18, and amino acids 78-91 of SEQ ID NO:22.

In some embodiments a therapeutically effective amount of the ADAM disintegrin domain is administered to a mammal in need of such treatment. In preferred embodiments the mammal is afflicted with a condition mediated by angiogenesis, an ocular disorder, malignant or metastatic condition, inflammatory disease, osteoporosis and other conditions mediated by accelerated bone resorption, restenosis, inappropriate platelet activation, recruitment, or aggregation, thrombosis, or a condition requiring tissue repair or wound healing. The ADAM disintegrin domain is, in some embodiments, administered in combination with radiation therapy and/or in combination with one or more additional therapeutic agents.

The invention also encompasses methods for identifying compounds that modulate integrin biological activity, that modulate the interaction between an integrin and an ADAM disintegrin domain, that inhibit endothelial cell migration, or that inhibit angiogenesis, comprising combining a test compound with an integrin or with endothelial cells and with an ADAM disintegrin domain polypeptide that binds to the integrin or endothelial cells and determining whether the test compound alters the binding of the ADAM disintegrin domain polypeptide to the integrin or endothelial cells.

These and other aspects of the present invention will become evident upon reference to the following detailed description, examples, and claims.

### DETAILED DESCRIPTION OF THE INVENTION

### A. Abbreviations and Terminology Used in the Specification

"4-1BB" and "4-1BB ligand" (4-1BB-L) are polypeptides described, inter alia, in U.S. Patent No. 5,674,704, including soluble forms thereof.

"ADAMs" are a family of transmembrane glycoproteins having disintegrin and metalloproteinase domains, also called MDC, metalloprotease/disintegrin/cysteine-rich proteins.

"Dis" is a disintegrin domain; "ADAMdis" is an ADAM disintegrin domain.

"CD40 ligand" (CD40L) is a polypeptide described, inter alia, in U.S. Patent No. 5,716,805, including soluble forms thereof.

"CD148" is a protein tyrosine phosphatase, also called DEP-1, ECRTP, and PTPRJ. CD 148 binding proteins are described in Daniel et al., PCT Publication No. WO 00/15258, 23 March 2000.

"DMEM" is Dulbecco's Modified Eagle Medium.

"FACS" is fluorescence activated cell sorting.

"Flt3L" is Flt3 ligand, a polypeptide described, inter alia, in U.S. Patent No. 5,554,512, including soluble forms thereof.

"HRMEC" are human renal microvascular endothelial cells.

"HMVEC-d" are human dermal microvascular endothelial cells.

"mAb" is a monoclonal antibody.

"MDC" is a family of cysteine-rich proteins having metalloprotease and disintegrin domains, also called ADAM.

"Nectin-3" is a cell adhesion molecule in the nectin family (which is described, inter alia, in Satoh-Horikawa et al., J. Biol. Chem. 275(14):10291, 2000). The GenBank accession numbers of human nectin-3 nucleic acid and polypeptide sequences are AF282874 and AAF97597 respectively (Reymond et al., 2000).

"PMA" is phorbol-12-myristate-13-acetate.

"Tek," which has also been called Tie2 and ork, is an receptor tyrosine kinase (RTK) that is predominantly expressed in vascular endothelium. The molecular cloning of human Tek (ork) has been described by Ziegler, U.S. Patent No. 5,447,860. "Tek antagonists" are described, inter alia, in Cerretti et al., PCT Publication No. WO 00/75323, 14 December 2000.

"TNF" is tumor necrosis factor. "TNFR" is a tumor necrosis factor receptor, including soluble forms thereof. "TNFR/Fc" is a tumor necrosis factor receptor-Fc fusion polypeptide.

"TRAIL" is TNF-related apoptosis-inducing ligand, a type II transmembrane polypeptide in the TNF family described, inter alia, in U.S. Patent No. 5,763,223, including soluble forms thereof.

"TWEAK" is TNF-weak effector of apoptosis, a type II transmembrane polypeptide in the TNF family described, inter alia, in Chicheportiche et al., J. Biol. Chem., 272(51):32401, 1997, including soluble forms thereof. "TWEAK-R" is the "TWEAK receptor," which is described, inter alia, in U.S. Serial Numbers 60/172,878 and 60/203,347 and Feng et al., Am. J. Pathol. 156(4):1253, 2000, including soluble forms thereof. TWEAK-R/Fc is a TWEAK receptor-Fc fusion polypeptide.

"VEGF" is vascular endothelial growth factor, also known as VPF or vascular permeability factor.

### B. ADAM Polypeptides and ADAM Disintegrin Domain Polypeptides

At least thirty ADAMs have been described. Table 1 provides reference information for selected human ADAMs.

ADAM disintegrin domains show sequence homology to the snake venom disintegrins, and are characterized by a framework of cysteines. For example, a typical disintegrin sequence comprises a framework such as:
CDCGX₃₋₅CX₃₋₆CCX₂₋₄CX₇CX₄₋₆CCX₂₋₄CX₈CX₅₋₇CX₃₋₅C (SEQ ID NO:20)
The sequences of several ADAM disintegrin domains are shown in Table 2 and in the Sequence Listing.

The present invention encompasses the use of various forms of ADAM disintegrin domains that retain at least one activity selected from the group consisting of integrin binding activity, inhibition of endothelial cell migration, and inhibition of angiogenesis. The term "ADAM disintegrin domain polypeptide" is intended to encompass polypeptides containing all or part of a native ADAM disintegrin domain, with or without other ADAM domains (such as the cysteine-rich region), as well as related forms including, but not limited to: (a) fragments, (b) variants, (c) derivatives, (d) fusion polypeptides, and (e) multimeric forms (multimers). The ability of these related forms to inhibit integrin binding, endothelial cell migration, and/or inhibition of angiogenesis may be determined in vitro or in vivo by using methods such as those exemplified below or by using other assays known in the art.

**Table 1**

| Selected Members of the ADAM Family | | | |
|---|---|---|---|
| ADAM | Other Names | GenBank Accession Number (Human) | Published Description |
| ADAM-8 | MS2, CD156 | D26579 | Genomics 41(1):56, 1997 |
| ADAM-9 | MDC9, meltrin gamma | U41766 | J. Cell. Biol. 132(4):717, 1996 |
| ADAM-10 | MADM, kuzbanian, reprolysin | AF009615 | J. Biol. Chem. 272(39):24588, 1997 |
| ADAM-15 | Metargidin, MDC15 | U46005 | J. Biol. Chem. 271(9):4593, 1996 |
| ADAM-17 | TACE, cSVP | U86755 | WO 96/41624 |
| ADAM-20 | SVPH1-26 | AF029899 | WO 99/23228 |
| ADAM-21 | SVPH1-8 | AF029900 | WO 99/36549 |
| ADAM-22 | SVPH3-13, MDC2 | AB009671 | WO 99/41388 |
| ADAM-23 | SVPH3-17, MDC3 | AB009672 | WO 99/41388 |
| ADAM-29 | SVPH1 | AF171929 | Biochem. Biophys. Res. Commun. 263:810, 1999 |

The term "variant" includes polypeptides that are substantially homologous to native ADAM disintegrin domains, but which have an amino acid sequence different from that of a native ADAM disintegrin domain because of one or more deletions, insertions or substitutions. Particular embodiments include, but are not limited to, ADAM disintegrin domain polypeptides that comprise from one to ten deletions, insertions or substitutions of amino acid residues, when compared to a native ADAM disintegrin domain sequence. Included as variants of ADAM disintegrin domain polypeptides are those variants that are naturally occurring, such as allelic forms and alternatively spliced forms, as well as variants that have been constructed by modifying the amino acid sequence of a ADAM disintegrin domain polypeptide or the nucleotide sequence of a nucleic acid encoding a ADAM disintegrin domain polypeptide.

Generally, substitutions for one or more amino acids present in the native polypeptide should be made conservatively. Examples of conservative substitutions include substitution of amino acids outside of the active domain(s), and substitution of amino acids that do not alter the secondary and/or tertiary structure of the ADAM disintegrin domain. Additional examples include substituting one aliphatic residue for another, such as Ile, Val, Leu, or Ala for one another, or substitutions of one polar residue for another, such as between Lys and Arg; Glu and Asp; or Gln and Asn, or substitutions of one aromatic residue for another, such as Phe, Trp, or Tyr for one another. Other such conservative substitutions, for example, substitutions of entire regions having similar hydrophobicity characteristics, are known in the art.

In some preferred embodiments the ADAM disintegrin domain variant is at least about 70% identical in amino acid sequence to the amino acid sequence of a native ADAM disintegrin domain; in some preferred embodiments the ADAM disintegrin domain variant is at least about 80% identical in amino acid sequence to the amino acid sequence of a native ADAM disintegrin domain. In some more preferred embodiments the ADAM disintegrin domain variant is at least about 90% identical in amino acid sequence to the amino acid sequence of a native ADAM disintegrin domain; in some more preferred embodiments the ADAM disintegrin domain variant is at least about 95% identical in amino acid sequence to the amino acid sequence of a native ADAM disintegrin domain. In some most preferred embodiments the ADAM disintegrin domain variant is at least about 98% identical in amino acid sequence to the amino acid sequence of a native ADAM disintegrin domain; in some most preferred embodiments the ADAM disintegrin domain variant is at least about 99% identical in amino acid sequence to the amino acid sequence of a native ADAM disintegrin domain.

Percent identity, in the case of both polypeptides and nucleic acids, may be determined by visual inspection. Percent identity may be determined using the alignment method of Needleman and Wunsch (J. Mol. Biol. 48:443, 1970) as revised by Smith and Waterman (Adv. Appl. Math 2:482, 1981. Preferably, percent identity is determined by using a computer program, for example, the GAP computer program version 10.x available from the Genetics Computer Group (GCG; Madison, WI, see also Devereux et al., Nucl. Acids Res. 12:387, 1984). The preferred default parameters for the GAP program include: (1) a unary comparison matrix (containing a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979 for amino acids; (2) a penalty of 30 (amino acids) or 50 (nucleotides) for each gap and an additional 1 (amino acids) or 3 (nucleotides) penalty for each symbol in each gap; (3) no penalty for end gaps; and (4) no maximum penalty for long gaps. Other programs used by one skilled in the art of sequence comparison may also be used. For fragments of ADAM disintegrin domains, the percent identity is calculated based on that portion of ADAM disintegrin domain that is present in the fragment.

When a deletion or insertion strategy is adopted, the potential effect of the deletion or insertion on biological activity (such as integrin binding activity, inhibition of endothelial cell migration, or inhibition of angiogenesis) must be considered. Subunits of the inventive polypeptides may be constructed by deleting terminal or internal residues or sequences. Additional guidance as to the types of mutations that can be made is provided by a comparison of the sequence of ADAM disintegrin domain polypeptides to polypeptides that have similar structures, as well as by performing structural analysis of the inventive polypeptides.

The term "variant" also includes ADAM disintegrin domain polypeptides that are encoded by nucleic acids capable of hybridizing under moderately stringent conditions (e.g., prewashing solution of 5 X SSC, 0.5% SDS, 1.0 mM EDTA (pH 8.0) and hybridization conditions of 50°C, 5 X SSC, overnight) or higher stringency conditions to DNA sequences encoding ADAM disintegrin domain polypeptides, and which encode polypeptides that retain at least one activity selected from the group consisting of integrin binding activity, inhibition of endothelial cell migration, and inhibition of angiogenesis. The skilled artisan can determine additional combinations of salt and temperature that constitute moderate hybridization stringency. Conditions of higher stringency include higher temperatures for hybridization and post-hybridization washes, and/or lower salt concentration.

Mutations can be introduced into nucleic acids by synthesizing oligonucleotides containing a mutant sequence, flanked by restriction sites enabling ligation to fragments of the native sequence. Following ligation, the resulting reconstructed sequence encodes a variant having the desired amino acid insertion, substitution, or deletion. Alternatively, oligonucleotide-directed site-specific mutagenesis procedures can be employed to provide an altered gene having particular codons altered according to the substitution, deletion, or insertion required. The well known polymerase chain reaction (PCR) procedure also may be employed to generate and amplify a DNA sequence encoding a desired polypeptide or fragment thereof. Oligonucleotides that define the desired termini of the DNA fragment are employed as 5' and 3' primers. The oligonucleotides may additionally contain recognition sites for restriction endonucleases to facilitate insertion of the amplified DNA fragment into an expression vector.

The present invention further encompasses the use of ADAM disintegrin domain polypeptides with or without associated native-pattern glycosylation. ADAM disintegrin domain expressed in yeast or mammalian expression systems (e.g., COS-1 or COS-7 cells) may be similar to or significantly different from a native ADAM disintegrin domain polypeptide in molecular weight and glycosylation pattern, depending upon the choice of expression system. Expression of ADAM disintegrin domain polypeptides in bacterial expression systems, such as *E. coli,* provides non-glycosylated molecules. Different host cells may also process polypeptides differentially, resulting in heterogeneous mixtures of polypeptides with variable N- or C-termini.

The primary amino acid structure of ADAM disintegrin domain polypeptides may be modified to create derivatives by forming covalent or aggregative conjugates with other chemical moieties, such as glycosyl groups, lipids, phosphate, acetyl groups and the like. Covalent derivatives of ADAM disintegrin domain polypeptides may be prepared by linking particular functional groups to ADAM disintegrin domain amino acid side chains or at the N-terminus or C-terminus of a ADAM disintegrin domain polypeptide.

Fusion polypeptides of ADAM disintegrin domains that are useful in practicing the invention include covalent or aggregative conjugates of ADAMdis or its fragments with other polypeptides, such as by synthesis in recombinant culture as N-terminal or C-terminal fusions. One class of fusion polypeptides are discussed below in connection with ADAM disintegrin oligomers. As another example, a fusion polypeptide may comprise a signal peptide (which is also variously referred to as a signal sequence, signal, leader peptide, leader sequence, or leader) at the N-terminal region or C-terminal region of an ADAM disintegrin domain polypeptide which co-translationally or post-translationally directs transfer of the polypeptide from its site of synthesis to a site inside or outside of the cell membrane or cell wall. It is particularly advantageous to fuse a signal peptide that promotes extracellular secretion to the N-terntinus of a soluble ADAMdis polypeptide. In this case, the signal peptide is typically cleaved upon secretion of the soluble polypeptide from the cell.

Secreted soluble polypeptides may be identified (and distinguished from its non-soluble membrane-bound counterparts) by separating intact cells which express the desired polypeptide from the culture medium, e.g., by centrifugation, and assaying the medium (supernatant) for the presence of the desired polypeptide. The presence of the desired polypeptide in the medium indicates that the polypeptide was secreted from the cells and thus is a soluble form of the polypeptide. Soluble polypeptides may be prepared by any of a number of conventional techniques. A DNA sequence encoding a desired soluble polypeptide may be subcloned into an expression vector for production of the polypeptide, or the desired encoding DNA fragment may be chemically synthesized.

Soluble ADAM disintegrin domain polypeptides comprise all or part of the ADAM disintegrin domain, with or without additional segments from the extracellular portion of the ADAM (such as the cysteine-rich region) but generally lack a transmembrane domain that would cause retention of the polypeptide at the cell surface. Soluble polypeptides may include part of the transmembrane domain or all or part of the cytoplasmic domain as long as the polypeptide is secreted from the cell in which it is produced. Examples of soluble ADAM disintegrin domain polypeptides are provided in the examples. In some preferred embodiments of the present invention, a multimeric form of a soluble ADAM disintegrin domain polypeptide is used to inhibit integrin binding to ligands and, hence, integrin biological activity. In some most preferred embodiments the soluble ADAM disintegrin domain polypeptide is used to inhibit endothelial cell migration and/or inhibit angiogenesis. These inhibitory activities may include both integrin-mediated and integrin-independent mechanisms.

ADAM disintegrin domain multimers are covalently-linked or non-covalently-linked multimers, including dimers, trimers, and higher multimers. Oligomers may be linked by disulfide bonds formed between cysteine residues on different ADAM disintegrin domain polypeptides. One embodiment of the invention is directed to multimers comprising multiple ADAM disintegrin domain polypeptides joined via covalent or non-covalent interactions between peptide moieties fused to the ADAM disintegrin domain polypeptides. Such peptides may be peptide linkers (spacers), or peptides that have the property of promoting multimerization. Leucine zippers and certain polypeptides derived from antibodies are among the peptides that can promote multimerization of ADAM disintegrin domain polypeptides attached thereto, as described in more detail below. In particular embodiments, the multimers comprise from two to four ADAM disintegrin domain polypeptides.

In some embodiments, a ADAM disintegrin domain multimer is prepared using polypeptides derived from immunoglobulins. Preparation of fusion proteins comprising certain heterologous polypeptides fused to various portions of antibody-derived polypeptides (including the Fc domain) has been described, e.g., by Ashkenazi et al. (Proc. Natl. Acad. Sci. USA 88:10535, 1991); Byrn et al. (Nature 344:677, 1990); and Hollenbaugh and Aruffo ("Construction of Immunoglobulin Fusion Proteins", in Current Protocols in Immunology, Suppl. 4, pages 10.19.1-10.19.11, 1992).

A preferred embodiment of the present invention is directed to an ADAM disintegrin domain (ADAMdis) dimer comprising two fusion polypeptides created by fusing an ADAM disintegrin domain to an Fc polypeptide. A gene fusion encoding the ADAMdis-Fc fusion polypeptide is inserted into an appropriate expression vector. ADAMdis-Fc fusion polypeptides are expressed in host cells transformed with the recombinant expression vector, and allowed to assemble much like antibody molecules, whereupon interchain disulfide bonds form between the Fc moieties to yield divalent soluble ADAMdis polypeptides. The term "Fc polypeptide" as used herein includes native and mutein forms of polypeptides derived from the Fc region of an antibody. Truncated forms of such polypeptides containing the hinge region that promotes dimerization are also included.

One suitable Fc polypeptide, described in PCT application WO 93/10151, is a single chain polypeptide extending from the N-terminal hinge region to the native C-terminus of the Fc region of a human IgG1 antibody. Another useful Fc polypeptide is the Fc mutein described in U.S. Patent 5,457,035 and by Baum et al., EMBO J. 13:3992, 1994. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors. Fusion polypeptides comprising Fc moieties, and multimers formed therefrom, offer an advantage of facile purification by affinity chromatography over Protein A or Protein G columns, and Fc fusion polypeptides may provide a longer in vivo half life, which is useful in therapeutic applications, than unmodified polypeptides.

In other embodiments, a soluble ADAM disintegrin domain polypeptide may be substituted for the variable portion of an antibody heavy or light chain. If fusion proteins are made with both heavy and light chains of an antibody, it is possible to form an ADAM disintegrin domain multimer with as many as four soluble ADAM disintegrin domain polypeptides.

Alternatively, the ADAM disintegrin domain multimer is a fusion polypeptide comprising multiple ADAM disintegrin domain polypeptides, with or without peptide linkers (spacers), or peptides that have the property of promoting multimerization.. Among the suitable peptide linkers are those described in U.S. Patents 4,751,180 and 4,935,233. A DNA sequence encoding a desired peptide linker may be inserted between, and in the same reading frame as, the DNA sequences encoding ADAMdis, using conventional techniques known in the art. For example, a chemically synthesized oligonucleotide encoding the linker may be ligated between sequences encoding ADAMdis. In particular embodiments, a fusion protein comprises from two to four ADAM disintegrin domain polypeptides, separated by peptide linkers.

Another method for preparing ADAM disintegrin domain multimers involves use of a leucine zipper domain. Leucine zipper domains are peptides that promote multimerization of the proteins in which they are found. Leucine zippers were originally identified in several DNA-binding proteins (Landschulz et al., Science 240:1759, 1988), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al. FEBS Lett. 344:191, 1994. The use of a modified leucine zipper that allows for stable trimerization of a heterologous protein fused thereto is described in Fanslow et al., Semin. Immunol. 6:267, 1994. Recombinant fusion polypeptides comprising an ADAM disintegrin domain polypeptide fused to a leucine zipper peptide are expressed in suitable host cells, and the ADAM disintegrin domain multimer that forms is recovered from the culture supernatant.

### C. Recombinant Production of ADAM Disintegrin Domain Polypeptides

The ADAM disintegrin domain polypeptides used in the present invention may be prepared using a recombinant expression system. Host cells transformed with a recombinant expression vector encoding the ADAM disintegrin domain polypeptide are cultured under conditions that promote expression of ADAM disintegrin domain and the ADAM disintegrin domain is recovered. ADAM disintegrin domain polypeptides can also be produced in transgenic plants or animals.

Any suitable expression system may be employed. Recombinant expression vectors include DNA encoding an ADAM disintegrin domain polypeptide operably linked to suitable transcriptional and translational regulatory nucleotide sequences, such as those derived from a mammalian, microbial, viral, or insect gene. Nucleotide sequences are operably linked when the regulatory sequence functionally relates to the ADAM disintegrin domain DNA sequence. Thus, a promoter nucleotide sequence is operably linked to an ADAM disintegrin domain DNA sequence if the promoter nucleotide sequence controls the transcription of the ADAM disintegrin domain DNA sequence. Examples of regulatory sequences include transcriptional promoters, operators, or enhancers, an mRNA ribosomal binding site, and appropriate sequences which control transcription and translation initiation and termination. A sequence encoding an appropriate signal peptide (native or heterologous) can be incorporated into expression vectors. A DNA sequence for a signal peptide (secretory leader) may be fused in frame to the ADAM disintegrin domain sequence so that the ADAM disintegrin domain polypeptide is initially translated as a fusion protein comprising the signal peptide. A signal peptide that is functional in the intended host cells promotes extracellular secretion of the ADAM disintegrin domain polypeptide. The signal peptide is cleaved from the ADAM disintegrin domain polypeptide upon secretion from the cell. Suitable host cells for expression of ADAM disintegrin domain polypeptides include prokaryotes, yeast and higher eukaryotic cells, including insect and mammalian cells. Appropriate cloning and expression vectors for use with bacterial, fungal, yeast, insect, and mammalian cellular hosts are known in the art.

Using the techniques of recombinant DNA including mutagenesis and the polymerase chain reaction (PCR), the skilled artisan can produce DNA sequences that encode ADAM disintegrin domain polypeptides comprising various additions or substitutions of amino acid residues or sequences, or deletions of terminal or internal residues or sequences, including ADAM disintegrin domain fragments, variants, derivatives, multimers, and fusion polypeptides.

The procedures for purifying expressed ADAM disintegrin domain polypeptides will vary according to the host system employed, and whether or not the recombinant polypeptide is secreted. ADAM disintegrin domain polypeptides may be purified using methods known in the art, including one or more concentration, salting-out, ion exchange, hydrophobic interaction, affinity purification, HPLC, or size exclusion chromatography steps. Fusion polypeptides comprising Fc moieties (and multimers formed therefrom) offer the advantage of facile purification by affinity chromatography over Protein A or Protein G columns.

### D. Therapeutic Methods

The disclosed methods may be used to inhibit integrin binding and integrin biological activity, and to inhibit endothelial cell migration, and/or angiogenesis in a mammal in need of such treatment. The treatment is advantageously administered in order to prevent the onset or the recurrence of a disease or condition mediated by an integrin, or to treat a mammal that has a disease or condition mediated by an integrin.

Examples of the therapeutic uses of ADAM disintegrin domain polypeptides and compositions thereof include the treatment of individuals afflicted with conditions mediated by angiogenesis such as ocular disorders, dermatological disorders, and malignant or metastatic conditions, inflammatory diseases, osteoporosis and other conditions mediated by accelerated bone resorption, restenosis, inappropriate platelet activation, recruitment, or aggregation, thrombosis, or a condition requiring tissue repair or wound healing.

Among the ocular disorders that can be treated according to the present invention are eye diseases characterized by ocular neovascularization including, but not limited to, diabetic retinopathy (a major complication of diabetes), retinopathy of prematurity (this devastating eye condition, that frequently leads to chronic vision problems and carries a high risk of blindness, is a severe complication during the care of premature infants), neovascular glaucoma, retinoblastoma, retrolental fibroplasia, rubeosis, uveitis, macular degeneration, and corneal graft neovascularization. Other eye inflammatory diseases, ocular tumors, and diseases associated with choroidal or iris neovascularization can also be treated according to the present invention.

The present invention can also be used to treat malignant and metastatic conditions such as solid tumors. Solid tumors include both primary and metastatic sarcomas and carcinomas.

The present invention can also be used to treat inflammatory diseases including, but not limited to, arthritis, rheumatism, inflammatory bowel disease, and psoriasis.

Among the conditions mediated by inappropriate platelet activation, recruitment, aggregation, or thrombosis that can be treated according to the present invention are coronary artery disease or injury, myocardial infarction or injury following myocardial infarction, stroke, unstable angina, atherosclerosis, arteriosclerosis, preeclampsia, embolism, platelet-associated ischemic disorders including lung ischemia, coronary ischemia, and cerebral ischemia, restenosis following percutaneous coronary intervention including angioplasty, atherectomy, stent placement, and bypass surgery, thrombotic disorders including coronary artery thrombosis, cerebral artery thrombosis, intracardiac thrombosis, peripheral artery thrombosis, venous thrombosis, thrombosis and coagulopathies associated with exposure to a foreign or injured tissue surface, and reocclusion following thrombosis, deep venous thrombosis (DVT), pulmonary embolism (PE), transient ischemic attacks (TIAs), and another conditions where vascular occlusion is a common underlying feature. In some embodiments the methods according to the invention are used in individuals at high risk for thrombus formation or reformation, advanced coronary artery disease, or for occlusion, reocclusion, stenosis and/or restenosis of blood vessels, or stroke. In some embodiments the methods according to the invention are used in combination with angioplasty procedures, such as balloon angioplasty, laser angioplasty, coronary atherectomy or similar techniques, carotid endarterectomy, anastomosis of vascular grafts, surgery having a high risk of thrombus formation (i.e., coronary bypass surgery, insertion of a prosthetic valve or vessel and the like), atherectomy, stent placement, placement of a chronic cardiovascular device such as an in-dwelling catheter or prosthetic valve or vessel, organ transplantation, or bypass surgery.

Other diseases and conditions that can be treated according to the present invention include benign tumors and preneoplastic conditions, myocardial angiogenesis, hemophilic joints, scleroderma, vascular adhesions, asthma and allergy, eczema and dermatitis, graft versus host disease, sepsis, adult respirator distress syndrome, telangiectasia, and wound granulation.

The methods according to the present invention can be tested in in vivo animal models for the desired prophylactic or therapeutic activity, as well as to determine the optimal therapeutic dosage, prior to administration to humans.

The amount of a particular ADAM disintegrin domain polypeptide that will be effective in a particular method of treatment depends upon age, type and severity of the condition to be treated, body weight, desired duration of treatment, method of administration, and other parameters. Effective dosages are determined by a physician or other qualified medical professional. Typical effective dosages are about 0.01 mg/kg to about 100 mg/kg body weight. In some preferred embodiments the dosage is about 0.1-50 mg/kg; in some preferred embodiments the dosage is about 0.5-10 mg/kg. The dosage for local administration is typically lower than for systemic administration. In some embodiments a single administration is sufficient; in some embodiments the ADAM disintegrin domain is administered as multiple doses over one or more days.

The ADAM disintegrin domain polypeptides are typically administered in the form of a pharmaceutical composition comprising one or more pharmacologically acceptable carriers. Pharmaceutically acceptable carriers include diluents, fillers, adjuvants, excipients, and vehicles which are pharmaceutically acceptable for the route of administration, and may be aqueous or oleaginous suspensions formulated using suitable dispersing, wetting, and suspending agents.

Pharmaceutically acceptable carriers are generally sterile and free of pyrogenic agents, and may include water, oils, solvents, salts, sugars and other carbohydrates, emulsifying agents, buffering agents, antimicrobial agents, and chelating agents. The particular pharmaceutically acceptable carrier and the ratio of active compound to carrier are determined by the solubility and chemical properties of the composition, the mode of administration, and standard pharmaceutical practice.

The ADAM disintegrin domain polypeptides are administered to the patient in a manner appropriate to the indication. Thus, for example, ADAM disintegrin domain polypeptides, or pharmaceutical compositions thereof, may be administered by intravenous, transdermal, intradermal, intraperitoneal, intramuscular, intranasal, epidural, oral, topical, subcutaneous, intracavity, sustained release from implants, peristaltic routes, or by any other suitable technique. Parenteral administration is preferred.

In certain embodiments of the claimed invention, the treatment further comprises treating the mammal with one or more additional therapeutic agents. The additional therapeutic agent(s) may be administered prior to, concurrently with, or following the administration of the ADAM disintegrin domain polypeptide. The use of more than one therapeutic agent is particularly advantageous when the mammal that is being treated has a solid tumor. In some embodiments of the claimed invention, the treatment further comprises treating the mammal with radiation. Radiation, including brachytherapy and teletherapy, may be administered prior to, concurrently with, or following the administration of the ADAM disintegrin domain polypeptide and/or additional therapeutic agent(s).

In some preferred embodiments the method includes the administration of, in addition to an ADAM disintegrin domain polypeptide, one or more therapeutics selected from the group consisting of alkylating agents, antimetabolites, vinca alkaloids and other plant-derived chemotherapeutics, antitumor antibiotics, antitumor enzymes, topoisomerase inhibitors, platinum analogs, adrenocortical suppressants, hormones and antihormones, antibodies, immunotherapeutics, radiotherapeutics, and biological response modifiers.

In some preferred embodiments the method includes administration of, in addition to an ADAM disintegrin domain polypeptide, one or more therapeutics selected from the group consisting of cisplatin, cyclophosphamide, mechloretamine, melphalan, bleomycin, carboplatin, fluorouracil, 5-fluorodeoxyuridine, methotrexate, taxol, asparaginase, vincristine, and vinblastine, lymphokines and cytokines such as interleukins, interferons (alpha., beta. or delta.) and TNF, chlorambucil, busulfan, carmustine, lomustine, semustine, streptozocin, dacarbazine, cytarabine, mercaptopurine, thioguanine, vindesine, etoposide, teniposide, dactinomycin, daunorubicin, doxorubicin, bleomycin, plicamycin, mitomycin, L-asparaginase, hydroxyurea, methylhydrazine, mitotane, tamoxifen, fluoxymesterone, IL-8 inhibitors, angiostatin, endostatin, kringle 5, angiopoietin-2 or other antagonists of angiopoietin-1, antagonists of platelet-activating factor, antagonists of basic fibroblast growth factor, and COX-2 inhibitors.

In some preferred embodiments the method includes administration of, in addition to an ADAM disintegrin domain polypeptide, one or more therapeutic polypeptides, including soluble forms thereof, selected from the group consisting of Flt3 ligand, CD40 ligand, interleukin-2, interleukin-12, 4-1BB ligand, anti-4-1BB antibodies, TRAIL, TNF antagonists and TNF receptor antagonists including TNFR/Fc. Tek antagonists, TWEAK antagonists and TWEAK-R antagonists including TWEAK-R/Fc, VEGF antagonists including anti-VEGF antibodies, VEGF receptor (including VEGF-R 1 and VEGF-R2, also known as FltI and FlkI or KDR) antagonists, CD 148 (also referred to as DEP-1, ECRTP, and PTPRJ, see Takahashi et al., J. Am. Soc. Nephrol. 10:2135-45, 1999; and PCT Publication No. WO 00/15258, 23 March 2000) binding proteins, and nectin-3 antagonists.

In some preferred embodiments the ADAM disintegrin domain polypeptides of the invention are used as a component of, or in combination with. "metronomic therapy," such as that described by Browder et al. and Klement et al. (Cancer Research 60:1878, 2000; J. Clin. Invest. 105(8):R15, 2000; see also Barinaga, Science 288:245, 2000).

As used herein, the terms "therapy," "therapeutic," "treat," and "treatment" generally include prophylaxis, i.e. prevention, in addition to therapy or treatment for an extant disease or condition. The methods of the present invention may be used as a first line treatment, for the treatment of residual disease following primary therapy, or as an adjunct to other therapies. Methods of measuring biological effectiveness are known in the art and are illustrated in the Examples below.

### EXAMPLES

The following examples are intended to illustrate particular embodiments and not to limit the scope of the invention.

### EXAMPLE 1

### ADAM Disintegrin Domain Polypeptides

This example describes one method for the recombinant production of ADAM disintegrin domain polypeptides.

Expression cassettes encoding an IgKappa leader sequence, ADAM disintegrin domain, and C-terminal Fc region were constructed in bacterial plasmids then transferred into eukaryotic expression vectors (pDC409, EMBO J. 10:2821, 1991, or another mammalian expression vector). The coding regions of the various constructs are summarized in Table 2. In addition to the disintegrin domain, these constructs encode additional portions of the extracellular portion of the ADAM (e.g., cysteine-rich region and EGF-like domain).

The expression vectors were transfected into COS-1, CV-1/EBNA, or 293/EBNA cells. Two days after transfection the cells were ³⁵S labeled for four hours. Supernatants and total cell lysates were prepared and aliquots were immunoprecipitated using protein A-sepharose beads to capture the Fc tagged polypeptides. ³⁵S labeled ADAM disintegrin-Fc polypeptides were run on 8-16% reducing gels and detected via autoradiography.

The cell type that produced the most soluble protein in the supernatant was used in a large scale (T-175 format, 20 flasks) transient transfection, and approximately one liter of supernatant was harvested after one week. ADAM disintegrin-Fc polypeptides were purified from the supernatants using affinity chromatography (protein A column). The polypeptides were characterized by determining the N-terminal amino acid sequence, amino acid composition, and protein integrity (SDS-PAGE under reducing and non-reducing conditions) before the polypeptides were used in FACS, immunoprecipitations, and biological assays such as those described below.

**Table 2**

| ADAM Disintegrin Domain Polypeptide Constructs | | | | |
|---|---|---|---|---|
| Construct | SEQ ID NOs: DNA/polypeptide | IgK Leader^{1,2} | ADAM disintegrin^{1,3} (dis Framework)^{1,4} | Fc Region¹ |
| ADAM-8dis-Fc | 1/2 | 1-20 | 23-264 (34-91) | 267-494 |
| ADAM-9dis-Fc | 3/4 | 1-20 | 23-303 (34-92) | 306-533 |
| ADAM-10dis-Fc | 5/6 | 1-20 | 23-235 (34-99) | 238-465 |
| ADAM-15dis-Fc | 7/8 | 1-20 | 23-292 (34-92) | 295-522 |
| ADAM-17dis-Fc | 9/10 | 1-20 | 23-216 (34-93) | 219-446 |
| ADAM-20dis-Fc | 11/12 | 1-20 | 23-305 (34-91) | 308-535 |
| ADAM-21dis-Fc | 13/14 | 1-20 | 23-293 (34-91) | 296-523 |
| ADAM-22dis-Fc | 15/16 | 1-20 | 23-312 (34-92) | 315-542 |
| ADAM-23dis-Fc | 17/18 | 1-20 | 23-310 (34-91) | 313-540 |
| ADAM-29dis-Fc | 21/22 | 1-20 | 23-298 (34-91) | 301-528 |

| | | | | |
|---|---|---|---|---|
| ¹ residues in the polypeptide sequence ² the predicted cleavage site is after residue 20 ³ segment of the construct that includes ADAMdis, but may also contain additional ADAM sequences ⁴ disintegrin framework, e.g., SEQ ID NO:20 | | | | |

### EXAMPLE 2

### Binding of ADAM Disintegrin Domain Polypeptides to Cells

### A. Binding to Endothelial cells

This example describes a flow cytometric integrin mAb based binding inhibition assay, which is used to show binding of ADAM disintegrin-Fc polypeptides to integrins expressed on the surface of endothelial cells. Human endothelial cells express αᵥβ₃, αᵥβ₅, β₁, β₄, α₁, α₂, α₃, α₄, α₅, and αₒ integrins.

Primary human dermal microvascular endothelial cells (HMVEC-d) were maintained in supplemented endothelial growth medium (Clonetics Corporation, Walkersville, MD). The ADAM disintegrin-Fc polypeptides produced in Example 1 were shown to bind specifically to HMVEC-d. Monoclonal antibodies specific for human integrins αᵥβ₃ (LM609, anti CD51/61, Chemicon, Temecula, CA Brooks et al., Science 264:569, 1994), α₂β₁ (BHA2.1 anti CD49b, Chemicon, Wang et al., Mol. Biol. of the Cell 9:865, 1998), α₅β₁ (SAM-1 anti CD49e, Biodesign, A. te Velde et al., J. Immunol. 140:1548, 1988), α₃β₁ (ASC-6 anti-CD49c, Chemicon, Pattaramalai et al., Exp. Cell. Res. 222: 281, 1996), α₄β₁ (HP2/1 anti CD49d, Immunotech, Marseilles, France. Workshop of the 4^{th} International Conference on Human Leukocyte Differentiation Antigens, Vienna Austria, 1989, workshop number p091), α₆β₁. (GoH3 anti CD49f, Immunotech, Workshop 4^{th} International Conference on Human Leukocyte Differentiation Antigens, workshop number p055), α₆β₄ (439-9B anti CD104, Pharmingen, San Diego, CA., Schlossman et al., 1995 Leukocyte Typing V: White Cell Diffemtiation Antigens. Oxford University Press, New York), and αᵥβ₅ (MAB 1961, Chemicon International, monoclonal anti-human integrin αᵥβ₅ mAb, IgG1 isotype, inhibits αᵥβ₅ mediated binding/adhesion to vitronectin/fibronectin; Weinaker, et al., J. Biol. Chem. 269:6940, 1994) were also shown to bind specifically to HMVEC-d. Each of these antibodies is known to specifically block binding of the indicated integrin to its ligands (e.g., fibronectin, vitronectin, fibrinogen). The ability of integrin mAbs to inhibit the binding of ADAM disintegrin-Fc polypeptides reveals which integrins the disintegrin domains bind and, indirectly, which integrin binding activities the disintegrin domains are able to antagonize. The ability of the antibodies to inhibit binding of the ADAM disintegrin-Fc polypeptides to endothelial cells was tested as described below.

Prior to performing binding studies, HMVEC-d were removed from culture vessels using trypsin-EDTA. The cells were washed in media containing serum and resuspended in binding medium which consisted of PBS containing 1 mM Ca2+, 1 mM Mg2+ and 0.5 mM Mn2+, 0.1% sodium azide, 10% Normal goat serum, 2% rabbit serum and 2% fetal bovine serum. Under these binding conditions, ADAM-8, -9, -10, -15, -17, -20, -21, -22, -23, and -29dis-Fc all bind to human endothelial cells.

One hundred microliters of cell suspension, containing 200,000 to 500,000 HMVEC-d, were added to 12x75mm plastic test tubes. Monoclonal antibodies specific for one of the integrins, or a control monoclonal antibody (CD29 or M15), were added to the cell suspensions at a concentration of 100 µg/ml (5-8 fold mass excess) 15 minutes prior to addition of disintegrin-Fc fusion proteins. ADAM disintegrin-Fc polypeptides and control Fc fusion polypeptides (P7.5II.Fc) were added, at various concentrations from 12.5 to 20 µg/ml, to the cell suspensions and incubated for 1 hour at 30° C. Unbound Fc polypeptides were washed away by centrifugation of cells in 2 mls of binding media. The washed cell pellets were resuspended in binding medium and then incubated at 30° C for 30 minutes with goat anti-human Fc-specific biotinylated antibody at a concentration of 2.5 µg/ml for 30 minutes. After centrifugation and washing of the cell pellets, the cells were resuspended in binding medium and bound anti-human Fc-biotin was detected by adding streptavidin-phycoerythrin conjugate to the cell suspension at a 1:1000 dilution (1 µg/ml) and incubating at 30° C for 30 minutes. The unbound streptavidin-phycoerythrin was washed away and the cells were resuspended in binding medium containing propidum iodide. The level of fluorescent binding (disintegrin-Fc binding) was determined by flow cytometry.

The level of binding of each ADAM disintegrin-Fc polypeptide was determined in the presence of anti-integrin specific mAb and in the presence of control mAb. Both the intensity of binding (MFI) and the percentage of cells binding were determined. Percent inhibition was calculated using the formula [1 - (MFI control-MFI integrin mAb) / MFI control. The results of these studies are summarized in Table 3.

ADAM-15, -17, -20 and -22 disintegrin domain polypeptides bound to αᵥβ₃; ADAM 23 disintegrin domain polypeptide bound to α₂β₁; ADAM-15, -21, -22 and -23 disintegrin domain polypeptides bound to α₅β₁; ADAM-10, -17, -22 and -23 disintegrin domain polypeptides bound to the α₆ integrins; ADAM-10 and -15 disintegrin domain polypeptides bound to αᵥβ₅. An excess of a non blocking αᵥβ₅ antibody did significantly affect the binding of ADAM-10, -22, and -23 disintegrin polypeptides to endothelial cells, suggesting that these ADAMdis polypeptides interact with integrin sites other than or in addition to the ligand (e.g., fibronectin, vitronectin) binding site. Based upon results from a different type of assay, Cal et al. have reported that the ADAM-23 disintegrin domain interacts with the αᵥβ₃ integrin through an RGD-independent mechanism (Molec. Biol. of the Cell 11:1457, 2000).

Binding experiments are repeated using other ADAM disintegrin domains and other monoclonal antibodies. ADAM disintegrin-Fc polypeptides that bind to selected integrins are further tested for the ability to disrupt integrin-ligand interactions and to modulate endothelial cell function, angiogenesis, and other biological activities in vitro and in vivo.

**Table 3**

| Binding of ADAM Disintegrin-Fc Polypeptides to Integrins Expressed on Human Endothelial Cells | | | | | | | |
|---|---|---|---|---|---|---|---|
| | **Integrin** | | | | | | |
| | Binding¹ (+ or - or ND. not done) and Percent (%) Binding² | | | | | | |
| ADAM | αᵥβ₃ | α₂β₁ | α₃β₁ | α₄β₁ | α₅β₁ | α₆β₁.α₆β₄ | αᵥβ₅ |
| ADAM-8 | ND | ND | - (<10) | - (<10) | ND | ND | - <20) |
| ADAM-9 | - (<10) | - (<10) | - (<10) | - (<20) | - (<10) | - (<10) | - (<10) |
| ADAM-10 | - (<10) | - (<10) | - (<10) | - (<20) | - (<10) | + (48) | + (25) |
| ADAM-15 | + (60) | - (<10) | - (<10) | - (<20) | + (30) | - (<10) | + (25) |
| ADAM-17 | + (50) | - (<10) | - (<10) | - (<10) | - (<10) | + (69) | - (<10) |
| ADAM-20 | + (58) | - (<10) | - (<10) | - (<10) | - (<20) | - (<10) | - (<10) |
| ADAM-21 | - (<10) | - (<10) | - (<10) | - (<10) | + (54) | - (<10) | - (<10) |
| ADAM-22 | + (42) | - (<10) | - (<10) | - (<10) | + (36) | + (32) | - (<10) |
| ADAM-23 | - (<10) | + (22) | - (<10) | - (<10) | + (49) | + (31) | - (<10) |

| | | | | | | | |
|---|---|---|---|---|---|---|---|
| ¹positive binding defined as >20% binding inhibition; normal background variation 5-10%, baseline positive approx. 2X over background ²percent inhibition of binding by ADAM-dis-Fc in the presence of 5-8 fold excess integrin mAb as compared to control mAb | | | | | | | |

### B. Binding to Primary Human T-Cells

Primary human T-cells were purified from whole blood. These cells were used in FACS experiments to assess cell surface binding of purified ADAMdis-Fc polypeptides. ADAMdis-Fc binding was assessed with and without Con A (5 µg/ml) or immobilized OTK3 antibody (1 mg/ml, immobilized for 1 hour, 37°C) stimulation. ADAMdis-Fc polypeptides (20 µg/ml) were bound at either 4° C or 30° C in the presence of cations (Ca++, Mg++, Mn++, 0.5 mM each). Cell surface integrin expression was assessed using a panel of murine and rat anti-human integrin antibodies. αᵥβ₅. α₁, α₃, α₄, α₆, β₁, and β₇ integrins were detected on the surface of these cells. ADAMdis-Fc polypeptides did not bind to primary human T-cells at 4° C. ADAM-8-, ADAM-9-, ADAM-15-, ADAM-20-, ADAM-21-, ADAM-22-, and ADAM-23-dis-Fc polypeptides did bind primary T-cells at 30° C with Con A stimulation. ADAMdis-Fc binding was not inhibited by a three-fold molar excess of antibodies to the integrins listed above.

### C. Binding to Resting Platelets

Binding of ADAMdis-Fc polypeptides to citrated washed resting platelets was performed at 4°C or 30°C. Binding was analyzed by flow cytometry using a biotinylated-anti-human Fc specific antibody and streptavidin-PE. Resting platelets express the integrins CD41/CD61 and CD49e. ADAM-9dis-Fc and ADAM-8dis-Fc bound resting platelets at 30°C but not at 4°C. ADAM-9dis-Fc binding to resting platelets at 30°C was not inhibited by a ten-fold excess of CD41a mAb.

### EXAMPLE 3

### Activity of ADAM Disintegrin Domain Polypeptides In a Wound Closure Assay

A planar endothelial cell migration (wound closure) assay was used to quantitate the inhibition of angiogenesis by ADAM disintegrin-Fc polypeptides in vitro. In this assay, endothelial cell migration is measured as the rate of closure of a circular wound in a cultured cell monolayer. The rate of wound closure is linear, and is dynamically regulated by agents that stimulate and inhibit angiogenesis in vivo.

Primary human renal microvascular endothelial cells, HRMEC, were isolated, cultured, and used at the third passage after thawing, as described in Martin et al., In Vitro Cell Dev Biol 33:261, 1997. Replicate circular lesions, "wounds," (600-800 micron diameter) were generated in confluent HRMEC monolayers using a silicon-tipped drill press. At the time of wounding the medium (DMEM + 1% BSA) was supplemented with 20 ng/ml PMA (phorbol-12-myristate-13-acetate), a range of concentrations of ADAM disintegrin-Fc polypeptide, or combinations of PMA and ADAM disintegrin-Fc polypeptide. The residual wound area was measured as a function of time (0-12 hours) using a microscope and image analysis software (Bioquant, Nashville, TN). The relative migration rate was calculated for each agent and combination of agents by linear regression of residual wound area plotted over time. The inhibition of PMA-induced endothelial migration by ADAM disintegrin-Fc polypeptides is shown in Table 4.

The effect of ADAM-dis-Fc polypeptides on EGF-induced migration was also determined. For these experiments EGF (epidermal growth factor, 40 ng/ml) was added to the medium, instead of PMA, at the time of wounding. The results are shown in Table 5.

**Table 4**

| Effect of ADAM-15, -17, -20, and -23dis-Fc Polypeptides in PMA-Induced Endothelial Cell Wound Closure Migration Assay | | | | | | | |
|---|---|---|---|---|---|---|---|
| Expt. ID | No Addition | PMA 20 ng/ml | PMA + IgG | PMA + ADAM-15dis-Fc | PMA + ADAM-17dis-Fc | PMA + ADAM-20dis-Fc | PMA + ADAM-23dis-Fc |
| HL-H-142 15 µg/ml dis-Fc | 0.0436¹ (0.0016)² | 0.0655 (0.0004) | | | | 0.0499 (0.0009) 72%³ | |
| HL-H-147 15 µg/ml dis-Fc | 0.0244 (0.0023) | 0.0424 (0.0002) | 0.0449 (0.0012) 0% | 0.0357 (0.0007) 37% | | | 0.0225 (0.0022) 100% |
| HL-H-153 15 µg/ml dis-Fc | 0.0253 0.00013 | 0.0460 (0.0022) | 0.0491 (0.006) 0% | | 0.0392 (0.0016) 33% | 0.0388 (0.005) 36% | 0.0317 (0.005) 70% |
| HL-H-154 15 µg/ml dis-Fc | 0.0119 (0.0012) | 0.0312 (0.0016) | | | 0.0283 (0.0008) 15% | 0.0160 (0.0017) 79% | |

| | | | | | | | |
|---|---|---|---|---|---|---|---|
| ¹ Slopes to average triplicate Y values and treat as a single data point in order to test whether the slopes are significantly different ² Data in parentheses is the +/- standard error of slopes ³ Percent inhibition compared to migration rate observed in the presence of PMA | | | | | | | |

**Table 5**

| Effect of ADAM-17, -20, and -23dis-Fc Polypeptides in EGF-Induced Endothelial Cell Wound Closure Migration Assay | | | | | | |
|---|---|---|---|---|---|---|
| Expt. ID | No Addition | EGF 40 ng/ml | EGF + IgG | EGF + ADAM-17dis-Fc | EGF + ADAM-20dis-Fc | EGF + ADAM-23dis-Fc |
| HL-H-154 15 µg/ml dis-Fc | 0.0119 (0.0012) | 0.0378 (0.0061) | | 0.0242 (0.0029) 53% | 0.0172 (0.0031) 80% | 0.0310 (0.0036) 26% |
| HL-H-155 9 µg/ml dis-Fc | 0.0164 (0.0010) | 0.0468 (0.0059) | 0.0454 (0.0052) 5% | 0.0412 (0.0107) 18% | 0.0227 (0.0035) 79% | 0.0207 (0.0016) 86% |

| | | | | | | |
|---|---|---|---|---|---|---|
| ¹ Slopes to average triplicate Y values and treat as a single data point in order to test whether the slopes are significantly different ² Data in parentheses is the +/- standard error of slopes ³ Percent inhibition compared to migration rate observed in the presence of EGF alone | | | | | | |

ADAM-20 and -23dis-Fc polypeptides showed the greatest inhibition of both EGF- and PMA-induced endothelial migration at 15 µg/ml. ADAM-15 and -17dis-Fc polypeptides were less effective at inhibiting endothelial cell migration at 15 µg/ml. Hu IgG did not inhibite EGF- or PMA-induced endothelial cell migration in any of the experiments performed where it was included as a control Fc protein.

### EXAMPLE 4

### Activity of ADAM Disintegrin Domain Polypeptides In a Corneal Pocket Assay

A mouse corneal pocket assay is used to quantitate the inhibition of angiogenesis by ADAM disintegrin-Fc polypeptides in vivo. In this assay, agents to be tested for angiogenic or anti-angiogenic activity are immobilized in a slow release form in a hydron pellet, which is implanted into micropockets created in the corneal epithelium of anesthetized mice. Vascularization is measured as the appearance, density, and extent of vessel ingrowth from the vascularized corneal limbus into the normally avascular cornea.

Hydron pellets, as described in Kenyon et al., Invest Opthamol. & Visual Science 37:1625, 1996, incorporate sucralfate with bFGF (90 ng/pellet), bFGF and IgG (11 µg/pellet, control), or bFGF and a range of concentrations of ADAM disintegrin-Fc polypeptide. The pellets are surgically implanted into corneal stromal micropockets created by micro-dissection 1 mm medial to the lateral corneal limbus of 6-8 week old male C57BL mice. After five days, at the peak of neovascular response to bFGF, the corneas are photographed, using a Zeiss slit lamp, at an incipient angle of 35-50° from the polar axis in the meridian containing the pellet. Images are digitized and processed by subtractive color filters (Adobe Photoshop 4.0) to delineate established microvessels by hemoglobin content. Image analysis software (Bioquant, Nashville, TN) is used to calculate the fraction of the corneal image that is vascularized, the vessel density within the vascularized area, and the vessel density within the total cornea. The inhibition of bFGF-induced corneal angiogenesis, as a function of the dose of ADAM disintegrin-Fc polypeptide, is determined.

### EXAMPLE 5

### Inhibition of Neovascularization by ADAM Disintegrin Domain Polypeptides in a Murine Transplant Model

Survival of heterotopically transplanted cardiac tissue from one mouse donor to the ear skin of another genetically similar mouse requires adequate neovascularization by the transplanted heart and the surrounding tissue, to promote survival and energy for cardiac muscle function. Inadequate vasculature at the site of transplant causes excessive ischemia to the heart, tissue damage, and failure of the tissue to engraft. Agents that antagonize factors involved in endothelial cell migration and vessel formation can decrease angiogenesis at the site of transplant, thereby limiting graft tissue function and ultimately engraftment itself. A murine heterotopic cardiac isograft model is used to demonstrate the antagonistic effects of ADAM disintegrin-Fc polypeptides on neovascularization. Female BALB/c (≈12 weeks of age) recipients are given neonatal heart grafts from donor mice of the same strain. The donor heart tissue is grafted into the left ear pinnae of the recipient on day 0 and the mice are divided into two groups. The control group receives human IgG (Hu IgG) while the other group receives ADAM disintegrin-Fc polypeptide, both intraperitoneally. The treatments are continued for five consecutive days. The functionality of the grafts is determined by monitoring visible pulsatile activity on days 7 and 14 post-engraftment. The inhibition of functional engraftment, as a function of the dose of ADAM disintegrin-Fc polypeptide, is determined. The histology of the transplanted hearts is examined is order to visualize the effects of ADAM disintegrin-Fc polypeptides on edema at the site of transplant and host and donor tissue vasculature (using, e.g., Factor VIII staining).

### EXAMPLE 6

### Treatment of Tumors With ADAM Disintegrin Domain Polypeptides

ADAM disintegrin-Fc polypeptides are tested in animal models of solid tumors. The effect of the ADAM disintegrin-Fc polypeptides is determined by measuring tumor frequency and tumor growth.

The biological activity of ADAM disintegrin-Fc polypeptides is also demonstrated in other in vitro, ex vivo, and in vivo assays known to the skilled artisan, such as calcium mobilization assays and assays to measure platelet activation, recruitment, or aggregation.

The relevant disclosures of publications cited herein are specifically incorporated by reference. The examples presented above are not intended to be exhaustive or to limit the scope of the invention. The skilled artisan will understand that variations and modifications and variations are possible in light of the above teachings, and such modifications and variations are intended to be within the scope of the invention.

## Claims

1. A method of antagonizing the binding of an integrin to its ligands comprising contacting a cell that expresses the integrin with an effective amount of an ADAM disintegrin domain polypeptide.

2. A method of antagonizing the binding of an integrin to its ligands in a mammal in need of such treatment comprising administering an effective amount of an ADAM disintegrin domain polypeptide.

3. The method of claim 2 wherein the mammal is afflicted with a condition selected from the group consisting of ocular disorders, malignant and metastatic conditions, inflammatory diseases, osteoporosis and other conditions mediated by accelerated bone resorption, restenosis, inappropriate platelet activation, recruitment, or aggregation, thrombosis, or a condition requiring tissue repair or wound healing.

4. A method of inhibiting angiogenesis in a mammal in need of such treatment, comprising administering to the mammal an inhibition-effective amount of an ADAM disintegrin domain polypeptide, wherein the disintegrin domain does not contain an RGD sequence.

5. The method of one of claims 1-4 wherein the ADAM disintegrin domain is in the form of a multimer.

6. The method of claim 5 wherein the multimer is a dimer or trimer.

7. The method of claim 5 wherein the multimer comprises an Fc polypeptide or a leucine zipper.

8. The method of one of claims 1-7 wherein the ADAM disintegrin domain is from a human ADAM.

9. The method of claim 8 wherein the ADAM disintegrin domain is from an ADAM selected from the group consisting of ADAM-8, ADAM-9, ADAM-10, ADAM-15, ADAM-17, ADAM-20, ADAM-21, ADAM-22, ADAM-23, and ADAM-29.

10. The method of claim 9 wherein the ADAM disintegrin domain is from ADAM-17, ADAM-20, or ADAM-23.

11. The method of one of claims 1-10 wherein the ADAM disintegrin domain polypeptide comprises an amino acid sequence selected from the group consisting of:
(a) amino acids 1-494 of SEQ ID NO:2, amino acids 23-264 of SEQ ID NO:2, amino acids 1-533 of SEQ ID NO:4, amino acids 23-303 of SEQ ID NO:4, amino acids 1-465 of SEQ ID NO:6, amino acids 23-235 of SEQ ID NO:6, amino acids 1-522 of SEQ ID NO:8, amino acids 23-292 of SEQ ID NO:8, amino acids 1-446 of SEQ ID NO:10, amino acids 23-216 of SEQ ID NO:10, amino acids 1-535 of SEQ ID NO:12, amino acids 23-305 of SEQ ID NO: 12, amino acids 1-523 of SEQ ID NO:14, amino acids 23-293 of SEQ ID NO:14, amino acids 1-542 of SEQ ID NO:16, amino acids 23-312 of SEQ ID NO:16, amino acids 1-540 of SEQ ID NO:18, amino acids 23-310 of SEQ ID NO:18, amino acids 1-528 of SEQ ID NO:22, amino acids 23-298 of SEQ ID NO:22;
(b) fragments of the polypeptides of (a) wherein said fragments retain at least one ADAMdis activity;
(c) variants of the polypeptides of (a) or (b), wherein said variants retain at least one ADAMdis activity; and
(d) fusion polypeptides comprising the polypeptides of (a), (b), or (c), wherein said fusion polypeptides retain at least one ADAMdis activity.

12. The method of claim 1 1 wherein the ADAM disintegrin domain comprises an amino acid sequence selected from the group consisting of amino acids 34-91 of SEQ ID NO:2, 34-92 of SEQ ID NO:4, 34-99 of SEQ ID NO:6. 34-92 of SEQ ID NO:8, 34-93 of SEQ ID NO:10, 34-91 of SEQ ID NO:12, 34-91 of SEQ ID NO: 14,34-92 of SEQ ID NO: 16,34-91 of SEQ ID NO:18, or 34-91 of SEQ ID NO:22.

13. The method of one of claims 1-12 wherein the ADAM disintegrin domain polypeptide is a variant that is at least 70%, 80%, 90%, 95%, 98%, or 99% identical in amino acid sequence to a polypeptide selected from the group consisting of:
(a) amino acids 1-494 of SEQ ID NO:2, amino acids 23-264 of SEQ ID NO:2, amino acids 1-533 of SEQ ID NO:4, amino acids 23-303 of SEQ ID NO:4, amino acids 1-465 of SEQ ID NO:6, amino acids 23-235 of SEQ ID NO:6, amino acids 1-522 of SEQ ID NO:8, amino acids 23-292 of SEQ ID NO:8, amino acids 1-446 of SEQ ID NO: 1 0, amino acids 23-216 of SEQ ID NO:10, amino acids 1-535 of SEQ ID NO:12, amino acids 23-305 of SEQ ID NO: 12, amino acids 1-523 of SEQ ID NO: 14, amino acids 23-293 of SEQ ID NO: 14, amino acids 1-542 of SEQ ID NO: 16, amino acids 23-312 of SEQ ID NO: 16, amino acids 1-540 of SEQ ID NO:18, amino acids 23-310 of SEQ ID NO: 18, amino acids 1-528 of SEQ ID NO:22, amino acids 23-298 of SEQ ID NO:22; and
(b) fragments of the polypeptides of (a),
wherein said variant polypeptide retains at least one ADAMdis activity.

14. The method of one of claims 1-10 wherein the ADAM disintegrin domain polypeptide is encoded by a nucleic acid comprising a sequence selected from the group consisting of:
(a) nucleotides 118-1599 of SEQ ID NO:1, nucleotides 184-909 of SEQ ID NO:1, nucleotides 46-1644 of SEQ ID NO:3, nucleotides 112-954 of SEQ ID NO:3, nucleotides 25-1419 of SEQ ID NO:5, nucleotides 91-729 of SEQ ID NO:5, nucleotides 41-1606 of SEQ ID NO:7, nucleotides 107-916 of SEQ ID NO:7, nucleotides 25-1362 of SEQ ID NO:9, nucleotides 91-672 of SEQ ID NO:9, nucleotides 25-1629 of SEQ ID NO:11, nucleotides 91-939 of SEQ ID NO:11, nucleotides 25-1593 of SEQ ID NO:13, nucleotides 91-903 of SEQ ID NO: 13, nucleotides 25-1650 of SEQ ID NO:15, nucleotides 91-960 of SEQ ID NO: 15, nucleotides 25-1644 of SEQ ID NO: 17, nucleotides 91-954 of SEQ ID NO:17, nucleotides 118-1701 of SEQ ID NO:21, nucleotides 184-101 of SEQ ID NO:21;
(b) sequences which, due to the degeneracy of the genetic code, encode a polypeptide encoded by a nucleic acid of (a); and
(c) sequences that hybridize under conditions of moderate or high stringency to a sequence of (a) or (b) and that encode a polypeptide that retains at least one ADAMdis activity.

15. The method of one of claim 11-14 wherein the ADAMdis activity is selected from the group consisting of integrin binding activity, inhibition of endothelial cell migration, and inhibition of angiogenesis.

16. The method of one of claims 1-15 wherein the ADAM disintegrin domain polypeptide has been produced by culturing a recombinant cell that encodes the ADAM disintegrin domain polypeptide under conditions permitting expression of the ADAM disintegrin domain polypeptide, and recovering the ADAM disintegrin domain polypeptide.

17. The method of one of claims 1-16 wherein the ADAM disintegrin domain polypeptide is present in a composition comprising a pharmaceutically acceptable carrier.

18. The method of claim 2 wherein the mammal has a disease or condition mediated by angiogenesis.

19. The method of claim 18 wherein the disease or condition is **characterized by** ocular neovascularization.

20. The method of claim 18 wherein the disease or condition is a solid tumor.

21. The method of one of claims 1-20 wherein the method further comprises treating the mammal with radiation.

22. The method of one of claims 1-21 wherein the method further comprises treating the mammal with a second therapeutic agent.

23. The method of claim 22 wherein the second therapeutic agent is selected from the group consisting of alkylating agents, antimetabolites, vinca alkaloids and other plant-derived chemotherapeutics, antitumor antibiotics, antitumor enzymes, topoisomerase inhibitors, platinum analogs, adrenocortical suppressants, hormones and antihormones, antibodies, immunotherapeutics, radiotherapeutics, and biological response modifiers.

24. The method of claim 22 wherein the second therapeutic agent is selected from the group consisting of cisplatin, cyclophosphamide, bleomycin, carboplatin, fluorouracil, 5-fluorouracil, 5-fluorodeoxyuridine, methotrexate, taxol, asparaginase, vincristine, vinblastine, mechloretamine, melphalan, 5-tluorodeoxyuridine, lymphokines and cytokines such as interleukins, interferons (alpha., beta, or delta.) and TNF, chlorambucil, busulfan, carmustine, lomustine, semustine, streptozocin, dacarbazine, cytarabine, mercaptopurine, thioguanine, vindesine, etoposide, teniposide, dactinomycin, daunorubicin, doxorubicin, bleomycin, plicamycin, mitomycin, L-asparaginase, hydroxyurea, methylhydrazine, mitotane, tamoxifen, fluoxymesterone, and COX-2 inhibitors.

25. The method of claim 22 wherein the second therapeutic agent is a polypeptide, including soluble forms thereof, selected from the group consisting of Flt3 ligand, CD40 ligand, interleukin-2, interleukin-12, 4-IBB ligand, anti-4-1BB antibodies, TRAIL, TNF antagonists and TNF receptor antagonists including TNFR/Fc, Tek antagonists, TWEAK antagonists and TWEAK-R antagonists including TWEAK-R/Fc, VEGF antagonists including anti-VEGF antibodies, VEGF receptor antagonists, CD 148 binding proteins, and nectin-3 antagonists.

26. The method of claim 2 wherein the ADAM disintegrin domain is administered parenterally.

27. A method for inhibiting the biological activity of an integrin selected from the group consisting of αᵥβ₃, α₂β₁, α₅β₁, α₆β₁, α₆β₄, and αᵥβ₅ comprising contacting the integrin with an inhibition-effective amount of an ADAM disintegrin domain polypeptide.

28. The method of claim 27 wherein the integrin is αᵥβ₃ and wherein the ADAM disintegrin domain does not contain an RGD sequence.

29. The method of claim 28 wherein the ADAM is ADAM-17, ADAM-20, or ADAM-22.

30. The method of claim 27 wherein the integrin is α₂β₁ and the ADAM is ADAM-23.

31. The method of claim 27 wherein the integrin is α₅β₁ and the ADAM is ADAM-15 ADAM-21, ADAM-22, or ADAM-23.

32. The method of claim 27 wherein the integrin is α₆β₁ or α₆β₄ and the ADAM is ADAM-10, ADAM-17, ADAM-22, or ADAM-23.

33. The method of claim 27 wherein the integrin is αᵥβ₅ and the ADAM is ADAM-10, ADAM-15, or ADAM-23.

34. A method for identifying a compound that modulates integrin biological activity comprising:
(a) combining a test compound with an integrin and an ADAM disintegrin domain polypeptide that binds to the integrin; and
(b) determining whether the test compound alters the binding of the ADAM disintegrin domain polypeptide to the integrin.

35. A method for identifying a compound that modulates the interaction between an integrin and an ADAM disintegrin domain comprising:
(a) combining a test compound with the integrin and an ADAM disintegrin domain polypeptide that binds to the integrin; and
(b) determining whether the test compound alters the binding of the ADAM disintegrin domain polypeptide to the integrin.

36. The method of claim 34 or 35 wherein the integrin is present on a cell surface.

37. The method of claim 36 wherein the cell is an endothelial cell.

38. The method of one of claims 34-37 wherein the integrin is selected from the group consisting of αᵥβ₃, α₂β₁, α₅β₁, α₆β₁, α₆β₄, and αᵥβ₅.

39. The method of one of claims 34-38 wherein the integrin biological activity or integrin binding activity is at least partially inhibited.

40. A method for identifying a compound that inhibits endothelial cell migration and/or angiogenesis comprising:
(a) combining a test compound with endothelial cells and with an ADAM disintegrin domain polypeptide that binds to endothelial cells; and
(b) determining whether the test compound alters the binding of the ADAM disintegrin domain polypeptide to the endothelial cells.

41. The method of one of claims 34-40 wherein the ADAM disintegrin domain polypeptide comprises an ADAM disintegrin domain from ADAM-8, ADAM-9, ADAM-10, ADAM-15, ADAM-17, ADAM-20, ADAM-21, ADAM-22, ADAM-23, or ADAM-29.

42. The method of claim 41 wherein the ADAM disintegrin domain polypeptide comprises an ADAM disintegrin domain from ADAM-17, ADAM-20, or ADAM-23.


### Annex to the application documents - subsequently filed sequences listing

SEQUENCE LISTING

<110> Immunex Corporation
      Fanslow, William C.
      Poindexter, Kurt
      Cerretti, Douglas P.
      Black, Roy A.

<120> INTEGRIN ANTAGONISTS

<130> 2958-WO

<140>
<141>

<150> 60/184,865
<151> 2000-02-25

<160> 22

<170> PatentIn Ver. 2.1

<210> 1
<211> 1700
<212> DNA
<213> Artificial Sequence

<220>
<223> Description of Artificial Sequence: fusion
      polypeptide

<220>
<221> CDS
<222> (118)..(1602)

<400> 1
gggttttccc agtcacgacg ttgtaaaacg acggccagtg aattgtaata cgactcacta 60

tagggcgaat tgggtaccgg gccccccctc gaggtcgacc caagctggct agccacc    117

atg gag aca gac aca ctc ctg cta tgg gta ctg ctg ctc tgg gtt cca   165
Met Glu Thr Asp Thr Leu Leu Leu Trp Val Leu Leu Leu Trp Val Pro
  1               5                  10                  15

ggt tcc act ggt act agt tgt ggg aac ctg ttt gtg gag cgt ggg gag   213
Gly Ser Thr Gly Thr Ser Cys Gly Asn Leu Phe Val Glu Arg Gly Glu
             20                  25                  30

cag tgc gac tgc ggc ccc ccc gag gac tgc cgg aac cgc tgc tgc aac   261
Gln Cys Asp Cys Gly Pro Pro Glu Asp Cys Arg Asn Arg Cys Cys Asn
         35                  40                  45

tct acc acc tgc cag ctg gct gag ggg gcc cag tgt gcg cac ggt acc   309
Ser Thr Thr Cys Gln Leu Ala Glu Gly Ala Gln Cys Ala His Gly Thr
     50                  55                  60

tgc tgc cag gag tgc aag gtg aag ccg gct ggt gag ctg tgc cgt ccc   357
Cys Cys Gln Glu Cys Lys Val Lys Pro Ala Gly Glu Leu Cys Arg Pro
 65                  70                  75                  80

aag aag gac atg tgt gac ctc gag gag ttc tgt gac ggc cgg cac cct   405
Lys Lys Asp Met Cys Asp Leu Glu Glu Phe Cys Asp Gly Arg His Pro
                 85                  90                  95

gag tgc ccg gaa gac gcc ttc cag gag aac ggc acg ccc tgc tcc ggg   453

Glu Cys Pro Glu Asp Ala Phe Gln Glu Asn Gly Thr Pro Cys Ser Gly
            100                 105                 110

ggc tac tgc tac aac ggg gcc tgt ccc aca ctg gcc cag cag tgc cag 501
Gly Tyr Cys Tyr Asn Gly Ala Cys Pro Thr Leu Ala Gln Gln Cys Gln
        115                 120                 125

gcc ttc tgg ggg cca ggt ggg cag gct gcc gag gag tcc tgc ttc tcc 549
Ala Phe Trp Gly Pro Gly Gly Gln Ala Ala Glu Glu Ser Cys Phe Ser
    130                 135                 140

tat gac atc cta cca ggc tgc aag gcc agc cgg tac agg gct gac atg 597
Tyr Asp Ile Leu Pro Gly Cys Lys Ala Ser Arg Tyr Arg Ala Asp Met
145                 150                 155                 160

tgt ggc gtt ctg caa tgt aaa ggt ggt caa caa cct tta ggt aga gct 645
Cys Gly Val Leu Gln Cys Lys Gly Gly Gln Gln Pro Leu Gly Arg Ala
                165                 170                 175

ata tgt att gtc gac gtg tgc cac gcg ctc acc aca gag gat ggc act 693
Ile Cys Ile Val Asp Val Cys His Ala Leu Thr Thr Glu Asp Gly Thr
            180                 185                 190

gcg tat gaa cca gtg ccc gag ggc acc cgg tgt gga cca gag aag gtt 741
Ala Tyr Glu Pro Val Pro Glu Gly Thr Arg Cys Gly Pro Glu Lys Val
        195                 200                 205

tgc tgg aaa gga cgt tgc cag gac tta cac gtt tac aga tcc agc aac 789
Cys Trp Lys Gly Arg Cys Gln Asp Leu His Val Tyr Arg Ser Ser Asn
    210                 215                 220

tgc tct gcc cag tgc cac aac cat ggg gtg tgc aac cac aag cag gag 837
Cys Ser Ala Gln Cys His Asn His Gly Val Cys Asn His Lys Gln Glu
225                 230                 235                 240

tgc cac tgc cac gcg ggc tgg gcc ccg ccc cac tgc gcg aag ctg ctg 885
Cys His Cys His Ala Gly Trp Ala Pro Pro His Cys Ala Lys Leu Leu
                245                 250                 255

act gag gtg cac gca gcg tcc ggg aga tct tgt gac aaa act cac aca 933
Thr Glu Val His Ala Ala Ser Gly Arg Ser Cys Asp Lys Thr His Thr
            260                 265                 270

tgc cca ccg tgc cca gca cct gaa gcc gag ggc gcg ccg tca gtc ttc 981
Cys Pro Pro Cys Pro Ala Pro Glu Ala Glu Gly Ala Pro Ser Val Phe
        275                 280                 285

ctc ttc ccc cca aaa ccc aag gac acc ctc atg atc tcc cgg acc cct 1029
Leu Phe Pro Pro Lys Pro Lys Asp Thr Leu Met Ile Ser Arg Thr Pro
    290                 295                 300

gag gtc aca tgc gtg gtg gtg gac gtg agc cac gaa gac cct gag gtc 1077
Glu Val Thr Cys Val Val Val Asp Val Ser His Glu Asp Pro Glu Val
305                 310                 315                 320

aag ttc aac tgg tac gtg gac ggc gtg gag gtg cat aat gcc aag aca 1125
Lys Phe Asn Trp Tyr Val Asp Gly Val Glu Val His Asn Ala Lys Thr
                325                 330                 335

aag ccg cgg gag gag cag tac aac agc acg tac cgt gtg gtc agc gtc 1173
Lys Pro Arg Glu Glu Gln Tyr Asn Ser Thr Tyr Arg Val Val Ser Val
            340                 345                 350

ctc acc gtc ctg cac cag gac tgg ctg aat ggc aag gag tac aag tgc 1221
Leu Thr Val Leu His Gln Asp Trp Leu Asn Gly Lys Glu Tyr Lys Cys
        355                 360                 365

aag gtc tcc aac aaa gcc ctc cca gcc ccc atc gag aaa acc atc tcc 1269
Lys Val Ser Asn Lys Ala Leu Pro Ala Pro Ile Glu Lys Thr Ile Ser
    370                 375                 380
aaa gcc aaa ggg cag ccc cga gaa cca cag gtg tac acc ctg ccc cca 1317
Lys Ala Lys Gly Gln Pro Arg Glu Pro Gln Val Tyr Thr Leu Pro Pro
385                 390                 395                 400

tcc cgg gag gag atg acc aag aac cag gtc agc ctg acc tgc ctg gtc 1365
Ser Arg Glu Glu Met Thr Lys Asn Gln Val Ser Leu Thr Cys Leu Val
                405                 410                 415

aaa ggc ttc tat ccc agc gac atc gcc gtg gag tgg gag agc aat ggg 1413
Lys Gly Phe Tyr Pro Ser Asp Ile Ala Val Glu Trp Glu Ser Asn Gly
            420                 425                 430

cag ccg gag aac aac tac aag acc acg cct ccc gtg ctg gac tcc gac 1461
Gln Pro Glu Asn Asn Tyr Lys Thr Thr Pro Pro Val Leu Asp Ser Asp
        435                 440                 445

ggc tcc ttc ttc ctc tat agc aag ctc acc gtg gac aag agc agg tgg 1509
Gly Ser Phe Phe Leu Tyr Ser Lys Leu Thr Val Asp Lys Ser Arg Trp
    450                 455                 460

cag cag ggg aac gtc ttc tca tgc tcc gtg atg cat gag gct ctg cac 1557
Gln Gln Gly Asn Val Phe Ser Cys Ser Val Met His Glu Ala Leu His
465                 470                 475                 480

aac cac tac acg cag aag agc ctc tcc ctg tct ccg ggt aaa tga 1602
Asn His Tyr Thr Gln Lys Ser Leu Ser Leu Ser Pro Gly Lys
485 490 495

actagagcgg ccgccaccgc ggtggagctc cagcttttgt tccctttagt gagggttaat 1662

ttcgagcttg gcgtaatcat ggtcatagct gtttcctg 1700

<210> 2
<211> 494
<212> PRT
<213> Artificial Sequence
<223> Description of Artificial Sequence: fusion
polypeptide

<400> 2
Met Glu Thr Asp Thr Leu Leu Leu Trp Val Leu Leu Leu Trp Val Pro
1               5                   10                  15
Gly Ser Thr Gly Thr Ser Cys Gly Asn Leu Phe Val Glu Arg Gly Glu
            20                  25                  30
Gln Cys Asp Cys Gly Pro Pro Glu Asp Cys Arg Asn Arg Cys Cys Asn
        35                  40                  45
Ser Thr Thr Cys Gln Leu Ala Glu Gly Ala Gln Cys Ala His Gly Thr
    50                  55                  60
Cys Cys Gln Glu Cys Lys Val Lys Pro Ala Gly Glu Leu Cys Arg Pro
65                  70                  75                  80
Lys Lys Asp Met Cys Asp Leu Glu Glu Phe Cys Asp Gly Arg His Pro
                85                  90                  95
Glu Cys Pro Glu Asp Ala Phe Gln Glu Asn Gly Thr Pro Cys Ser Gly
            100                 105                 110
Gly Tyr Cys Tyr Asn Gly Ala Cys Pro Thr Leu Ala Gln Gln Cys Gln
        115                 120                 125
Ala Phe Trp Gly Pro Gly Gly Gln Ala Ala Glu Glu Ser Cys Phe Ser
    130                 135                 140
Tyr Asp Ile Leu Pro Gly Cys Lys Ala Ser Arg Tyr Arg Ala Asp Met
145                 150                 155                 160
Cys Gly Val Leu Gln Cys Lys Gly Gly Gln Gln Pro Leu Gly Arg Ala
                165                 170                 175

Ile Cys Ile Val Asp Val Cys His Ala Leu Thr Thr Glu Asp Gly Thr
            180                 185                 190
Ala Tyr Glu Pro Val Pro Glu Gly Thr Arg Cys Gly Pro Glu Lys Val
        195                 200                 205
Cys Trp Lys Gly Arg Cys Gln Asp Leu His Val Tyr Arg Ser Ser Asn
    210                 215                 220
Cys Ser Ala Gln Cys His Asn His Gly Val Cys Asn His Lys Gln Glu
225                 230                 235                 240
Cys His Cys His Ala Gly Trp Ala Pro Pro His Cys Ala Lys Leu Leu
                245                 250                 255
Thr Glu Val His Ala Ala Ser Gly Arg Ser Cys Asp Lys Thr His Thr
            260                 265                 270
Cys Pro Pro Cys Pro Ala Pro Glu Ala Glu Gly Ala Pro Ser Val Phe
        275                 280                 285
Leu Phe Pro Pro Lys Pro Lys Asp Thr Leu Met Ile Ser Arg Thr Pro
    290                 295                 300
Glu Val Thr Cys Val Val Val Asp Val Ser His Glu Asp Pro Glu Val
305                 310                 315                 320
Lys Phe Asn Trp Tyr Val Asp Gly Val Glu Val His Asn Ala Lys Thr
                325                 330                 335
Lys Pro Arg Glu Glu Gln Tyr Asn Ser Thr Tyr Arg Val Val Ser Val
            340                 345                 350
Leu Thr Val Leu His Gln Asp Trp Leu Asn Gly Lys Glu Tyr Lys Cys
        355                 360                 365
Lys Val Ser Asn Lys Ala Leu Pro Ala Pro Ile Glu Lys Thr Ile Ser
    370                 375                 380
Lys Ala Lys Gly Gln Pro Arg Glu Pro Gln Val Tyr Thr Leu Pro Pro
385                 390                 395                 400
Ser Arg Glu Glu Met Thr Lys Asn Gln Val Ser Leu Thr Cys Leu Val
                405                 410                 415
Lys Gly Phe Tyr Pro Ser Asp Ile Ala Val Glu Trp Glu Ser Asn Gly
            420                 425                 430
Gln Pro Glu Asn Asn Tyr Lys Thr Thr Pro Pro Val Leu Asp Ser Asp
        435                 440                 445
Gly Ser Phe Phe Leu Tyr Ser Lys Leu Thr Val Asp Lys Ser Arg Trp
    450                 455                 460
Gln Gln Gly Asn Val Phe Ser Cys Ser Val Met His Glu Ala Leu His
465                 470                 475                 480
Asn His Tyr Thr Gln Lys Ser Leu Ser Leu Ser Pro Gly Lys
                485                 490

<210> 3
<211> 1668
<212> DNA
<213> Artificial Sequence

<220>
<223> Description of Artificial Sequence: fusion
      polypeptide

<220>
<221> CDS
<222> (46)..(1647)

<400> 3
ggtaccgggc ccccctcga ggtcgaccca agctggctag ccacc atg gag aca gac 57
                                                 Met Glu Thr Asp
                                                   1

aca ctc ctg cta tgg gta ctg ctg ctc tgg gtt cca ggt tcc act ggt   105
Thr Leu Leu Leu Trp Val Leu Leu Leu Trp Val Pro Gly Ser Thr Gly
  5                  10                  15                  20

act agt tgt ggt aat aag ttg gtg gac gct ggg gaa gag tgt gac tgt   153

Thr Ser Cys Gly Asn Lys Leu Val Asp Ala Gly Glu Glu Cys Asp Cys
                25                  30                  35

ggt act cca aag gaa tgt gaa ttg gac cct tgc tgc gaa gga agt acc 201
Gly Thr Pro Lys Glu Cys Glu Leu Asp Pro Cys Cys Glu Gly Ser Thr
            40                  45                  50

tgt aag ctt aaa tca ttt gct gag tgt gca tat ggt gac tgt tgt aaa 249
Cys Lys Leu Lys Ser Phe Ala Glu Cys Ala Tyr Gly Asp Cys Cys Lys
        55                  60                  65

gac tgt cgg ttc ctt cca gga ggt act tta tgc cga gga aaa acc agt 297
Asp Cys Arg Phe Leu Pro Gly Gly Thr Leu Cys Arg Gly Lys Thr Ser
    70                  75                  80

gag tgt gat gtt cca gag tac tgc aat ggt tct tct cag ttc tgt cag 345
Glu Cys Asp Val Pro Glu Tyr Cys Asn Gly Ser Ser Gln Phe Cys Gln
85                  90                  95                  100

cca gat gtt ttt att cag aat gga tat cct tgc cag aat aac aaa gcc 393
Pro Asp Val Phe Ile Gln Asn Gly Tyr Pro Cys Gln Asn Asn Lys Ala
                105                 110                 115

tat tgc tac aac ggc atg tgc cag tat tat gat gct caa tgt caa gtc 441
Tyr Cys Tyr Asn Gly Met Cys Gln Tyr Tyr Asp Ala Gln Cys Gln Val
            120                 125                 130

atc ttt ggc tca aaa gcc aag gct gcc ccc aaa gat tgt ttc att gaa 489
Ile Phe Gly Ser Lys Ala Lys Ala Ala Pro Lys Asp Cys Phe Ile Glu
        135                 140                 145

gtg aat tct aaa ggt gac aga ttt ggc aat tgt ggt ttc tct ggc aat 537
Val Asn Ser Lys Gly Asp Arg Phe Gly Asn Cys Gly Phe Ser Gly Asn
    150                 155                 160

gaa tac aag aag tgt gcc act ggg aat gct ttg tgt gga aag ctt cag 585
Glu Tyr Lys Lys Cys Ala Thr Gly Asn Ala Leu Cys Gly Lys Leu Gln
165                 170                 175                 180

tgt gag aat gta caa gag ata cct gta ttt gga att gtg cct gct att 633
Cys Glu Asn Val Gln Glu Ile Pro Val Phe Gly Ile Val Pro Ala Ile
                185                 190                 195

att caa acg cct agt cga ggc acc aaa tgt tgg ggt gtg gat ttc cag 681
Ile Gln Thr Pro Ser Arg Gly Thr Lys Cys Trp Gly Val Asp Phe Gln
            200                 205                 210

cta gga tca gat gtt cca gat cct ggg atg gtt aac gaa ggc aca aaa 729
Leu Gly Ser Asp Val Pro Asp Pro Gly Met Val Asn Glu Gly Thr Lys
        215                 220                 225

tgt ggt gct gga aag atc tgt aga aac ttc cag tgt gta gat gct tct 777
Cys Gly Ala Gly Lys Ile Cys Arg Asn Phe Gln Cys Val Asp Ala Ser
    230                 235                 240

gtt ctg aat tat gac tgt gat gtt cag aaa aag tgt cat gga cat ggg 825
Val Leu Asn Tyr Asp Cys Asp Val Gln Lys Lys Cys His Gly His Gly
245                 250                 255                 260

gta tgt aat agc aat aag aat tgt cac tgt gaa aat ggc tgg gct ccc 873
Val Cys Asn Ser Asn Lys Asn Cys His Cys Glu Asn Gly Trp Ala Pro
                265                 270                 275

cca aat tgt gag act aaa gga tac gga gga agt gtg gac agt gga cct 921
Pro Asn Cys Glu Thr Lys Gly Tyr Gly Gly Ser Val Asp Ser Gly Pro
            280                 285                 290

aca tac aat gaa atg aat act gca ttg agg gac gga tct tgt gac aaa    969
Thr Tyr Asn Glu Met Asn Thr Ala Leu Arg Asp Gly Ser Cys Asp Lys
        295                 300                 305

act cac aca tgc cca ccg tgc cca gca cct gaa gcc gag ggc gcg ccg    1017
Thr His Thr Cys Pro Pro Cys Pro Ala Pro Glu Ala Glu Gly Ala Pro
    310                 315                 320

tca gtc ttc ctc ttc ccc cca aaa ccc aag gac acc ctc atg atc tcc    1065
Ser Val Phe Leu Phe Pro Pro Lys Pro Lys Asp Thr Leu Met Ile Ser
325                 330                 335                 340

cgg acc cct gag gtc aca tgc gtg gtg gtg gac gtg agc cac gaa gac    1113
Arg Thr Pro Glu Val Thr Cys Val Val Val Asp Val Ser His Glu Asp
                345                 350                 355

cct gag gtc aag ttc aac tgg tac gtg gac ggc gtg gag gtg cat aat    1161
Pro Glu Val Lys Phe Asn Trp Tyr Val Asp Gly Val Glu Val His Asn
            360                 365                 370

gcc aag aca aag ccg cgg gag gag cag tac aac agc acg tac cgg gtg    1209
Ala Lys Thr Lys Pro Arg Glu Glu Gln Tyr Asn Ser Thr Tyr Arg Val
        375                 380                 385

gtc agc gtc ctc acc gtc ctg cac cag gac tgg ctg aat ggc aag gag    1257
Val Ser Val Leu Thr Val Leu His Gln Asp Trp Leu Asn Gly Lys Glu
    390                 395                 400

tac aag tgc aag gtc tcc aac aaa gcc ctc cca gcc ccc atc gag aaa    1305
Tyr Lys Cys Lys Val Ser Asn Lys Ala Leu Pro Ala Pro Ile Glu Lys
405                 410                 415                 420

acc atc tcc aaa gcc aaa ggg cag ccc cga gaa cca cag gtg tac acc    1353
Thr Ile Ser Lys Ala Lys Gly Gln Pro Arg Glu Pro Gln Val Tyr Thr
                425                 430                 435

ctg ccc cca tcc cgg gat gag ctg acc aag aac cag gtc agc ctg acc    1401
Leu Pro Pro Ser Arg Asp Glu Leu Thr Lys Asn Gln Val Ser Leu Thr
            440                 445                 450

tgc ctg gtc aaa ggc ttc tat ccc agc gac atc gcc gtg gag tgg gag    1449
Cys Leu Val Lys Gly Phe Tyr Pro Ser Asp Ile Ala Val Glu Trp Glu
        455                 460                 465

agc aat ggg cag ccg gag aac aac tac aag acc acg cct ccc gtg ctg    1497
Ser Asn Gly Gln Pro Glu Asn Asn Tyr Lys Thr Thr Pro Pro Val Leu
    470                 475                 480

gac tcc gac ggc tcc ttc ttc ctc tac agc aag ctc acc gtg gac aag    1545
Asp Ser Asp Gly Ser Phe Phe Leu Tyr Ser Lys Leu Thr Val Asp Lys
485                 490                 495                 500

agc agg tgg cag cag ggg aac gtc ttc tca tgc tcc gtg atg cat gag    1593
Ser Arg Trp Gln Gln Gly Asn Val Phe Ser Cys Ser Val Met His Glu
                505                 510                 515

gct ctg cac aac cac tac acg cag aag agc ctc tcc ctg tct ccg ggt    1641
Ala Leu His Asn His Tyr Thr Gln Lys Ser Leu Ser Leu Ser Pro Gly
            520                 525                 530

aaa tga actagagcgg ccgctacaga t                                    1668
Lys

<210> 4

<211> 533
<212> PRT
<213> Artificial Sequence
<223> Description of Artificial Sequence: fusion
      polypeptide

<400> 4
Met Glu Thr Asp Thr Leu Leu Leu Trp Val Leu Leu Leu Trp Val Pro
  1               5                  10                  15
Gly Ser Thr Gly Thr Ser Cys Gly Asn Lys Leu Val Asp Ala Gly Glu
             20                  25                  30
Glu Cys Asp Cys Gly Thr Pro Lys Glu Cys Glu Leu Asp Pro Cys Cys
         35                  40                  45
Glu Gly Ser Thr Cys Lys Leu Lys Ser Phe Ala Glu Cys Ala Tyr Gly
     50                  55                  60
Asp Cys Cys Lys Asp Cys Arg Phe Leu Pro Gly Gly Thr Leu Cys Arg
 65                  70                  75                  80
Gly Lys Thr Ser Glu Cys Asp Val Pro Glu Tyr Cys Asn Gly Ser Ser
                 85                  90                  95
Gln Phe Cys Gln Pro Asp Val Phe Ile Gln Asn Gly Tyr Pro Cys Gln
            100                 105                 110
Asn Asn Lys Ala Tyr Cys Tyr Asn Gly Met Cys Gln Tyr Tyr Asp Ala
        115                 120                 125
Gln Cys Gln Val Ile Phe Gly Ser Lys Ala Lys Ala Ala Pro Lys Asp
    130                 135                 140
Cys Phe Ile Glu Val Asn Ser Lys Gly Asp Arg Phe Gly Asn Cys Gly
145                 150                 155                 160
Phe Ser Gly Asn Glu Tyr Lys Lys Cys Ala Thr Gly Asn Ala Leu Cys
                165                 170                 175
Gly Lys Leu Gln Cys Glu Asn Val Gln Glu Ile Pro Val Phe Gly Ile
            180                 185                 190
Val Pro Ala Ile Ile Gln Thr Pro Ser Arg Gly Thr Lys Cys Trp Gly
        195                 200                 205
Val Asp Phe Gln Leu Gly Ser Asp Val Pro Asp Pro Gly Met Val Asn
    210                 215                 220
Glu Gly Thr Lys Cys Gly Ala Gly Lys Ile Cys Arg Asn Phe Gln Cys
225                 230                 235                 240
Val Asp Ala Ser Val Leu Asn Tyr Asp Cys Asp Val Gln Lys Lys Cys
                245                 250                 255
His Gly His Gly Val Cys Asn Ser Asn Lys Asn Cys His Cys Glu Asn
            260                 265                 270
Gly Trp Ala Pro Pro Asn Cys Glu Thr Lys Gly Tyr Gly Gly Ser Val
        275                 280                 285
Asp Ser Gly Pro Thr Tyr Asn Glu Met Asn Thr Ala Leu Arg Asp Gly
    290                 295                 300
Ser Cys Asp Lys Thr His Thr Cys Pro Pro Cys Pro Ala Pro Glu Ala
305                 310                 315                 320
Glu Gly Ala Pro Ser Val Phe Leu Phe Pro Pro Lys Pro Lys Asp Thr
                325                 330                 335
Leu Met Ile Ser Arg Thr Pro Glu Val Thr Cys Val Val Val Asp Val
            340                 345                 350
Ser His Glu Asp Pro Glu Val Lys Phe Asn Trp Tyr Val Asp Gly Val
        355                 360                 365
Glu Val His Asn Ala Lys Thr Lys Pro Arg Glu Glu Gln Tyr Asn Ser
    370                 375                 380
Thr Tyr Arg Val Val Ser Val Leu Thr Val Leu His Gln Asp Trp Leu
385                 390                 395                 400
Asn Gly Lys Glu Tyr Lys Cys Lys Val Ser Asn Lys Ala Leu Pro Ala
                405                 410                 415
Pro Ile Glu Lys Thr Ile Ser Lys Ala Lys Gly Gln Pro Arg Glu Pro
            420                 425                 430
Gln Val Tyr Thr Leu Pro Pro Ser Arg Asp Glu Leu Thr Lys Asn Gln
        435                 440                 445
Val Ser Leu Thr Cys Leu Val Lys Gly Phe Tyr Pro Ser Asp Ile Ala
    450                 455                 460

Val Glu Trp Glu Ser Asn Gly Gln Pro Glu Asn Asn Tyr Lys Thr Thr
465                 470                 475                 480
Pro Pro Val Leu Asp Ser Asp Gly Ser Phe Phe Leu Tyr Ser Lys Leu
                485                 490                 495
Thr Val Asp Lys Ser Arg Trp Gln Gln Gly Asn Val Phe Ser Cys Ser
            500                 505                 510
Val Met His Glu Ala Leu His Asn His Tyr Thr Gln Lys Ser Leu Ser
        515                 520                 525
Leu Ser Pro Gly Lys
    530


<210> 5
<211> 1443
<212> DNA
<213> Artificial Sequence

<220>
<223> Description of Artificial Sequence: fusion
      polypeptide

<220>
<221> CDS
<222> (25)..(1422)

<400> 5
gtcgacccaa gctggctagc cacc atg gag aca gac aca ctc ctg cta tgg     51
                           Met Glu Thr Asp Thr Leu Leu Leu Trp
                             1               5

gta ctg ctg ctc tgg gtt cca ggt tcc act ggt act agt tgt gga aat     99
Val Leu Leu Leu Trp Val Pro Gly Ser Thr Gly Thr Ser Cys Gly Asn
 10                  15                  20                  25

gga atg gta gaa caa ggt gaa gaa tgt gat tgt ggc tat agt gac cag    147
Gly Met Val Glu Gln Gly Glu Glu Cys Asp Cys Gly Tyr Ser Asp Gln
                 30                  35                  40

tgt aaa gat gaa tgc tgc ttc gat gca aat caa cca gag gga aga aaa    195
Cys Lys Asp Glu Cys Cys Phe Asp Ala Asn Gln Pro Glu Gly Arg Lys
             45                  50                  55

tgc aaa ctg aaa cct ggg aaa cag tgc agt cca agt caa ggt cct tgt    243
Cys Lys Leu Lys Pro Gly Lys Gln Cys Ser Pro Ser Gln Gly Pro Cys
         60                  65                  70

tgt aca gca cag tgt gca ttc aag tca aag tct gag aag tgt cgg gat    291
Cys Thr Ala Gln Cys Ala Phe Lys Ser Lys Ser Glu Lys Cys Arg Asp
     75                  80                  85

gat tca gac tgt gca agg gaa gga ata tgt aat ggc ttc aca gct ctc    339
Asp Ser Asp Cys Ala Arg Glu Gly Ile Cys Asn Gly Phe Thr Ala Leu
 90                  95                 100                 105

tgc cca gca tct gac cct aaa cca aac ttc aca gac tgt aat agg cat    387
Cys Pro Ala Ser Asp Pro Lys Pro Asn Phe Thr Asp Cys Asn Arg His
                110                 115                 120

aca caa gtg tgc att aat ggg caa tgt gca ggt tct atc tgt gag aaa    435
Thr Gln Val Cys Ile Asn Gly Gln Cys Ala Gly Ser Ile Cys Glu Lys
            125                 130                 135

tat ggc tta gag gag tgt acg tgt gcc agt tct gat ggc aaa gat gat    483
Tyr Gly Leu Glu Glu Cys Thr Cys Ala Ser Ser Asp Gly Lys Asp Asp
        140                 145                 150

aaa gaa tta tgc cat gta tgc tgt atg aag aaa atg gac cca tca act    531

Lys Glu Leu Cys His Val Cys Cys Met Lys Lys Met Asp Pro Ser Thr
    155                 160                 165

tgt gcc agt aca ggg tct gtg cag tgg agt agg cac ttc agt ggt cga    579
Cys Ala Ser Thr Gly Ser Val Gln Trp Ser Arg His Phe Ser Gly Arg
170                 175                 180                 185

acc atc acc ctg caa cct gga tcc cct tgc aac gat ttt aga ggt tac    627
Thr Ile Thr Leu Gln Pro Gly Ser Pro Cys Asn Asp Phe Arg Gly Tyr
                190                 195                 200

tgt gat gtt ttc atg cgg tgc aga tta gta gat gct gat ggt cct cta    675
Cys Asp Val Phe Met Arg Cys Arg Leu Val Asp Ala Asp Gly Pro Leu
            205                 210                 215

gct agg ctt aaa aaa gca att ttt agt cca gag ctc tat gaa aac att    723
Ala Arg Leu Lys Lys Ala Ile Phe Ser Pro Glu Leu Tyr Glu Asn Ile
        220                 225                 230

gct gaa aga tct tgt gac aaa act cac aca tgc cca ccg tgc cca gca    771
Ala Glu Arg Ser Cys Asp Lys Thr His Thr Cys Pro Pro Cys Pro Ala
    235                 240                 245

cct gaa gcc gag ggc gcg ccg tca gtc ttc ctc ttc ccc cca aaa ccc    819
Pro Glu Ala Glu Gly Ala Pro Ser Val Phe Leu Phe Pro Pro Lys Pro
250                 255                 260                 265

aag gac acc ctc atg atc tcc cgg acc cct gag gtc aca tgc gtg gtg    867
Lys Asp Thr Leu Met Ile Ser Arg Thr Pro Glu Val Thr Cys Val Val
                270                 275                 280

gtg gac gtg agc cac gaa gac cct gag gtc aag ttc aac tgg tac gtg    915
Val Asp Val Ser His Glu Asp Pro Glu Val Lys Phe Asn Trp Tyr Val
            285                 290                 295

gac ggc gtg gag gtg cat aat gcc aag aca aag ccg cgg gag gag cag    963
Asp Gly Val Glu Val His Asn Ala Lys Thr Lys Pro Arg Glu Glu Gln
        300                 305                 310

tac aac agc acg tac cgg gtg gtc agc gtc ctc acc gtc ctg cac cag    1011
Tyr Asn Ser Thr Tyr Arg Val Val Ser Val Leu Thr Val Leu His Gln
    315                 320                 325

gac tgg ctg aat ggc aag gag tac aag tgc aag gtc tcc aac aaa gcc    1059
Asp Trp Leu Asn Gly Lys Glu Tyr Lys Cys Lys Val Ser Asn Lys Ala
330                 335                 340                 345

ctc cca gcc ccc atc gag aaa acc atc tcc aaa gcc aaa ggg cag ccc    1107
Leu Pro Ala Pro Ile Glu Lys Thr Ile Ser Lys Ala Lys Gly Gln Pro
                350                 355                 360

cga gaa cca cag gtg tac acc ctg ccc cca tcc cgg gat gag ctg acc    1155
Arg Glu Pro Gln Val Tyr Thr Leu Pro Pro Ser Arg Asp Glu Leu Thr
            365                 370                 375

aag aac cag gtc agc ctg acc tgc ctg gtc aaa ggc ttc tat ccc agc    1203
Lys Asn Gln Val Ser Leu Thr Cys Leu Val Lys Gly Phe Tyr Pro Ser
        380                 385                 390

gac atc gcc gtg gag tgg gag agc aat ggg cag ccg gag aac aac tac    1251
Asp Ile Ala Val Glu Trp Glu Ser Asn Gly Gln Pro Glu Asn Asn Tyr
    395                 400                 405

aag acc acg cct ccc gtg ctg gac tcc gac ggc tcc ttc ttc ctc tac    1299

Lys Thr Thr Pro Pro Val Leu Asp Ser Asp Gly Ser Phe Phe Leu Tyr
410                 415                 420                 425

agc aag ctc acc gtg gac aag agc agg tgg cag cag ggg aac gtc ttc     1347
Ser Lys Leu Thr Val Asp Lys Ser Arg Trp Gln Gln Gly Asn Val Phe
                430                 435                 440

tca tgc tcc gtg atg cat gag gct ctg cac aac cac tac acg cag aag     1395
Ser Cys Ser Val Met His Glu Ala Leu His Asn His Tyr Thr Gln Lys
            445                 450                 455

agc ctc tcc ctg tct ccg ggt aaa tga actagagcgg ccgctacaga t         1443
Ser Leu Ser Leu Ser Pro Gly Lys
        460                 465

<210> 6
<211> 465
<212> PRT
<213> Artificial Sequence
<223> Description of Artificial Sequence: fusion
      polypeptide

<400> 6
Met Glu Thr Asp Thr Leu Leu Leu Trp Val Leu Leu Leu Trp Val Pro
  1               5                  10                  15
Gly Ser Thr Gly Thr Ser Cys Gly Asn Gly Met Val Glu Gln Gly Glu
             20                  25                  30
Glu Cys Asp Cys Gly Tyr Ser Asp Gln Cys Lys Asp Glu Cys Cys Phe
         35                  40                  45
Asp Ala Asn Gln Pro Glu Gly Arg Lys Cys Lys Leu Lys Pro Gly Lys
     50                  55                  60
Gln Cys Ser Pro Ser Gln Gly Pro Cys Cys Thr Ala Gln Cys Ala Phe
 65                  70                  75                  80
Lys Ser Lys Ser Glu Lys Cys Arg Asp Asp Ser Asp Cys Ala Arg Glu
                 85                  90                  95
Gly Ile Cys Asn Gly Phe Thr Ala Leu Cys Pro Ala Ser Asp Pro Lys
            100                 105                 110
Pro Asn Phe Thr Asp Cys Asn Arg His Thr Gln Val Cys Ile Asn Gly
        115                 120                 125
Gln Cys Ala Gly Ser Ile Cys Glu Lys Tyr Gly Leu Glu Glu Cys Thr
    130                 135                 140
Cys Ala Ser Ser Asp Gly Lys Asp Asp Lys Glu Leu Cys His Val Cys
145                 150                 155                 160
Cys Met Lys Lys Met Asp Pro Ser Thr Cys Ala Ser Thr Gly Ser Val
                165                 170                 175
Gln Trp Ser Arg His Phe Ser Gly Arg Thr Ile Thr Leu Gln Pro Gly
            180                 185                 190
Ser Pro Cys Asn Asp Phe Arg Gly Tyr Cys Asp Val Phe Met Arg Cys
        195                 200                 205
Arg Leu Val Asp Ala Asp Gly Pro Leu Ala Arg Leu Lys Lys Ala Ile
    210                 215                 220
Phe Ser Pro Glu Leu Tyr Glu Asn Ile Ala Glu Arg Ser Cys Asp Lys
225                 230                 235                 240
Thr His Thr Cys Pro Pro Cys Pro Ala Pro Glu Ala Glu Gly Ala Pro
                245                 250                 255
Ser Val Phe Leu Phe Pro Pro Lys Pro Lys Asp Thr Leu Met Ile Ser
            260                 265                 270
Arg Thr Pro Glu Val Thr Cys Val Val Val Asp Val Ser His Glu Asp
        275                 280                 285
Pro Glu Val Lys Phe Asn Trp Tyr Val Asp Gly Val Glu Val His Asn
    290                 295                 300
Ala Lys Thr Lys Pro Arg Glu Glu Gln Tyr Asn Ser Thr Tyr Arg Val
305                 310                 315                 320
Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350
Thr Ile Ser Lys Ala Lys Gly Gln Pro Arg Glu Pro Gln Val Tyr Thr
        355                 360                 365
Leu Pro Pro Ser Arg Asp Glu Leu Thr Lys Asn Gln Val Ser Leu Thr
    370                 375                 380
Cys Leu Val Lys Gly Phe Tyr Pro Ser Asp Ile Ala Val Glu Trp Glu
385                 390                 395                 400
Ser Asn Gly Gln Pro Glu Asn Asn Tyr Lys Thr Thr Pro Pro Val Leu
                405                 410                 415
Asp Ser Asp Gly Ser Phe Phe Leu Tyr Ser Lys Leu Thr Val Asp Lys
            420                 425                 430
Ser Arg Trp Gln Gln Gly Asn Val Phe Ser Cys Ser Val Met His Glu
        435                 440                 445
Ala Leu His Asn His Tyr Thr Gln Lys Ser Leu Ser Leu Ser Pro Gly
    450                 455                 460
Lys
465

<210> 7
<211> 1638
<212> DNA
<213> Artificial Sequence

<220>
<223> Description of Artificial Sequence: fusion
      polypeptide

<220>
<221> CDS
<222> (41)..(1609)

<400> 7
cgggcccccc ctcgaggtcg acccaagctg gctagccacc atg gag aca gac aca    55
                                            Met Glu Thr Asp Thr
                                              1               5

ctc ctg cta tgg gta ctg ctg ctc tgg gtt cca ggt tcc act ggt act    103
Leu Leu Leu Trp Val Leu Leu Leu Trp Val Pro Gly Ser Thr Gly Thr
                 10                  15                  20

agt tgc gga aat atg ttt gtg gag ccg ggc gag cag tgt gac tgt ggc    151
Ser Cys Gly Asn Met Phe Val Glu Pro Gly Glu Gln Cys Asp Cys Gly
             25                  30                  35

ttc ctg gat gac tgc gtc gat ccc tgc tgt gat tct ttg acc tgc cag    199
Phe Leu Asp Asp Cys Val Asp Pro Cys Cys Asp Ser Leu Thr Cys Gln
         40                  45                  50

ctg agg cca ggt gca cag tgt gca tct gac gga ccc tgt tgt caa aat    247
Leu Arg Pro Gly Ala Gln Cys Ala Ser Asp Gly Pro Cys Cys Gln Asn
     55                  60                  65

tgc cag ctg cgc ccg tct ggc tgg cag tgt cgt cct acc aga ggg gat    295
Cys Gln Leu Arg Pro Ser Gly Trp Gln Cys Arg Pro Thr Arg Gly Asp
 70                  75                  80                  85

tgt gac ttg cct gaa ttc tgc cca gga gac agc tcc cag tgt ccc cct    343
Cys Asp Leu Pro Glu Phe Cys Pro Gly Asp Ser Ser Gln Cys Pro Pro
                 90                  95                 100

gat gtc agc cta ggg gat ggc gag ccc tgc gct ggc ggg caa gct gtg    391
Asp Val Ser Leu Gly Asp Gly Glu Pro Cys Ala Gly Gly Gln Ala Val
            105                 110                 115

tgc atg cac ggg cgt tgt gcc tcc tat gcc cag cag tgc cag tca ctt 439

Cys Met His Gly Arg Cys Ala Ser Tyr Ala Gln Gln Cys Gln Ser Leu
        120                 125                 130

tgg gga cct gga gcc cag ccc gct gcg cca ctt tgc ctc cag aca gct 487
Trp Gly Pro Gly Ala Gln Pro Ala Ala Pro Leu Cys Leu Gln Thr Ala
    135                 140                 145

aat act cgg gga aat gct ttt ggg agc tgt ggg cgc aac ccc agt ggc 535
Asn Thr Arg Gly Asn Ala Phe Gly Ser Cys Gly Arg Asn Pro Ser Gly
150                 155                 160                 165

agt tat gtg tcc tgc acc cct aga gat gcc att tgt ggg cag ctc cag 583
Ser Tyr Val Ser Cys Thr Pro Arg Asp Ala Ile Cys Gly Gln Leu Gln
                170                 175                 180

tgc cag aca ggt agg acc cag cct ctg ctg ggc tcc atc cgg gat cta 631
Cys Gln Thr Gly Arg Thr Gln Pro Leu Leu Gly Ser Ile Arg Asp Leu
            185                 190                 195

ctc tgg gag aca ata gat gtg aat ggg act gag ctg aac tgc agc tgg 679
Leu Trp Glu Thr Ile Asp Val Asn Gly Thr Glu Leu Asn Cys Ser Trp
        200                 205                 210

gtg cac ctg gac ctg ggc agt gat gtg gcc cag ccc ctc ctg act ctg 727
Val His Leu Asp Leu Gly Ser Asp Val Ala Gln Pro Leu Leu Thr Leu
    215                 220                 225

cct ggc aca gcc tgt ggc cct ggc ctg gtg tgt ata gac cat cga tgc 775
Pro Gly Thr Ala Cys Gly Pro Gly Leu Val Cys Ile Asp His Arg Cys
230                 235                 240                 245

cag cgt gtg gat ctc ctg ggg gca cag gaa tgt cga agc aaa tgc cat 823
Gln Arg Val Asp Leu Leu Gly Ala Gln Glu Cys Arg Ser Lys Cys His
                250                 255                 260

gga cat ggg gtc tgt gac agc aac agg cac tgc tac tgt gag gag ggc 871
Gly His Gly Val Cys Asp Ser Asn Arg His Cys Tyr Cys Glu Glu Gly
            265                 270                 275

tgg gca ccc cct gac tgc acc act cag ctc aaa gca acc agc tcc aga 919
Trp Ala Pro Pro Asp Cys Thr Thr Gln Leu Lys Ala Thr Ser Ser Arg
        280                 285                 290

tct tgt gac aaa act cac aca tgc cca ccg tgc cca gca cct gaa gcc 967
Ser Cys Asp Lys Thr His Thr Cys Pro Pro Cys Pro Ala Pro Glu Ala
    295                 300                 305

gag ggc gcg ccg tca gtc ttc ctc ttc ccc cca aaa ccc aag gac acc 1015
Glu Gly Ala Pro Ser Val Phe Leu Phe Pro Pro Lys Pro Lys Asp Thr
310                 315                 320                 325

ctc atg atc tcc cgg acc cct gag gtc aca tgc gtg gtg gtg gac gtg 1063
Leu Met Ile Ser Arg Thr Pro Glu Val Thr Cys Val Val Val Asp Val
                330                 335                 340

agc cac gaa gac cct gag gtc aag ttc aac tgg tac gtg gac ggc gtg 1111
Ser His Glu Asp Pro Glu Val Lys Phe Asn Trp Tyr Val Asp Gly Val
            345                 350                 355

gag gtg cat aat gcc aag aca aag ccg cgg gag gag cag tac aac agc 1159
Glu Val His Asn Ala Lys Thr Lys Pro Arg Glu Glu Gln Tyr Asn Ser
        360                 365                 370

acg tac cgt gtg gtc agc gtc ctc acc gtc ctg cac cag gac tgg ctg 1207

Thr Tyr Arg Val Val Ser Val Leu Thr Val Leu His Gln Asp Trp Leu
    375                 380                 385

aat ggc aag gag tac aag tgc aag gtc tcc aac aaa gcc ctc cca gcc    1255
Asn Gly Lys Glu Tyr Lys Cys Lys Val Ser Asn Lys Ala Leu Pro Ala
390                 395                 400                 405

ccc atc gag aaa acc atc tcc aaa gcc aaa ggg cag ccc cga gaa cca    1303
Pro Ile Glu Lys Thr Ile Ser Lys Ala Lys Gly Gln Pro Arg Glu Pro
                410                 415                 420

cag gtg tac acc ctg ccc cca tcc cgg gag gag atg acc aag aac cag    1351
Gln Val Tyr Thr Leu Pro Pro Ser Arg Glu Glu Met Thr Lys Asn Gln
            425                 430                 435

gtc agc ctg acc tgc ctg gtc aaa ggc ttc tat ccc agc gac atc gcc    1399
Val Ser Leu Thr Cys Leu Val Lys Gly Phe Tyr Pro Ser Asp Ile Ala
        440                 445                 450

gtg gag tgg gag agc aat ggg cag ccg gag aac aac tac aag acc acg    1447
Val Glu Trp Glu Ser Asn Gly Gln Pro Glu Asn Asn Tyr Lys Thr Thr
    455                 460                 465

cct ccc gtg ctg gac tcc gac ggc tcc ttc ttc ctc tat agc aag ctc    1495
Pro Pro Val Leu Asp Ser Asp Gly Ser Phe Phe Leu Tyr Ser Lys Leu
470                 475                 480                 485

acc gtg gac aag agc agg tgg cag cag ggg aac gtc ttc tca tgc tcc    1543
Thr Val Asp Lys Ser Arg Trp Gln Gln Gly Asn Val Phe Ser Cys Ser
                490                 495                 500

gtg atg cat gag gct ctg cac aac cac tac acg cag aag agc ctc tcc    1591
Val Met His Glu Ala Leu His Asn His Tyr Thr Gln Lys Ser Leu Ser
            505                 510                 515

ctg tct ccg ggt aaa tga actagagcgg ccgccaccgc ggtggagct            1638
Leu Ser Pro Gly Lys
        520


<210> 8
<211> 522
<212> PRT
<213> Artificial Sequence
<223> Description of Artificial Sequence: fusion
      polypeptide

<400> 8
Met Glu Thr Asp Thr Leu Leu Leu Trp Val Leu Leu Leu Trp Val Pro
  1               5                  10                  15
Gly Ser Thr Gly Thr Ser Cys Gly Asn Met Phe Val Glu Pro Gly Glu
             20                  25                  30
Gln Cys Asp Cys Gly Phe Leu Asp Asp Cys Val Asp Pro Cys Cys Asp
         35                  40                  45
Ser Leu Thr Cys Gln Leu Arg Pro Gly Ala Gln Cys Ala Ser Asp Gly
     50                  55                  60
Pro Cys Cys Gln Asn Cys Gln Leu Arg Pro Ser Gly Trp Gln Cys Arg
 65                  70                  75                  80
Pro Thr Arg Gly Asp Cys Asp Leu Pro Glu Phe Cys Pro Gly Asp Ser
                 85                  90                  95
Ser Gln Cys Pro Pro Asp Val Ser Leu Gly Asp Gly Glu Pro Cys Ala
            100                 105                 110
Gly Gly Gln Ala Val Cys Met His Gly Arg Cys Ala Ser Tyr Ala Gln
        115                 120                 125
Gln Cys Gln Ser Leu Trp Gly Pro Gly Ala Gln Pro Ala Ala Pro Leu
    130                 135                 140

Cys Leu Gln Thr Ala Asn Thr Arg Gly Asn Ala Phe Gly Ser Cys Gly
145                 150                 155                 160
Arg Asn Pro Ser Gly Ser Tyr Val Ser Cys Thr Pro Arg Asp Ala Ile
                165                 170                 175
Cys Gly Gln Leu Gln Cys Gln Thr Gly Arg Thr Gln Pro Leu Leu Gly
            180                 185                 190
Ser Ile Arg Asp Leu Leu Trp Glu Thr Ile Asp Val Asn Gly Thr Glu
        195                 200                 205
Leu Asn Cys Ser Trp Val His Leu Asp Leu Gly Ser Asp Val Ala Gln
    210                 215                 220
Pro Leu Leu Thr Leu Pro Gly Thr Ala Cys Gly Pro Gly Leu Val Cys
225                 230                 235                 240
Ile Asp His Arg Cys Gln Arg Val Asp Leu Leu Gly Ala Gln Glu Cys
                245                 250                 255
Arg Ser Lys Cys His Gly His Gly Val Cys Asp Ser Asn Arg His Cys
            260                 265                 270
Tyr Cys Glu Glu Gly Trp Ala Pro Pro Asp Cys Thr Thr Gln Leu Lys
        275                 280                 285
Ala Thr Ser Ser Arg Ser Cys Asp Lys Thr His Thr Cys Pro Pro Cys
    290                 295                 300
Pro Ala Pro Glu Ala Glu Gly Ala Pro Ser Val Phe Leu Phe Pro Pro
305                 310                 315                 320
Lys Pro Lys Asp Thr Leu Met Ile Ser Arg Thr Pro Glu Val Thr Cys
                325                 330                 335
Val Val Val Asp Val Ser His Glu Asp Pro Glu Val Lys Phe Asn Trp
            340                 345                 350
Tyr Val Asp Gly Val Glu Val His Asn Ala Lys Thr Lys Pro Arg Glu
        355                 360                 365
Glu Gln Tyr Asn Ser Thr Tyr Arg Val Val Ser Val Leu Thr Val Leu
    370                 375                 380
His Gln Asp Trp Leu Asn Gly Lys Glu Tyr Lys Cys Lys Val Ser Asn
385                 390                 395                 400
Lys Ala Leu Pro Ala Pro Ile Glu Lys Thr Ile Ser Lys Ala Lys Gly
                405                 410                 415
Gln Pro Arg Glu Pro Gln Val Tyr Thr Leu Pro Pro Ser Arg Glu Glu
            420                 425                 430
Met Thr Lys Asn Gln Val Ser Leu Thr Cys Leu Val Lys Gly Phe Tyr
        435                 440                 445
Pro Ser Asp Ile Ala Val Glu Trp Glu Ser Asn Gly Gln Pro Glu Asn
    450                 455                 460
Asn Tyr Lys Thr Thr Pro Pro Val Leu Asp Ser Asp Gly Ser Phe Phe
465                 470                 475                 480
Leu Tyr Ser Lys Leu Thr Val Asp Lys Ser Arg Trp Gln Gln Gly Asn
                485                 490                 495
Val Phe Ser Cys Ser Val Met His Glu Ala Leu His Asn His Tyr Thr
            500                 505                 510
Gln Lys Ser Leu Ser Leu Ser Pro Gly Lys
        515                 520

<210> 9
<211> 1386
<212> DNA
<213> Artificial Sequence

<220>
<223> Description of Artificial Sequence: fusion
      polypeptide

<220>
<221> CDS
<222> (25)..(1365)

<400> 9
gtcgacccaa gctggctagc cacc atg gag aca gac aca ctc ctg cta tgg     51

Met Glu Thr Asp Thr Leu Leu Leu Trp
1               5

gta ctg ctg ctc tgg gtt cca ggt tcc act ggt act agt tgt ggg aac    99
Val Leu Leu Leu Trp Val Pro Gly Ser Thr Gly Thr Ser Cys Gly Asn
10                  15                  20                  25

tcg agg gtg gat gaa gga gaa gag tgt gat cct ggc atc atg tat ctg    147
Ser Arg Val Asp Glu Gly Glu Glu Cys Asp Pro Gly Ile Met Tyr Leu
                30                  35                  40

aac aac gac acc tgc tgc aac agc gac tgc acg ttg aag gaa ggt gtc    195
Asn Asn Asp Thr Cys Cys Asn Ser Asp Cys Thr Leu Lys Glu Gly Val
            45                  50                  55

cag tgc agt gac agg aac agt cct tgc tgt aaa aac tgt cag ttt gag    243
Gln Cys Ser Asp Arg Asn Ser Pro Cys Cys Lys Asn Cys Gln Phe Glu
        60                  65                  70

act gcc cag aag aag tgc cag gag gcg att aat gct act tgc aaa ggc    291
Thr Ala Gln Lys Lys Cys Gln Glu Ala Ile Asn Ala Thr Cys Lys Gly
    75                  80                  85

gtg tcc tac tgc aca ggt aat agc agt gag tgc ccg cct cca gga aat    339
Val Ser Tyr Cys Thr Gly Asn Ser Ser Glu Cys Pro Pro Pro Gly Asn
90                  95                  100                 105

gct gaa gat gac act gtt tgc ttg gat ctt ggc aag tgt aag gat ggg    387
Ala Glu Asp Asp Thr Val Cys Leu Asp Leu Gly Lys Cys Lys Asp Gly
                110                 115                 120

aaa tgc atc cct ttc tgc gag agg gaa cag cag ctg gag tcc tgt gca    435
Lys Cys Ile Pro Phe Cys Glu Arg Glu Gln Gln Leu Glu Ser Cys Ala
            125                 130                 135

tgt aat gaa act gac aac tcc tgc aag gtg tgc tgc agg gac ctt tcc    483
Cys Asn Glu Thr Asp Asn Ser Cys Lys Val Cys Cys Arg Asp Leu Ser
        140                 145                 150

ggc cgc tgt gtg ccc tat gtc gat gct gaa caa aag aac tta ttt ttg    531
Gly Arg Cys Val Pro Tyr Val Asp Ala Glu Gln Lys Asn Leu Phe Leu
    155                 160                 165

agg aaa gga aag ccc tgt aca gta gga ttt tgt gac atg aat ggc aaa    579
Arg Lys Gly Lys Pro Cys Thr Val Gly Phe Cys Asp Met Asn Gly Lys
170                 175                 180                 185

tgt gag aaa cga gta cag gat gta att gaa cga ttt tgg gat ttc att    627
Cys Glu Lys Arg Val Gln Asp Val Ile Glu Arg Phe Trp Asp Phe Ile
                190                 195                 200

gac cag ctg agc atc aat act ttt gga aag ttt tta gca gac aac aga    675
Asp Gln Leu Ser Ile Asn Thr Phe Gly Lys Phe Leu Ala Asp Asn Arg
            205                 210                 215

tct tgt gac aaa act cac aca tgc cca ccg tgc cca gca cct gaa gcc    723
Ser Cys Asp Lys Thr His Thr Cys Pro Pro Cys Pro Ala Pro Glu Ala
        220                 225                 230

gag ggc gcg ccg tca gtc ttc ctc ttc ccc cca aaa ccc aag gac acc    771
Glu Gly Ala Pro Ser Val Phe Leu Phe Pro Pro Lys Pro Lys Asp Thr
    235                 240                 245

ctc atg atc tcc cgg acc cct gag gtc aca tgc gtg gtg gtg gac gtg    819
Leu Met Ile Ser Arg Thr Pro Glu Val Thr Cys Val Val Val Asp Val
250                 255                 260                 265

agc cac gaa gac cct gag gtc aag ttc aac tgg tac gtg gac ggc gtg     867
Ser His Glu Asp Pro Glu Val Lys Phe Asn Trp Tyr Val Asp Gly Val
                270                 275                 280

gag gtg cat aat gcc aag aca aag ccg cgg gag gag cag tac aac agc     915
Glu Val His Asn Ala Lys Thr Lys Pro Arg Glu Glu Gln Tyr Asn Ser
            285                 290                 295

acg tac cgg gtg gtc agc gtc ctc acc gtc ctg cac cag gac tgg ctg     963
Thr Tyr Arg Val Val Ser Val Leu Thr Val Leu His Gln Asp Trp Leu
        300                 305                 310

aat ggc aag gag tac aag tgc aag gtc tcc aac aaa gcc ctc cca gcc     1011
Asn Gly Lys Glu Tyr Lys Cys Lys Val Ser Asn Lys Ala Leu Pro Ala
    315                 320                 325

ccc atc gag aaa acc atc tcc aaa gcc aaa ggg cag ccc cga gaa cca     1059
Pro Ile Glu Lys Thr Ile Ser Lys Ala Lys Gly Gln Pro Arg Glu Pro
330                 335                 340                 345

cag gtg tac acc ctg ccc cca tcc cgg gat gag ctg acc aag aac cag     1107
Gln Val Tyr Thr Leu Pro Pro Ser Arg Asp Glu Leu Thr Lys Asn Gln
                350                 355                 360

gtc agc ctg acc tgc ctg gtc aaa ggc ttc tat ccc agc gac atc gcc     1155
Val Ser Leu Thr Cys Leu Val Lys Gly Phe Tyr Pro Ser Asp Ile Ala
            365                 370                 375

gtg gag tgg gag agc aat ggg cag ccg gag aac aac tac aag acc acg     1203
Val Glu Trp Glu Ser Asn Gly Gln Pro Glu Asn Asn Tyr Lys Thr Thr
        380                 385                 390

cct ccc gtg ctg gac tcc gac ggc tcc ttc ttc ctc tac agc aag ctc     1251
Pro Pro Val Leu Asp Ser Asp Gly Ser Phe Phe Leu Tyr Ser Lys Leu
    395                 400                 405

acc gtg gac aag agc agg tgg cag cag ggg aac gtc ttc tca tgc tcc     1299
Thr Val Asp Lys Ser Arg Trp Gln Gln Gly Asn Val Phe Ser Cys Ser
410                 415                 420                 425

gtg atg cat gag gct ctg cac aac cac tac acg cag aag agc ctc tcc     1347
Val Met His Glu Ala Leu His Asn His Tyr Thr Gln Lys Ser Leu Ser
                430                 435                 440

ctg tct ccg ggt aaa tga actagagcgg ccgctacaga t                     1386
Leu Ser Pro Gly Lys
            445

<210> 10
<211> 446
<212> PRT
<213> Artificial Sequence
<223> Description of Artificial Sequence: fusion
      polypeptide

<400> 10
Met Glu Thr Asp Thr Leu Leu Leu Trp Val Leu Leu Leu Trp Val Pro
  1               5                  10                  15
Gly Ser Thr Gly Thr Ser Cys Gly Asn Ser Arg Val Asp Glu Gly Glu
             20                  25                  30
Glu Cys Asp Pro Gly Ile Met Tyr Leu Asn Asn Asp Thr Cys Cys Asn
         35                  40                  45
Ser Asp Cys Thr Leu Lys Glu Gly Val Gln Cys Ser Asp Arg Asn Ser
     50                  55                  60

Pro Cys Cys Lys Asn Cys Gln Phe Glu Thr Ala Gln Lys Lys Cys Gln
65                  70                  75                  80
Glu Ala Ile Asn Ala Thr Cys Lys Gly Val Ser Tyr Cys Thr Gly Asn
                85                  90                  95
Ser Ser Glu Cys Pro Pro Pro Gly Asn Ala Glu Asp Asp Thr Val Cys
            100                 105                 110
Leu Asp Leu Gly Lys Cys Lys Asp Gly Lys Cys Ile Pro Phe Cys Glu
        115                 120                 125
Arg Glu Gln Gln Leu Glu Ser Cys Ala Cys Asn Glu Thr Asp Asn Ser
    130                 135                 140
Cys Lys Val Cys Cys Arg Asp Leu Ser Gly Arg Cys Val Pro Tyr Val
145                 150                 155                 160
Asp Ala Glu Gln Lys Asn Leu Phe Leu Arg Lys Gly Lys Pro Cys Thr
                165                 170                 175
Val Gly Phe Cys Asp Met Asn Gly Lys Cys Glu Lys Arg Val Gln Asp
            180                 185                 190
Val Ile Glu Arg Phe Trp Asp Phe Ile Asp Gln Leu Ser Ile Asn Thr
        195                 200                 205
Phe Gly Lys Phe Leu Ala Asp Asn Arg Ser Cys Asp Lys Thr His Thr
    210                 215                 220
Cys Pro Pro Cys Pro Ala Pro Glu Ala Glu Gly Ala Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210> 11
<211> 1653
<212> DNA
<213> Artificial Sequence

<220>
<223> Description of Artificial Sequence: fusion
      polypeptide

<220>
<221> CDS
<222> (25)..(1632)

<400> 11
gtcgacccaa gctggctagc cacc atg gag aca gac aca ctc ctg cta tgg     51
gta ctg ctg ctc tgg gtt cca ggt tcc act ggt act agt tgt ggg aat    99
cta gtg gtt gaa gaa ggg gag gaa tgt gac tgt gga acc ata cgg cag    147
tgt gca aaa gat ccc tgt tgt ctg tta aac tgt act cta cat cct ggg    195
gct gct tgt gct ttt gga ata tgt tgc aaa gac tgc aaa ttt ctg cca    243
tca gga act tta tgt aga caa caa gtt ggt gaa tgt gac ctt cca gag    291
tgg tgc aat ggg aca tcc cat caa tgc cca gat gat gtg tat gtg cag    339
gac ggg atc tcc tgt aat gtg aat gcc ttc tgc tat gaa aag acg tgt    387
aat aac cat gat ata caa tgt aaa gag att ttt ggc caa gat gca agg    435
agt gca tct cag agt tgc tac caa gaa atc aac acc caa gga aac cgt    483
ttc ggt cac tgt ggt att gta ggc aca aca tat gta aaa tgt tgg acc    531
cct gat atc atg tgt ggg agg gtt cag tgt gaa aat gtg gga gta att    579
ccc aat ctg ata gag cat tct aca gtg cag cag ttt cac ctc aat gac    627
acc act tgc tgg ggc act gat tat cat tta ggg atg gct ata cct gat    675
att ggt gag gtg aaa gat ggc aca gta tgt ggt cca gaa aag atc tgc    723
atc cgt aag aag tgt gcc agt atg gtt cat ctg tca caa gcc tgt cag    771
cct aag acc tgc aac atg agg gga atc tgc aac aac aaa caa cac tgt    819
cac tgc aac cat gaa tgg gca ccc cca tac tgc aag gac aaa ggc tat 867
gga ggt agt gct gat agt ggc cca cct cct aag aac aac atg gaa gga 915
tta aat gtg atg gga aag ttg cgt gga tct tgt gac aaa act cac aca 963
tgc cca ccg tgc cca gca cct gaa gcc gag ggc gcg ccg tca gtc ttc 1011
ctc ttc ccc cca aaa ccc aag gac acc ctc atg atc tcc cgg acc cct 1059
gag gtc aca tgc gtg gtg gtg gac gtg agc cac gaa gac cct gag gtc 1107
aag ttc aac tgg tac gtg gac ggc gtg gag gtg cat aat gcc aag aca 1155
aag ccg cgg gag gag cag tac aac agc acg tac cgg gtg gtc agc gtc 1203
ctc acc gtc ctg cac cag gac tgg ctg aat ggc aag gag tac aag tgc 1251
aag gtc tcc aac aaa gcc ctc cca gcc ccc atc gag aaa acc atc tcc 1299
aaa gcc aaa ggg cag ccc cga gaa cca cag gtg tac acc ctg ccc cca 1347
tcc cgg gat gag ctg acc aag aac cag gtc agc ctg acc tgc ctg gtc 1395
aaa ggc ttc tat ccc agc gac atc gcc gtg gag tgg gag agc aat ggg 1443
cag ccg gag aac aac tac aag acc acg cct ccc gtg ctg gac tcc gac 1491
ggc tcc ttc ttc ctc tac agc aag ctc acc gtg gac aag agc agg tgg 1539
cag cag ggg aac gtc ttc tca tgc tcc gtg atg cat gag gct ctg cac 1587
aac cac tac acg cag aag agc ctc tcc ctg tct ccg ggt aaa tga 1632
actagagcgg ccgctacaga t 1653

<210> 12
<211> 535
<212> PRT
<213> Artificial Sequence
<223> Description of Artificial Sequence: fusion
polypeptide

<400> 12

<210> 13
<211> 1617
<212> DNA
<213> Artificial Sequence

<220>
<223> Description of Artificial Sequence: fusion
      polypeptide

<220>
<221> CDS
<222> (25)..(1596)

<400> 13
gtcgacccaa gctggctagc cacc atg gag aca gac aca ctc ctg cta tgg      51
gta ctg ctg ctc tgg gtt cca ggt tcc act ggt act agt tgt ggg aat      99
ggt gtg gtt gaa aga gaa gag cag tgt gac tgt gga tcc gta cag cag     147
tgt gaa caa gac gcc tgt tgt ctg ttg aac tgc act cta agg cct ggg     195
gct gcc tgt gct ttt ggg ctt tgt tgc aaa gac tgc aag ttc atg cca     243
tca ggg gaa ctc tgt aga caa gag gtc aat gaa tgt gac ctt cca gaa     291
tgg tgc aat gga aca tct cat cag tgt cca gaa gat aga tat gtg cag     339
gac ggg atc ccc tgt agt gac agt gcc tac tgc tat caa aag agg tgt     387
aat aac cat gac cag cat tgc agg gag att ttt ggt aaa gat gca aaa     435
agt gca tct cag aat tgc tat aaa gaa atc aac tct cag gga aac cgt 483
ttt ggt cac tgt ggt ata aat ggc aca aca tac cta aaa tgt cat atc 531
tct gat gtc ttt tgt ggg aga gtt caa tgt gag aat gtg aga gac att 579
cct ctt ctc caa gat cat ttt act ttg cag cac act cat atc aat ggt 627
gtc acc tgc tgg ggt att gac tat cat tta agg atg aac ata tct gac 675
att ggt gaa gtg aaa gat ggt act gtg tgt ggc cca gga aag atc tgc 723
atc cat aag aag tgt gtc agt ctg tct gtc ttg tca cat gtc tgc ctt 771
cct gag acc tgc aat atg aag ggg atc tgc aat aac aaa cat cac tgc 819
cac tgt ggc tat ggg tgg tcc cca ccc tac tgc cag cac aga ggc tat 867
ggg ggc agt att gac agt ggc cca gca tct gca aag aga tct tgt gac 915
aaa act cac aca tgc cca ccg tgc cca gca cct gaa gcc gag ggc gcg 963
ccg tca gtc ttc ctc ttc ccc cca aaa ccc aag gac acc ctc atg atc 1011
tcc cgg acc cct gag gtc aca tgc gtg gtg gtg gac gtg agc cac gaa 1059
gac cct gag gtc aag ttc aac tgg tac gtg gac ggc gtg gag gtg cat 1107
aat gcc aag aca aag ccg cgg gag gag cag tac aac agc acg tac cgg 1155
gtg gtc agc gtc ctc acc gtc ctg cac cag gac tgg ctg aat ggc aag 1203
gag tac aag tgc aag gtc tcc aac aaa gcc ctc cca gcc ccc atc gag     1251
aaa acc atc tcc aaa gcc aaa ggg cag ccc cga gaa cca cag gtg tac     1299
acc ctg ccc cca tcc cgg gat gag ctg acc aag aac cag gtc agc ctg     1347
acc tgc ctg gtc aaa ggc ttc tat ccc agc gac atc gcc gtg gag tgg     1395
gag agc aat ggg cag ccg gag aac aac tac aag acc acg cct ccc gtg     1443
ctg gac tcc gac ggc tcc ttc ttc ctc tac agc aag ctc acc gtg gac     1491
aag agc agg tgg cag cag ggg aac gtc ttc tca tgc tcc gtg atg cat     1539
gag gct ctg cac aac cac tac acg cag aag agc ctc tcc ctg tct ccg     1587
ggt aaa tga actagagcgg ccgctacaga t                                 1617

<210> 14
<211> 523
<212> PRT
<213> Artificial Sequence
<223> Description of Artificial Sequence: fusion
      polypeptide

<400> 14

<210> 15
<211> 1674
<212> DNA
<213> Artificial Sequence

<220>
<223> Description of Artificial Sequence: fusion
      polypeptide

<220>
<221> CDS
<222> (25)..(1653)

<400> 15
gtcgacccaa gctggctagc cacc atg gag aca gac aca ctc ctg cta tgg      51
gta ctg ctg ctc tgg gtt cca ggt tcc act ggt act agt tgt ggc aat      99
ggc ttc att gaa act gga gag gag tgt gat tgt gga acc ccg gcc gaa     147
tgt gtc ctt gaa gga gca gag tgt tgt aag aaa tgc acc ttg act caa     195
gac tct caa tgc agt gac ggt ctt tgc tgt aaa aag tgc aag ttt cag     243
cct atg ggc act gtg tgc cga gaa gca gta aat gat tgt gat att cgt     291
gaa acg tgc tca gga aat tca agc cag tgt gcc cct aat att cat aaa     339
atg gat gga tat tca tgt gat ggt gtt cag gga att tgc ttt gga gga     387
aga tgc aaa acc aga gat aga caa tgc aaa tac att tgg ggg caa aag     435
gtg aca gca tca gac aaa tat tgc tat gag aaa ctg aat att gaa ggg     483
acg gag aag ggt aac tgt ggg aaa gac aaa gac aca tgg ata cag tgc     531
aac aaa cgg gat gtg ctt tgt ggt tac ctt ttg tgt acc aat att ggc     579
aat atc cca agg ctt gga gaa ctc gat ggt gaa atc aca tct act tta     627
gtt gtg cag caa gga aga aca tta aac tgc agt ggt ggg cat gtt aag     675
ctt gaa gaa gat gta gat ctt ggc tat gtg gaa gat ggg aca cct tgt     723
ggt ccc caa atg atg tgc tta gaa cac agg tgt ctt cct gtg gct tct     771
ttc aac ttt agt act tgc ttg agc agt aaa gaa ggc act att tgc tca     819
gga aat gga gtt tgc agt aat gag ctg aag tgt gtg tgt aac aga cac     867
tgg ata ggt tct gat tgc aac act tac ttc cct cac aat gat gat gca 915
aag act ggt atc act ctg tct ggc aat ggt gtt gct ggc acc aat gga 963
tct tgt gac aaa act cac aca tgc cca ccg tgc cca gca cct gaa gcc 1011
gag ggc gcg ccg tca gtc ttc ctc ttc ccc cca aaa ccc aag gac acc 1059
ctc atg atc tcc cgg acc cct gag gtc aca tgc gtg gtg gtg gac gtg 1107
agc cac gaa gac cct gag gtc aag ttc aac tgg tac gtg gac ggc gtg 1155
gag gtg cat aat gcc aag aca aag ccg cgg gag gag cag tac aac agc 1203
acg tac cgg gtg gtc agc gtc ctc acc gtc ctg cac cag gac tgg ctg 1251
aat ggc aag gag tac aag tgc aag gtc tcc aac aaa gcc ctc cca gcc 1299
ccc atc gag aaa acc atc tcc aaa gcc aaa ggg cag ccc cga gaa cca 1347
cag gtg tac acc ctg ccc cca tcc cgg gat gag ctg acc aag aac cag 1395
gtc agc ctg acc tgc ctg gtc aaa ggc ttc tat ccc agc gac atc gcc 1443
gtg gag tgg gag agc aat ggg cag ccg gag aac aac tac aag acc acg 1491
cct ccc gtg ctg gac tcc gac ggc tcc ttc ttc ctc tac agc aag ctc 1539
acc gtg gac aag agc agg tgg cag cag ggg aac gtc ttc tca tgc tcc 1587
gtg atg cat gag gct ctg cac aac cac tac acg cag aag agc ctc tcc 1635
ctg tct ccg ggt aaa tga actagagcgg ccgctacaga t 1674

<210> 16
<211> 542
<212> PRT
<213> Artificial Sequence
<223> Description of Artificial Sequence: fusion
      polypeptide

<400> 16

<210> 17
<211> 1668
<212> DNA
<213> Artificial Sequence

<220>
<223> Description of Artificial Sequence: fusion
      polypeptide

<220>
<221> CDS
<222> (25)..(1647)

<400> 17
gtcgacccaa gctggctagc cacc atg gag aca gac aca ctc ctg cta tgg      51
                           Met Glu Thr Asp Thr Leu Leu Leu Trp
                             1               5

gta ctg ctg ctc tgg gtt cca ggt tcc act ggt act agt tgt gga aat      99
Val Leu Leu Leu Trp Val Pro Gly Ser Thr Gly Thr Ser Cys Gly Asn
 10                  15                  20                  25

gga tac gtc gaa gct ggg gag gag tgt gat tgt ggt ttt cat gtg gaa     147
Gly Tyr Val Glu Ala Gly Glu Glu Cys Asp Cys Gly Phe His Val Glu
                 30                  35                  40

tgc tat gga tta tgc tgt aag aaa tgt tcc ctc tcc aac ggg gct cac     195
Cys Tyr Gly Leu Cys Cys Lys Lys Cys Ser Leu Ser Asn Gly Ala His
             45                  50                  55

tgc agc gac ggg ccc tgc tgt aac aat acc tca tgt ctt ttt cag cca     243
Cys Ser Asp Gly Pro Cys Cys Asn Asn Thr Ser Cys Leu Phe Gln Pro
         60                  65                  70

cga ggg tat gaa tgc cgg gat gct gtg aac gag tgt gat att act gaa     291
Arg Gly Tyr Glu Cys Arg Asp Ala Val Asn Glu Cys Asp Ile Thr Glu
     75                  80                  85

tat tgt act gga gac tct ggt cag tgc cca cca aat ctt cat aag caa     339
Tyr Cys Thr Gly Asp Ser Gly Gln Cys Pro Pro Asn Leu His Lys Gln
 90                  95                 100                 105

gac gga tat gca tgc aat caa aat cag ggc cgc tgc tac aat ggc gag     387
Asp Gly Tyr Ala Cys Asn Gln Asn Gln Gly Arg Cys Tyr Asn Gly Glu
                110                 115                 120

tgc aag gcc aga gac aac cag tgt cag tac atc tgg gga aca aag gct     435
Cys Lys Ala Arg Asp Asn Gln Cys Gln Tyr Ile Trp Gly Thr Lys Ala
            125                 130                 135

gca ggg tct gac aag ttc tgc tat gaa aag ctg aat aca gaa ggc act 483
Ala Gly Ser Asp Lys Phe Cys Tyr Glu Lys Leu Asn Thr Glu Gly Thr
        140                 145                 150

gag aag gga aac tgc ggg aag gat gga gac cgg tgg att cag tgc agc 531
Glu Lys Gly Asn Cys Gly Lys Asp Gly Asp Arg Trp Ile Gln Cys Ser
    155                 160                 165

aaa cat gat gtg ttc tgt gga ttc tta ctc tgt acc aat ctt act cga 579
Lys His Asp Val Phe Cys Gly Phe Leu Leu Cys Thr Asn Leu Thr Arg
170                 175                 180                 185

gct cca cgt att ggt caa ctt cag ggt gag atc att cca act tcc ttc 627
Ala Pro Arg Ile Gly Gln Leu Gln Gly Glu Ile Ile Pro Thr Ser Phe
                190                 195                 200

tac cat caa ggc cgg gtg att gac tgc agt ggt gcc cat gta gtt tta 675
Tyr His Gln Gly Arg Val Ile Asp Cys Ser Gly Ala His Val Val Leu
            205                 210                 215

gat gat gat acg gat gtg ggc tat gta gaa gat gga acg cca tgt ggc 723
Asp Asp Asp Thr Asp Val Gly Tyr Val Glu Asp Gly Thr Pro Cys Gly
        220                 225                 230

ccg tct atg atg tgt tta gat cgg aag tgc cta caa att caa gcc cta 771
Pro Ser Met Met Cys Leu Asp Arg Lys Cys Leu Gln Ile Gln Ala Leu
    235                 240                 245

aat atg agc agc tgt cca ctc gat tcc aag ggt aaa gtc tgt tcg ggc 819
Asn Met Ser Ser Cys Pro Leu Asp Ser Lys Gly Lys Val Cys Ser Gly
250                 255                 260                 265

cat ggg gtg tgt agt aat gaa gcc acc tgc att tgt gat ttc acc tgg 867
His Gly Val Cys Ser Asn Glu Ala Thr Cys Ile Cys Asp Phe Thr Trp
                270                 275                 280

gca ggg aca gat tgc agt atc cgg gat cca gtt agg aac ctt cac ccc 915
Ala Gly Thr Asp Cys Ser Ile Arg Asp Pro Val Arg Asn Leu His Pro
            285                 290                 295

ccc aag gat gaa gga ccc aag ggt cct agt gcc acc aat aga tct tgt 963
Pro Lys Asp Glu Gly Pro Lys Gly Pro Ser Ala Thr Asn Arg Ser Cys
        300                 305                 310

gac aaa act cac aca tgc cca ccg tgc cca gca cct gaa gcc gag ggc 1011
Asp Lys Thr His Thr Cys Pro Pro Cys Pro Ala Pro Glu Ala Glu Gly
    315                 320                 325

gcg ccg tca gtc ttc ctc ttc ccc cca aaa ccc aag gac acc ctc atg 1059
Ala Pro Ser Val Phe Leu Phe Pro Pro Lys Pro Lys Asp Thr Leu Met
330                 335                 340                 345

atc tcc cgg acc cct gag gtc aca tgc gtg gtg gtg gac gtg agc cac 1107
Ile Ser Arg Thr Pro Glu Val Thr Cys Val Val Val Asp Val Ser His
                350                 355                 360

gaa gac cct gag gtc aag ttc aac tgg tac gtg gac ggc gtg gag gtg 1155
Glu Asp Pro Glu Val Lys Phe Asn Trp Tyr Val Asp Gly Val Glu Val
            365                 370                 375

cat aat gcc aag aca aag ccg cgg gag gag cag tac aac agc acg tac 1203
His Asn Ala Lys Thr Lys Pro Arg Glu Glu Gln Tyr Asn Ser Thr Tyr
        380                 385                 390

cgg gtg gtc agc gtc ctc acc gtc ctg cac cag gac tgg ctg aat ggc 1251

Arg Val Val Ser Val Leu Thr Val Leu His Gln Asp Trp Leu Asn Gly
    395                 400                 405

aag gag tac aag tgc aag gtc tcc aac aaa gcc ctc cca gcc ccc atc    1299
Lys Glu Tyr Lys Cys Lys Val Ser Asn Lys Ala Leu Pro Ala Pro Ile
410                 415                 420                 425

gag aaa acc atc tcc aaa gcc aaa ggg cag ccc cga gaa cca cag gtg    1347
Glu Lys Thr Ile Ser Lys Ala Lys Gly Gln Pro Arg Glu Pro Gln Val
                430                 435                 440

tac acc ctg ccc cca tcc cgg gat gag ctg acc aag aac cag gtc agc    1395
Tyr Thr Leu Pro Pro Ser Arg Asp Glu Leu Thr Lys Asn Gln Val Ser
            445                 450                 455

ctg acc tgc ctg gtc aaa ggc ttc tat ccc agc gac atc gcc gtg gag    1443
Leu Thr Cys Leu Val Lys Gly Phe Tyr Pro Ser Asp Ile Ala Val Glu
        460                 465                 470

tgg gag agc aat ggg cag ccg gag aac aac tac aag acc acg cct ccc    1491
Trp Glu Ser Asn Gly Gln Pro Glu Asn Asn Tyr Lys Thr Thr Pro Pro
    475                 480                 485

gtg ctg gac tcc gac ggc tcc ttc ttc ctc tac agc aag ctc acc gtg    1539
Val Leu Asp Ser Asp Gly Ser Phe Phe Leu Tyr Ser Lys Leu Thr Val
490                 495                 500                 505

gac aag agc agg tgg cag cag ggg aac gtc ttc tca tgc tcc gtg atg    1587
Asp Lys Ser Arg Trp Gln Gln Gly Asn Val Phe Ser Cys Ser Val Met
                510                 515                 520

cat gag gct ctg cac aac cac tac acg cag aag agc ctc tcc ctg tct    1635
His Glu Ala Leu His Asn His Tyr Thr Gln Lys Ser Leu Ser Leu Ser
            525                 530                 535

ccg ggt aaa tga actagagcgg ccgctacaga t                            1668
Pro Gly Lys
        540


<210> 18
<211> 540
<212> PRT
<213> Artificial Sequence
<223> Description of Artificial Sequence: fusion
      polypeptide

<400> 18
Met Glu Thr Asp Thr Leu Leu Leu Trp Val Leu Leu Leu Trp Val Pro
  1               5                  10                  15
Gly Ser Thr Gly Thr Ser Cys Gly Asn Gly Tyr Val Glu Ala Gly Glu
             20                  25                  30
Glu Cys Asp Cys Gly Phe His Val Glu Cys Tyr Gly Leu Cys Cys Lys
         35                  40                  45
Lys Cys Ser Leu Ser Asn Gly Ala His Cys Ser Asp Gly Pro Cys Cys
     50                  55                  60
Asn Asn Thr Ser Cys Leu Phe Gln Pro Arg Gly Tyr Glu Cys Arg Asp
 65                  70                  75                  80
Ala Val Asn Glu Cys Asp Ile Thr Glu Tyr Cys Thr Gly Asp Ser Gly
                 85                  90                  95
Gln Cys Pro Pro Asn Leu His Lys Gln Asp Gly Tyr Ala Cys Asn Gln
            100                 105                 110
Asn Gln Gly Arg Cys Tyr Asn Gly Glu Cys Lys Ala Arg Asp Asn Gln
        115                 120                 125
Cys Gln Tyr Ile Trp Gly Thr Lys Ala Ala Gly Ser Asp Lys Phe Cys
    130                 135                 140

Tyr Glu Lys Leu Asn Thr Glu Gly Thr Glu Lys Gly Asn Cys Gly Lys
145                 150                 155                 160
Asp Gly Asp Arg Trp Ile Gln Cys Ser Lys His Asp Val Phe Cys Gly
                165                 170                 175
Phe Leu Leu Cys Thr Asn Leu Thr Arg Ala Pro Arg Ile Gly Gln Leu
            180                 185                 190
Gln Gly Glu Ile Ile Pro Thr Ser Phe Tyr His Gln Gly Arg Val Ile
        195                 200                 205
Asp Cys Ser Gly Ala His Val Val Leu Asp Asp Asp Thr Asp Val Gly
    210                 215                 220
Tyr Val Glu Asp Gly Thr Pro Cys Gly Pro Ser Met Met Cys Leu Asp
225                 230                 235                 240
Arg Lys Cys Leu Gln Ile Gln Ala Leu Asn Met Ser Ser Cys Pro Leu
                245                 250                 255
Asp Ser Lys Gly Lys Val Cys Ser Gly His Gly Val Cys Ser Asn Glu
            260                 265                 270
Ala Thr Cys Ile Cys Asp Phe Thr Trp Ala Gly Thr Asp Cys Ser Ile
        275                 280                 285
Arg Asp Pro Val Arg Asn Leu His Pro Pro Lys Asp Glu Gly Pro Lys
    290                 295                 300
Gly Pro Ser Ala Thr Asn Arg Ser Cys Asp Lys Thr His Thr Cys Pro
305                 310                 315                 320
Pro Cys Pro Ala Pro Glu Ala Glu Gly Ala Pro Ser Val Phe Leu Phe
                325                 330                 335
Pro Pro Lys Pro Lys Asp Thr Leu Met Ile Ser Arg Thr Pro Glu Val
            340                 345                 350
Thr Cys Val Val Val Asp Val Ser His Glu Asp Pro Glu Val Lys Phe
        355                 360                 365
Asn Trp Tyr Val Asp Gly Val Glu Val His Asn Ala Lys Thr Lys Pro
    370                 375                 380
Arg Glu Glu Gln Tyr Asn Ser Thr Tyr Arg Val Val Ser Val Leu Thr
385                 390                 395                 400
Val Leu His Gln Asp Trp Leu Asn Gly Lys Glu Tyr Lys Cys Lys Val
                405                 410                 415
Ser Asn Lys Ala Leu Pro Ala Pro Ile Glu Lys Thr Ile Ser Lys Ala
            420                 425                 430
Lys Gly Gln Pro Arg Glu Pro Gln Val Tyr Thr Leu Pro Pro Ser Arg
        435                 440                 445
Asp Glu Leu Thr Lys Asn Gln Val Ser Leu Thr Cys Leu Val Lys Gly
    450                 455                 460
Phe Tyr Pro Ser Asp Ile Ala Val Glu Trp Glu Ser Asn Gly Gln Pro
465                 470                 475                 480
Glu Asn Asn Tyr Lys Thr Thr Pro Pro Val Leu Asp Ser Asp Gly Ser
                485                 490                 495
Phe Phe Leu Tyr Ser Lys Leu Thr Val Asp Lys Ser Arg Trp Gln Gln
            500                 505                 510
Gly Asn Val Phe Ser Cys Ser Val Met His Glu Ala Leu His Asn His
        515                 520                 525
Tyr Thr Gln Lys Ser Leu Ser Leu Ser Pro Gly Lys
    530                 535                 540

<210> 19
<211> 3
<212> PRT
<213> Artificial Sequence

<220>
<223> Description of Artificial Sequence: Consensus
      binding motif

<400> 19
Arg Gly Asp
  1

<210> 20
<211> 67
<212> PRT
<213> Artificial Sequence

<220>
<223> Description of Artificial Sequence: consensus
      disintegrin domain

<220>
<221> VARIANT
<222> (5)..(9)
<223> 3-5 varying residues in a consensus sequence

<220>
<221> VARIANT
<222> (11)..(16)
<223> 3-6 varying residues in a consensus sequence

<220>
<221> VARIANT
<222> (19)..(22)
<223> 2-4 varying residues in a consensus sequence

<220>
<221> VARIANT
<222> (24)..(30)
<223> 7 varying residues in a consensus sequence

<220>
<221> VARIANT
<222> (32)..(37)
<223> 4-6 varying residues in a consensus sequence

<220>
<221> VARIANT
<222> (40)..(43)
<223> 2-4 varying residues in a consensus sequence

<220>
<221> VARIANT
<222> (45)..(52)
<223> 8 varying residues in a consensus sequence

<220>
<221> VARIANT
<222> (54)..(60)
<223> 5-7 varying residues in a consensus sequence

<220>
<221> VARIANT
<222> (62)..(66)
<223> 3-5 varying residues in a consensus sequence

<400> 20
Cys Asp Cys Gly Xaa Xaa Xaa Xaa Xaa Cys Xaa Xaa Xaa Xaa Xaa Xaa
  1               5                  10                  15

Cys Cys Xaa Xaa Xaa Xaa Cys Xaa Xaa Xaa Xaa Xaa Xaa Xaa Cys Xaa
             20                  25                  30

Xaa Xaa Xaa Xaa Xaa Cys Cys Xaa Xaa Xaa Xaa Cys Xaa Xaa Xaa Xaa
         35                  40                  45

Xaa Xaa Xaa Xaa Cys Xaa Xaa Xaa Xaa Xaa Xaa Xaa Cys Xaa Xaa Xaa
     50                  55                  60

Xaa Xaa Cys
65

<210> 21
<211> 1725
<212> DNA
<213> Artificial Sequence

<220>
<223> Description of Artificial Sequence: fusion
      polypeptide

<220>
<221> CDS
<222> (118)..(1704)

<400> 21
gggttttccc agtcacgacg ttgtaaaacg acggccagtg aattgtaata cgactcacta 60

tagggcgaat tgggtaccgg gccccccctc gaggtcgacc caagctggct agccacc 117

atg gag aca gac aca ctc ctg cta tgg gta ctg ctg ctc tgg gtt cca 165
Met Glu Thr Asp Thr Leu Leu Leu Trp Val Leu Leu Leu Trp Val Pro
1               5                   10                  15

ggt tcc act ggt act agt tgt ggg aat ggt gtg gtt gaa gaa gga gaa 213
Gly Ser Thr Gly Thr Ser Cys Gly Asn Gly Val Val Glu Glu Gly Glu
            20                  25                  30

gag tgt gac tgt gga cct tta aag cat tgt gca aaa gat ccc tgc tgt 261
Glu Cys Asp Cys Gly Pro Leu Lys His Cys Ala Lys Asp Pro Cys Cys
        35                  40                  45

ctg tca aat tgc act ctg act gat ggt tct act tgt gct ttt ggg ctt 309
Leu Ser Asn Cys Thr Leu Thr Asp Gly Ser Thr Cys Ala Phe Gly Leu
    50                  55                  60

tgt tgc aaa gac tgc aag ttc cta cca tca ggg aaa gtg tgt aga aag 357
Cys Cys Lys Asp Cys Lys Phe Leu Pro Ser Gly Lys Val Cys Arg Lys
65                  70                  75                  80

gag gtc aat gaa tgt gat ctt cca gag tgg tgc aat ggt act tcc cat 405
Glu Val Asn Glu Cys Asp Leu Pro Glu Trp Cys Asn Gly Thr Ser His
                85                  90                  95

aag tgc cca gat gac ttt tat gtg gaa gat gga att ccc tgt aag gag 453
Lys Cys Pro Asp Asp Phe Tyr Val Glu Asp Gly Ile Pro Cys Lys Glu
            100                 105                 110

agg ggc tac tgc tat gaa aag agc tgt cat gac cgc aat gaa cag tgt 501
Arg Gly Tyr Cys Tyr Glu Lys Ser Cys His Asp Arg Asn Glu Gln Cys
        115                 120                 125

agg agg att ttt ggt gca ggc gca aat act gca agt gag act tgc tac 549
Arg Arg Ile Phe Gly Ala Gly Ala Asn Thr Ala Ser Glu Thr Cys Tyr
    130                 135                 140

aaa gaa ttg aac acc tta ggt gac cgt gtt ggt cac tgt ggt atc aaa 597
Lys Glu Leu Asn Thr Leu Gly Asp Arg Val Gly His Cys Gly Ile Lys
145                 150                 155                 160

aat gct aca tat ata aag tgt aat atc tca gat gtc cag tgt gga aga 645
Asn Ala Thr Tyr Ile Lys Cys Asn Ile Ser Asp Val Gln Cys Gly Arg
                165                 170                 175

att cag tgt gag aat gtg aca gaa att ccc aat atg agt gat cat act 693
Ile Gln Cys Glu Asn Val Thr Glu Ile Pro Asn Met Ser Asp His Thr
            180                 185                 190

act gtg cat tgg gct cgc ttc aat gac ata atg tgc tgg agt act gat 741
Thr Val His Trp Ala Arg Phe Asn Asp Ile Met Cys Trp Ser Thr Asp
        195                 200                 205

tac cat ttg ggg atg aag gga cct gat att ggt gaa gtg aaa gat gga 789
Tyr His Leu Gly Met Lys Gly Pro Asp Ile Gly Glu Val Lys Asp Gly
    210                 215                 220

aca gag tgt ggg ata gat cat ata tgc atc cac agg cac tgt gtc cat 837
Thr Glu Cys Gly Ile Asp His Ile Cys Ile His Arg His Cys Val His
225                 230                 235                 240

ata acc atc ttg aat agt aat tgc tca cct gca ttt tgt aac aag agg 885
Ile Thr Ile Leu Asn Ser Asn Cys Ser Pro Ala Phe Cys Asn Lys Arg
                245                 250                 255

ggc atc tgc aac aat aaa cat cac tgc cat tgc aat tat ctg tgg gac 933
Gly Ile Cys Asn Asn Lys His His Cys His Cys Asn Tyr Leu Trp Asp
            260                 265                 270

cct ccc aac tgc ctg ata aaa ggc tat gga ggt agt gtt gac agt ggc 981
Pro Pro Asn Cys Leu Ile Lys Gly Tyr Gly Gly Ser Val Asp Ser Gly
        275                 280                 285

cca ccc cct aag aga aag aag aaa aag aag aga tct tgt gac aaa act 1029
Pro Pro Pro Lys Arg Lys Lys Lys Lys Lys Arg Ser Cys Asp Lys Thr
    290                 295                 300

cac aca tgc cca ccg tgc cca gca cct gaa gcc gag ggc gcg ccg tca 1077
His Thr Cys Pro Pro Cys Pro Ala Pro Glu Ala Glu Gly Ala Pro Ser
305                 310                 315                 320

gtc ttc ctc ttc ccc cca aaa ccc aag gac acc ctc atg atc tcc cgg 1125
Val Phe Leu Phe Pro Pro Lys Pro Lys Asp Thr Leu Met Ile Ser Arg
                325                 330                 335

acc cct gag gtc aca tgc gtg gtg gtg gac gtg agc cac gaa gac cct 1173
Thr Pro Glu Val Thr Cys Val Val Val Asp Val Ser His Glu Asp Pro
            340                 345                 350

gag gtc aag ttc aac tgg tac gtg gac ggc gtg gag gtg cat aat gcc 1221
Glu Val Lys Phe Asn Trp Tyr Val Asp Gly Val Glu Val His Asn Ala
        355                 360                 365

aag aca aag ccg cgg gag gag cag tac aac agc acg tac cgg gtg gtc 1269
Lys Thr Lys Pro Arg Glu Glu Gln Tyr Asn Ser Thr Tyr Arg Val Val
    370                 375                 380

agc gtc ctc acc gtc ctg cac cag gac tgg ctg aat ggc aag gag tac 1317
Ser Val Leu Thr Val Leu His Gln Asp Trp Leu Asn Gly Lys Glu Tyr
385                 390                 395                 400

aag tgc aag gtc tcc aac aaa gcc ctc cca gcc ccc atc gag aaa acc 1365
Lys Cys Lys Val Ser Asn Lys Ala Leu Pro Ala Pro Ile Glu Lys Thr
                405                 410                 415

atc tcc aaa gcc aaa ggg cag ccc cga gaa cca cag gtg tac acc ctg 1413
Ile Ser Lys Ala Lys Gly Gln Pro Arg Glu Pro Gln Val Tyr Thr Leu
            420                 425                 430

ccc cca tcc cgg gat gag ctg acc aag aac cag gtc agc ctg acc tgc 1461

Pro Pro Ser Arg Asp Glu Leu Thr Lys Asn Gln Val Ser Leu Thr Cys
        435                 440                 445

ctg gtc aaa ggc ttc tat ccc agc gac atc gcc gtg gag tgg gag agc      1509
Leu Val Lys Gly Phe Tyr Pro Ser Asp Ile Ala Val Glu Trp Glu Ser
    450                 455                 460

aat ggg cag ccg gag aac aac tac aag acc acg cct ccc gtg ctg gac      1557
Asn Gly Gln Pro Glu Asn Asn Tyr Lys Thr Thr Pro Pro Val Leu Asp
465                 470                 475                 480

tcc gac ggc tcc ttc ttc ctc tac agc aag ctc acc gtg gac aag agc      1605
Ser Asp Gly Ser Phe Phe Leu Tyr Ser Lys Leu Thr Val Asp Lys Ser
                485                 490                 495

agg tgg cag cag ggg aac gtc ttc tca tgc tcc gtg atg cat gag gct      1653
Arg Trp Gln Gln Gly Asn Val Phe Ser Cys Ser Val Met His Glu Ala
            500                 505                 510

ctg cac aac cac tac acg cag aag agc ctc tcc ctg tct ccg ggt aaa      1701
Leu His Asn His Tyr Thr Gln Lys Ser Leu Ser Leu Ser Pro Gly Lys
        515                 520                 525

tga actagagcgg ccgctacaga t                                          1725

<210> 22
<211> 528
<212> PRT
<213> Artificial Sequence
<223> Description of Artificial Sequence: fusion
      polypeptide

<400> 22
Met Glu Thr Asp Thr Leu Leu Leu Trp Val Leu Leu Leu Trp Val Pro
 1               5                  10                  15
Gly Ser Thr Gly Thr Ser Cys Gly Asn Gly Val Val Glu Glu Gly Glu
            20                  25                  30
Glu Cys Asp Cys Gly Pro Leu Lys His Cys Ala Lys Asp Pro Cys Cys
        35                  40                  45
Leu Ser Asn Cys Thr Leu Thr Asp Gly Ser Thr Cys Ala Phe Gly Leu
    50                  55                  60
Cys Cys Lys Asp Cys Lys Phe Leu Pro Ser Gly Lys Val Cys Arg Lys
65                  70                  75                  80
Glu Val Asn Glu Cys Asp Leu Pro Glu Trp Cys Asn Gly Thr Ser His
                85                  90                  95
Lys Cys Pro Asp Asp Phe Tyr Val Glu Asp Gly Ile Pro Cys Lys Glu
            100                 105                 110
Arg Gly Tyr Cys Tyr Glu Lys Ser Cys His Asp Arg Asn Glu Gln Cys
        115                 120                 125
Arg Arg Ile Phe Gly Ala Gly Ala Asn Thr Ala Ser Glu Thr Cys Tyr
    130                 135                 140
Lys Glu Leu Asn Thr Leu Gly Asp Arg Val Gly His Cys Gly Ile Lys
145                 150                 155                 160
Asn Ala Thr Tyr Ile Lys Cys Asn Ile Ser Asp Val Gln Cys Gly Arg
                165                 170                 175
Ile Gln Cys Glu Asn Val Thr Glu Ile Pro Asn Met Ser Asp His Thr
            180                 185                 190
Thr Val His Trp Ala Arg Phe Asn Asp Ile Met Cys Trp Ser Thr Asp
        195                 200                 205
Tyr His Leu Gly Met Lys Gly Pro Asp Ile Gly Glu Val Lys Asp Gly
    210                 215                 220
Thr Glu Cys Gly Ile Asp His Ile Cys Ile His Arg His Cys Val His
225                 230                 235                 240
Ile Thr Ile Leu Asn Ser Asn Cys Ser Pro Ala Phe Cys Asn Lys Arg
                245                 250                 255

Gly Ile Cys Asn Asn Lys His His Cys His Cys Asn Tyr Leu Trp Asp
            260                 265                 270
Pro Pro Asn Cys Leu Ile Lys Gly Tyr Gly Gly Ser Val Asp Ser Gly
        275                 280                 285
Pro Pro Pro Lys Arg Lys Lys Lys Lys Lys Arg Ser Cys Asp Lys Thr
    290                 295                 300
His Thr Cys Pro Pro Cys Pro Ala Pro Glu Ala Glu Gly Ala Pro Ser
305                 310                 315                 320
Val Phe Leu Phe Pro Pro Lys Pro Lys Asp Thr Leu Met Ile Ser Arg
                325                 330                 335
Thr Pro Glu Val Thr Cys Val Val Val Asp Val Ser His Glu Asp Pro
            340                 345                 350
Glu Val Lys Phe Asn Trp Tyr Val Asp Gly Val Glu Val His Asn Ala
        355                 360                 365
Lys Thr Lys Pro Arg Glu Glu Gln Tyr Asn Ser Thr Tyr Arg Val Val
    370                 375                 380
Ser Val Leu Thr Val Leu His Gln Asp Trp Leu Asn Gly Lys Glu Tyr
385                 390                 395                 400
Lys Cys Lys Val Ser Asn Lys Ala Leu Pro Ala Pro Ile Glu Lys Thr
                405                 410                 415
Ile Ser Lys Ala Lys Gly Gln Pro Arg Glu Pro Gln Val Tyr Thr Leu
            420                 425                 430
Pro Pro Ser Arg Asp Glu Leu Thr Lys Asn Gln Val Ser Leu Thr Cys
        435                 440                 445
Leu Val Lys Gly Phe Tyr Pro Ser Asp Ile Ala Val Glu Trp Glu Ser
    450                 455                 460
Asn Gly Gln Pro Glu Asn Asn Tyr Lys Thr Thr Pro Pro Val Leu Asp
465                 470                 475                 480
Ser Asp Gly Ser Phe Phe Leu Tyr Ser Lys Leu Thr Val Asp Lys Ser
                485                 490                 495
Arg Trp Gln Gln Gly Asn Val Phe Ser Cys Ser Val Met His Glu Ala
            500                 505                 510
Leu His Asn His Tyr Thr Gln Lys Ser Leu Ser Leu Ser Pro Gly Lys
        515                 520                 525

SEQUENCE LISTING

<110> Immunex Corporation
      Fanslow, William C.
      Poindexter, Kurt
      Cerretti, Douglas P.
      Black, Roy A.

<120> INTEGRIN ANTAGONISTS

<130> P34255EP1

<140> EP06026259.9
<141> 2006-12-19

<150> EP01920133.4
<151> 2001-02-23

<150> US60/184,865
<151> 2000-02-25

<160> 22

<170> PatentIn Ver. 2.1

<210> 1
<211> 1700
<212> DNA
<213> Artificial Sequence

<220>
<223> Description of Artificial Sequence: fusion
      polypeptide

<220>
<221> CDS
<222> (118)..(1602)

<400> 1
gggttttccc agtcacgacg ttgtaaaacg acggccagtg aattgtaata cgactcacta 60

tagggcgaat tgggtaccgg gccccccctc gaggtcgacc caagctggct agccacc    117

atg gag aca gac aca ctc ctg cta tgg gta ctg ctg ctc tgg gtt cca   165
Met Glu Thr Asp Thr Leu Leu Leu Trp Val Leu Leu Leu Trp Val Pro
  1               5                  10                  15

ggt tcc act ggt act agt tgt ggg aac ctg ttt gtg gag cgt ggg gag   213
Gly Ser Thr Gly Thr Ser Cys Gly Asn Leu Phe Val Glu Arg Gly Glu
             20                  25                  30

cag tgc gac tgc ggc ccc ccc gag gac tgc cgg aac cgc tgc tgc aac   261
Gln Cys Asp Cys Gly Pro Pro Glu Asp Cys Arg Asn Arg Cys Cys Asn
         35                  40                  45

tct acc acc tgc cag ctg gct gag ggg gcc cag tgt gcg cac ggt acc   309
Ser Thr Thr Cys Gln Leu Ala Glu Gly Ala Gln Cys Ala His Gly Thr
     50                  55                  60

tgc tgc cag gag tgc aag gtg aag ccg gct ggt gag ctg tgc cgt ccc   357
Cys Cys Gln Glu Cys Lys Val Lys Pro Ala Gly Glu Leu Cys Arg Pro

65                  70                  75                  80

aag aag gac atg tgt gac ctc gag gag ttc tgt gac ggc cgg cac cct     405
Lys Lys Asp Met Cys Asp Leu Glu Glu Phe Cys Asp Gly Arg His Pro
                85                  90                  95

gag tgc ccg gaa gac gcc ttc cag gag aac ggc acg ccc tgc tcc ggg     453
Glu Cys Pro Glu Asp Ala Phe Gln Glu Asn Gly Thr Pro Cys Ser Gly
            100                 105                 110

ggc tac tgc tac aac ggg gcc tgt ccc aca ctg gcc cag cag tgc cag     501
Gly Tyr Cys Tyr Asn Gly Ala Cys Pro Thr Leu Ala Gln Gln Cys Gln
        115                 120                 125

gcc ttc tgg ggg cca ggt ggg cag gct gcc gag gag tcc tgc ttc tcc     549
Ala Phe Trp Gly Pro Gly Gly Gln Ala Ala Glu Glu Ser Cys Phe Ser
    130                 135                 140

tat gac atc cta cca ggc tgc aag gcc agc cgg tac agg gct gac atg     597
Tyr Asp Ile Leu Pro Gly Cys Lys Ala Ser Arg Tyr Arg Ala Asp Met
145                 150                 155                 160

tgt ggc gtt ctg caa tgt aaa ggt ggt caa caa cct tta ggt aga gct     645
Cys Gly Val Leu Gln Cys Lys Gly Gly Gln Gln Pro Leu Gly Arg Ala
                165                 170                 175

ata tgt att gtc gac gtg tgc cac gcg ctc acc aca gag gat ggc act     693
Ile Cys Ile Val Asp Val Cys His Ala Leu Thr Thr Glu Asp Gly Thr
            180                 185                 190

gcg tat gaa cca gtg ccc gag ggc acc cgg tgt gga cca gag aag gtt     741
Ala Tyr Glu Pro Val Pro Glu Gly Thr Arg Cys Gly Pro Glu Lys Val
        195                 200                 205

tgc tgg aaa gga cgt tgc cag gac tta cac gtt tac aga tcc agc aac     789
Cys Trp Lys Gly Arg Cys Gln Asp Leu His Val Tyr Arg Ser Ser Asn
    210                 215                 220

tgc tct gcc cag tgc cac aac cat ggg gtg tgc aac cac aag cag gag     837
Cys Ser Ala Gln Cys His Asn His Gly Val Cys Asn His Lys Gln Glu
225                 230                 235                 240

tgc cac tgc cac gcg ggc tgg gcc ccg ccc cac tgc gcg aag ctg ctg     885
Cys His Cys His Ala Gly Trp Ala Pro Pro His Cys Ala Lys Leu Leu
                245                 250                 255

act gag gtg cac gca gcg tcc ggg aga tct tgt gac aaa act cac aca     933
Thr Glu Val His Ala Ala Ser Gly Arg Ser Cys Asp Lys Thr His Thr
            260                 265                 270

tgc cca ccg tgc cca gca cct gaa gcc gag ggc gcg ccg tca gtc ttc     981
Cys Pro Pro Cys Pro Ala Pro Glu Ala Glu Gly Ala Pro Ser Val Phe
        275                 280                 285

ctc ttc ccc cca aaa ccc aag gac acc ctc atg atc tcc cgg acc cct
1029
Leu Phe Pro Pro Lys Pro Lys Asp Thr Leu Met Ile Ser Arg Thr Pro
    290                 295                 300

gag gtc aca tgc gtg gtg gtg gac gtg agc cac gaa gac cct gag gtc
1077

Glu Val Thr Cys Val Val Val Asp Val Ser His Glu Asp Pro Glu Val
305                 310                 315                 320

aag ttc aac tgg tac gtg gac ggc gtg gag gtg cat aat gcc aag aca
1125
Lys Phe Asn Trp Tyr Val Asp Gly Val Glu Val His Asn Ala Lys Thr
                325                 330                 335

aag ccg cgg gag gag cag tac aac agc acg tac cgt gtg gtc agc gtc
1173
Lys Pro Arg Glu Glu Gln Tyr Asn Ser Thr Tyr Arg Val Val Ser Val
            340                 345                 350

ctc acc gtc ctg cac cag gac tgg ctg aat ggc aag gag tac aag tgc
1221
Leu Thr Val Leu His Gln Asp Trp Leu Asn Gly Lys Glu Tyr Lys Cys
        355                 360                 365

aag gtc tcc aac aaa gcc ctc cca gcc ccc atc gag aaa acc atc tcc
1269
Lys Val Ser Asn Lys Ala Leu Pro Ala Pro Ile Glu Lys Thr Ile Ser
    370                 375                 380
aaa gcc aaa ggg cag ccc cga gaa cca cag gtg tac acc ctg ccc cca
1317
Lys Ala Lys Gly Gln Pro Arg Glu Pro Gln Val Tyr Thr Leu Pro Pro
385                 390                 395                 400

tcc cgg gag gag atg acc aag aac cag gtc agc ctg acc tgc ctg gtc
1365
Ser Arg Glu Glu Met Thr Lys Asn Gln Val Ser Leu Thr Cys Leu Val
                405                 410                 415

aaa ggc ttc tat ccc agc gac atc gcc gtg gag tgg gag agc aat ggg
1413
Lys Gly Phe Tyr Pro Ser Asp Ile Ala Val Glu Trp Glu Ser Asn Gly
            420                 425                 430

cag ccg gag aac aac tac aag acc acg cct ccc gtg ctg gac tcc gac
1461
Gln Pro Glu Asn Asn Tyr Lys Thr Thr Pro Pro Val Leu Asp Ser Asp
        435                 440                 445

ggc tcc ttc ttc ctc tat agc aag ctc acc gtg gac aag agc agg tgg
1509
Gly Ser Phe Phe Leu Tyr Ser Lys Leu Thr Val Asp Lys Ser Arg Trp
    450                 455                 460

cag cag ggg aac gtc ttc tca tgc tcc gtg atg cat gag gct ctg cac
1557
Gln Gln Gly Asn Val Phe Ser Cys Ser Val Met His Glu Ala Leu His
465                 470                 475                 480

aac cac tac acg cag aag agc ctc tcc ctg tct ccg ggt aaa tga
1602
Asn His Tyr Thr Gln Lys Ser Leu Ser Leu Ser Pro Gly Lys
                485                 490                 495

actagagcgg ccgccaccgc ggtggagctc cagcttttgt tccctttagt gagggttaat
1662

ttcgagcttg gcgtaatcat ggtcatagct gtttcctg
1700

<210> 2
<211> 494
<212> PRT
<213> Artificial Sequence

<220>
<223> Description of Artificial Sequence: fusion
      polypeptide

<400> 2
Met Glu Thr Asp Thr Leu Leu Leu Trp Val Leu Leu Leu Trp Val Pro
  1               5                  10                  15
Gly Ser Thr Gly Thr Ser Cys Gly Asn Leu Phe Val Glu Arg Gly Glu
             20                  25                  30
Gln Cys Asp Cys Gly Pro Pro Glu Asp Cys Arg Asn Arg Cys Cys Asn
         35                  40                  45
Ser Thr Thr Cys Gln Leu Ala Glu Gly Ala Gln Cys Ala His Gly Thr
     50                  55                  60
Cys Cys Gln Glu Cys Lys Val Lys Pro Ala Gly Glu Leu Cys Arg Pro
 65                  70                  75                  80
Lys Lys Asp Met Cys Asp Leu Glu Glu Phe Cys Asp Gly Arg His Pro
                 85                  90                  95

Glu Cys Pro Glu Asp Ala Phe Gln Glu Asn Gly Thr Pro Cys Ser Gly
            100                 105                 110
Gly Tyr Cys Tyr Asn Gly Ala Cys Pro Thr Leu Ala Gln Gln Cys Gln
        115                 120                 125
Ala Phe Trp Gly Pro Gly Gly Gln Ala Ala Glu Glu Ser Cys Phe Ser
    130                 135                 140
Tyr Asp Ile Leu Pro Gly Cys Lys Ala Ser Arg Tyr Arg Ala Asp Met
145                 150                 155                 160
Cys Gly Val Leu Gln Cys Lys Gly Gly Gln Gln Pro Leu Gly Arg Ala
                165                 170                 175
Ile Cys Ile Val Asp Val Cys His Ala Leu Thr Thr Glu Asp Gly Thr
            180                 185                 190
Ala Tyr Glu Pro Val Pro Glu Gly Thr Arg Cys Gly Pro Glu Lys Val
        195                 200                 205
Cys Trp Lys Gly Arg Cys Gln Asp Leu His Val Tyr Arg Ser Ser Asn
    210                 215                 220
Cys Ser Ala Gln Cys His Asn His Gly Val Cys Asn His Lys Gln Glu
225                 230                 235                 240
Cys His Cys His Ala Gly Trp Ala Pro Pro His Cys Ala Lys Leu Leu
                245                 250                 255
Thr Glu Val His Ala Ala Ser Gly Arg Ser Cys Asp Lys Thr His Thr
            260                 265                 270
Cys Pro Pro Cys Pro Ala Pro Glu Ala Glu Gly Ala Pro Ser Val Phe
        275                 280                 285
Leu Phe Pro Pro Lys Pro Lys Asp Thr Leu Met Ile Ser Arg Thr Pro
    290                 295                 300
Glu Val Thr Cys Val Val Val Asp Val Ser His Glu Asp Pro Glu Val
305                 310                 315                 320
Lys Phe Asn Trp Tyr Val Asp Gly Val Glu Val His Asn Ala Lys Thr
                325                 330                 335
Lys Pro Arg Glu Glu Gln Tyr Asn Ser Thr Tyr Arg Val Val Ser Val
            340                 345                 350
Leu Thr Val Leu His Gln Asp Trp Leu Asn Gly Lys Glu Tyr Lys Cys
        355                 360                 365

Lys Val Ser Asn Lys Ala Leu Pro Ala Pro Ile Glu Lys Thr Ile Ser
    370                 375                 380
Lys Ala Lys Gly Gln Pro Arg Glu Pro Gln Val Tyr Thr Leu Pro Pro
385                 390                 395                 400
Ser Arg Glu Glu Met Thr Lys Asn Gln Val Ser Leu Thr Cys Leu Val
                405                 410                 415
Lys Gly Phe Tyr Pro Ser Asp Ile Ala Val Glu Trp Glu Ser Asn Gly
            420                 425                 430
Gln Pro Glu Asn Asn Tyr Lys Thr Thr Pro Pro Val Leu Asp Ser Asp
        435                 440                 445
Gly Ser Phe Phe Leu Tyr Ser Lys Leu Thr Val Asp Lys Ser Arg Trp
    450                 455                 460
Gln Gln Gly Asn Val Phe Ser Cys Ser Val Met His Glu Ala Leu His
465                 470                 475                 480
Asn His Tyr Thr Gln Lys Ser Leu Ser Leu Ser Pro Gly Lys
                485                 490


<210> 3
<211> 1668
<212> DNA
<213> Artificial Sequence

<220>
<223> Description of Artificial Sequence: fusion
      polypeptide

<220>
<221> CDS
<222> (46)..(1647)

<400> 3
ggtaccgggc ccccctcga ggtcgaccca agctggctag ccacc atg gag aca gac 57
                                                 Met Glu Thr Asp
                                                   1

aca ctc ctg cta tgg gta ctg ctg ctc tgg gtt cca ggt tcc act ggt    105
Thr Leu Leu Leu Trp Val Leu Leu Leu Trp Val Pro Gly Ser Thr Gly
  5                  10                  15                  20

act agt tgt ggt aat aag ttg gtg gac gct ggg gaa gag tgt gac tgt    153
Thr Ser Cys Gly Asn Lys Leu Val Asp Ala Gly Glu Glu Cys Asp Cys
                 25                  30                  35

ggt act cca aag gaa tgt gaa ttg gac cct tgc tgc gaa gga agt acc    201
Gly Thr Pro Lys Glu Cys Glu Leu Asp Pro Cys Cys Glu Gly Ser Thr
             40                  45                  50

tgt aag ctt aaa tca ttt gct gag tgt gca tat ggt gac tgt tgt aaa    249
Cys Lys Leu Lys Ser Phe Ala Glu Cys Ala Tyr Gly Asp Cys Cys Lys
         55                  60                  65

gac tgt cgg ttc ctt cca gga ggt act tta tgc cga gga aaa acc agt    297
Asp Cys Arg Phe Leu Pro Gly Gly Thr Leu Cys Arg Gly Lys Thr Ser
     70                  75                  80

gag tgt gat gtt cca gag tac tgc aat ggt tct tct cag ttc tgt cag    345
Glu Cys Asp Val Pro Glu Tyr Cys Asn Gly Ser Ser Gln Phe Cys Gln
 85                  90                  95                 100

cca gat gtt ttt att cag aat gga tat cct tgc cag aat aac aaa gcc    393

Pro Asp Val Phe Ile Gln Asn Gly Tyr Pro Cys Gln Asn Asn Lys Ala
                105                 110                 115

tat tgc tac aac ggc atg tgc cag tat tat gat gct caa tgt caa gtc     441
Tyr Cys Tyr Asn Gly Met Cys Gln Tyr Tyr Asp Ala Gln Cys Gln Val
            120                 125                 130

atc ttt ggc tca aaa gcc aag gct gcc ccc aaa gat tgt ttc att gaa     489
Ile Phe Gly Ser Lys Ala Lys Ala Ala Pro Lys Asp Cys Phe Ile Glu
        135                 140                 145

gtg aat tct aaa ggt gac aga ttt ggc aat tgt ggt ttc tct ggc aat     537
Val Asn Ser Lys Gly Asp Arg Phe Gly Asn Cys Gly Phe Ser Gly Asn
    150                 155                 160

gaa tac aag aag tgt gcc act ggg aat gct ttg tgt gga aag ctt cag     585
Glu Tyr Lys Lys Cys Ala Thr Gly Asn Ala Leu Cys Gly Lys Leu Gln
165                 170                 175                 180

tgt gag aat gta caa gag ata cct gta ttt gga att gtg cct gct att     633
Cys Glu Asn Val Gln Glu Ile Pro Val Phe Gly Ile Val Pro Ala Ile
                185                 190                 195

att caa acg cct agt cga ggc acc aaa tgt tgg ggt gtg gat ttc cag     681
Ile Gln Thr Pro Ser Arg Gly Thr Lys Cys Trp Gly Val Asp Phe Gln
            200                 205                 210

cta gga tca gat gtt cca gat cct ggg atg gtt aac gaa ggc aca aaa     729
Leu Gly Ser Asp Val Pro Asp Pro Gly Met Val Asn Glu Gly Thr Lys
        215                 220                 225

tgt ggt gct gga aag atc tgt aga aac ttc cag tgt gta gat gct tct     777
Cys Gly Ala Gly Lys Ile Cys Arg Asn Phe Gln Cys Val Asp Ala Ser
    230                 235                 240

gtt ctg aat tat gac tgt gat gtt cag aaa aag tgt cat gga cat ggg     825
Val Leu Asn Tyr Asp Cys Asp Val Gln Lys Lys Cys His Gly His Gly
245                 250                 255                 260

gta tgt aat agc aat aag aat tgt cac tgt gaa aat ggc tgg gct ccc     873
Val Cys Asn Ser Asn Lys Asn Cys His Cys Glu Asn Gly Trp Ala Pro
                265                 270                 275

cca aat tgt gag act aaa gga tac gga gga agt gtg gac agt gga cct     921
Pro Asn Cys Glu Thr Lys Gly Tyr Gly Gly Ser Val Asp Ser Gly Pro
            280                 285                 290

aca tac aat gaa atg aat act gca ttg agg gac gga tct tgt gac aaa     969
Thr Tyr Asn Glu Met Asn Thr Ala Leu Arg Asp Gly Ser Cys Asp Lys
        295                 300                 305

act cac aca tgc cca ccg tgc cca gca cct gaa gcc gag ggc gcg ccg
1017
Thr His Thr Cys Pro Pro Cys Pro Ala Pro Glu Ala Glu Gly Ala Pro
    310                 315                 320

tca gtc ttc ctc ttc ccc cca aaa ccc aag gac acc ctc atg atc tcc
1065
Ser Val Phe Leu Phe Pro Pro Lys Pro Lys Asp Thr Leu Met Ile Ser
325                 330                 335                 340

cgg acc cct gag gtc aca tgc gtg gtg gtg gac gtg agc cac gaa gac
1113
Arg Thr Pro Glu Val Thr Cys Val Val Val Asp Val Ser His Glu Asp
                345                 350                 355

cct gag gtc aag ttc aac tgg tac gtg gac ggc gtg gag gtg cat aat
1161
Pro Glu Val Lys Phe Asn Trp Tyr Val Asp Gly Val Glu Val His Asn
            360                 365                 370

gcc aag aca aag ccg cgg gag gag cag tac aac agc acg tac cgg gtg
1209
Ala Lys Thr Lys Pro Arg Glu Glu Gln Tyr Asn Ser Thr Tyr Arg Val
        375                 380                 385

gtc agc gtc ctc acc gtc ctg cac cag gac tgg ctg aat ggc aag gag
1257
Val Ser Val Leu Thr Val Leu His Gln Asp Trp Leu Asn Gly Lys Glu
    390                 395                 400

tac aag tgc aag gtc tcc aac aaa gcc ctc cca gcc ccc atc gag aaa
1305
Tyr Lys Cys Lys Val Ser Asn Lys Ala Leu Pro Ala Pro Ile Glu Lys
405                 410                 415                 420

acc atc tcc aaa gcc aaa ggg cag ccc cga gaa cca cag gtg tac acc
1353
Thr Ile Ser Lys Ala Lys Gly Gln Pro Arg Glu Pro Gln Val Tyr Thr
                425                 430                 435

ctg ccc cca tcc cgg gat gag ctg acc aag aac cag gtc agc ctg acc
1401
Leu Pro Pro Ser Arg Asp Glu Leu Thr Lys Asn Gln Val Ser Leu Thr
            440                 445                 450

tgc ctg gtc aaa ggc ttc tat ccc agc gac atc gcc gtg gag tgg gag
1449
Cys Leu Val Lys Gly Phe Tyr Pro Ser Asp Ile Ala Val Glu Trp Glu
        455                 460                 465

agc aat ggg cag ccg gag aac aac tac aag acc acg cct ccc gtg ctg
1497
Ser Asn Gly Gln Pro Glu Asn Asn Tyr Lys Thr Thr Pro Pro Val Leu
    470                 475                 480

gac tcc gac ggc tcc ttc ttc ctc tac agc aag ctc acc gtg gac aag
1545
Asp Ser Asp Gly Ser Phe Phe Leu Tyr Ser Lys Leu Thr Val Asp Lys
485                 490                 495                 500

agc agg tgg cag cag ggg aac gtc ttc tca tgc tcc gtg atg cat gag
1593
Ser Arg Trp Gln Gln Gly Asn Val Phe Ser Cys Ser Val Met His Glu
                505                 510                 515

gct ctg cac aac cac tac acg cag aag agc ctc tcc ctg tct ccg ggt
1641
Ala Leu His Asn His Tyr Thr Gln Lys Ser Leu Ser Leu Ser Pro Gly
            520                 525                 530

aaa tga actagagcgg ccgctacaga t
1668
Lys

<210> 4
<211> 533
<212> PRT
<213> Artificial Sequence

<220>
<223> Description of Artificial Sequence: fusion
      polypeptide

<400> 4
Met Glu Thr Asp Thr Leu Leu Leu Trp Val Leu Leu Leu Trp Val Pro
  1               5                  10                  15
Gly Ser Thr Gly Thr Ser Cys Gly Asn Lys Leu Val Asp Ala Gly Glu
             20                  25                  30
Glu Cys Asp Cys Gly Thr Pro Lys Glu Cys Glu Leu Asp Pro Cys Cys
         35                  40                  45
Glu Gly Ser Thr Cys Lys Leu Lys Ser Phe Ala Glu Cys Ala Tyr Gly
     50                  55                  60
Asp Cys Cys Lys Asp Cys Arg Phe Leu Pro Gly Gly Thr Leu Cys Arg
 65                  70                  75                  80
Gly Lys Thr Ser Glu Cys Asp Val Pro Glu Tyr Cys Asn Gly Ser Ser
                 85                  90                  95
Gln Phe Cys Gln Pro Asp Val Phe Ile Gln Asn Gly Tyr Pro Cys Gln
            100                 105                 110
Asn Asn Lys Ala Tyr Cys Tyr Asn Gly Met Cys Gln Tyr Tyr Asp Ala
        115                 120                 125
Gln Cys Gln Val Ile Phe Gly Ser Lys Ala Lys Ala Ala Pro Lys Asp
    130                 135                 140
Cys Phe Ile Glu Val Asn Ser Lys Gly Asp Arg Phe Gly Asn Cys Gly
145                 150                 155                 160
Phe Ser Gly Asn Glu Tyr Lys Lys Cys Ala Thr Gly Asn Ala Leu Cys
                165                 170                 175
Gly Lys Leu Gln Cys Glu Asn Val Gln Glu Ile Pro Val Phe Gly Ile
            180                 185                 190
Val Pro Ala Ile Ile Gln Thr Pro Ser Arg Gly Thr Lys Cys Trp Gly
        195                 200                 205
Val Asp Phe Gln Leu Gly Ser Asp Val Pro Asp Pro Gly Met Val Asn
    210                 215                 220
Glu Gly Thr Lys Cys Gly Ala Gly Lys Ile Cys Arg Asn Phe Gln Cys
225                 230                 235                 240
Val Asp Ala Ser Val Leu Asn Tyr Asp Cys Asp Val Gln Lys Lys Cys
                245                 250                 255
His Gly His Gly Val Cys Asn Ser Asn Lys Asn Cys His Cys Glu Asn
            260                 265                 270
Gly Trp Ala Pro Pro Asn Cys Glu Thr Lys Gly Tyr Gly Gly Ser Val
        275                 280                 285
Asp Ser Gly Pro Thr Tyr Asn Glu Met Asn Thr Ala Leu Arg Asp Gly
    290                 295                 300
Ser Cys Asp Lys Thr His Thr Cys Pro Pro Cys Pro Ala Pro Glu Ala
305                 310                 315                 320
Glu Gly Ala Pro Ser Val Phe Leu Phe Pro Pro Lys Pro Lys Asp Thr
                325                 330                 335
Leu Met Ile Ser Arg Thr Pro Glu Val Thr Cys Val Val Val Asp Val
            340                 345                 350
Ser His Glu Asp Pro Glu Val Lys Phe Asn Trp Tyr Val Asp Gly Val
        355                 360                 365

Glu Val His Asn Ala Lys Thr Lys Pro Arg Glu Glu Gln Tyr Asn Ser
    370                 375                 380
Thr Tyr Arg Val Val Ser Val Leu Thr Val Leu His Gln Asp Trp Leu
385                 390                 395                 400
Asn Gly Lys Glu Tyr Lys Cys Lys Val Ser Asn Lys Ala Leu Pro Ala
                405                 410                 415
Pro Ile Glu Lys Thr Ile Ser Lys Ala Lys Gly Gln Pro Arg Glu Pro
            420                 425                 430
Gln Val Tyr Thr Leu Pro Pro Ser Arg Asp Glu Leu Thr Lys Asn Gln
        435                 440                 445
Val Ser Leu Thr Cys Leu Val Lys Gly Phe Tyr Pro Ser Asp Ile Ala
    450                 455                 460
Val Glu Trp Glu Ser Asn Gly Gln Pro Glu Asn Asn Tyr Lys Thr Thr
465                 470                 475                 480
Pro Pro Val Leu Asp Ser Asp Gly Ser Phe Phe Leu Tyr Ser Lys Leu
                485                 490                 495
Thr Val Asp Lys Ser Arg Trp Gln Gln Gly Asn Val Phe Ser Cys Ser
            500                 505                 510
Val Met His Glu Ala Leu His Asn His Tyr Thr Gln Lys Ser Leu Ser
        515                 520                 525
Leu Ser Pro Gly Lys
    530

<210> 5
<211> 1443
<212> DNA
<213> Artificial Sequence

<220>
<223> Description of Artificial Sequence: fusion
      polypeptide

<220>
<221> CDS
<222> (25)..(1422)

<400> 5
gtcgacccaa gctggctagc cacc atg gag aca gac aca ctc ctg cta tgg      51
                           Met Glu Thr Asp Thr Leu Leu Leu Trp
                             1               5

gta ctg ctg ctc tgg gtt cca ggt tcc act ggt act agt tgt gga aat      99
Val Leu Leu Leu Trp Val Pro Gly Ser Thr Gly Thr Ser Cys Gly Asn
 10                  15                  20                  25

gga atg gta gaa caa ggt gaa gaa tgt gat tgt ggc tat agt gac cag     147
Gly Met Val Glu Gln Gly Glu Glu Cys Asp Cys Gly Tyr Ser Asp Gln
                 30                  35                  40

tgt aaa gat gaa tgc tgc ttc gat gca aat caa cca gag gga aga aaa     195
Cys Lys Asp Glu Cys Cys Phe Asp Ala Asn Gln Pro Glu Gly Arg Lys
             45                  50                  55

tgc aaa ctg aaa cct ggg aaa cag tgc agt cca agt caa ggt cct tgt     243
Cys Lys Leu Lys Pro Gly Lys Gln Cys Ser Pro Ser Gln Gly Pro Cys
         60                  65                  70

tgt aca gca cag tgt gca ttc aag tca aag tct gag aag tgt cgg gat     291
Cys Thr Ala Gln Cys Ala Phe Lys Ser Lys Ser Glu Lys Cys Arg Asp
     75                  80                  85

gat tca gac tgt gca agg gaa gga ata tgt aat ggc ttc aca gct ctc     339
Asp Ser Asp Cys Ala Arg Glu Gly Ile Cys Asn Gly Phe Thr Ala Leu
 90                  95                 100                 105

tgc cca gca tct gac cct aaa cca aac ttc aca gac tgt aat agg cat     387
Cys Pro Ala Ser Asp Pro Lys Pro Asn Phe Thr Asp Cys Asn Arg His
                110                 115                 120

aca caa gtg tgc att aat ggg caa tgt gca ggt tct atc tgt gag aaa     435
Thr Gln Val Cys Ile Asn Gly Gln Cys Ala Gly Ser Ile Cys Glu Lys
            125                 130                 135

tat ggc tta gag gag tgt acg tgt gcc agt tct gat ggc aaa gat gat     483
Tyr Gly Leu Glu Glu Cys Thr Cys Ala Ser Ser Asp Gly Lys Asp Asp
        140                 145                 150

aaa gaa tta tgc cat gta tgc tgt atg aag aaa atg gac cca tca act     531
Lys Glu Leu Cys His Val Cys Cys Met Lys Lys Met Asp Pro Ser Thr
    155                 160                 165

tgt gcc agt aca ggg tct gtg cag tgg agt agg cac ttc agt ggt cga     579
Cys Ala Ser Thr Gly Ser Val Gln Trp Ser Arg His Phe Ser Gly Arg
170                 175                 180                 185

acc atc acc ctg caa cct gga tcc cct tgc aac gat ttt aga ggt tac     627
Thr Ile Thr Leu Gln Pro Gly Ser Pro Cys Asn Asp Phe Arg Gly Tyr
                190                 195                 200

tgt gat gtt ttc atg cgg tgc aga tta gta gat gct gat ggt cct cta     675
Cys Asp Val Phe Met Arg Cys Arg Leu Val Asp Ala Asp Gly Pro Leu
            205                 210                 215

gct agg ctt aaa aaa gca att ttt agt cca gag ctc tat gaa aac att     723
Ala Arg Leu Lys Lys Ala Ile Phe Ser Pro Glu Leu Tyr Glu Asn Ile
        220                 225                 230

gct gaa aga tct tgt gac aaa act cac aca tgc cca ccg tgc cca gca     771
Ala Glu Arg Ser Cys Asp Lys Thr His Thr Cys Pro Pro Cys Pro Ala
    235                 240                 245

cct gaa gcc gag ggc gcg ccg tca gtc ttc ctc ttc ccc cca aaa ccc     819
Pro Glu Ala Glu Gly Ala Pro Ser Val Phe Leu Phe Pro Pro Lys Pro
250                 255                 260                 265

aag gac acc ctc atg atc tcc cgg acc cct gag gtc aca tgc gtg gtg     867
Lys Asp Thr Leu Met Ile Ser Arg Thr Pro Glu Val Thr Cys Val Val
                270                 275                 280

gtg gac gtg agc cac gaa gac cct gag gtc aag ttc aac tgg tac gtg     915
Val Asp Val Ser His Glu Asp Pro Glu Val Lys Phe Asn Trp Tyr Val
            285                 290                 295

gac ggc gtg gag gtg cat aat gcc aag aca aag ccg cgg gag gag cag     963
Asp Gly Val Glu Val His Asn Ala Lys Thr Lys Pro Arg Glu Glu Gln
        300                 305                 310

tac aac agc acg tac cgg gtg gtc agc gtc ctc acc gtc ctg cac cag
1011
Tyr Asn Ser Thr Tyr Arg Val Val Ser Val Leu Thr Val Leu His Gln
    315                 320                 325

gac tgg ctg aat ggc aag gag tac aag tgc aag gtc tcc aac aaa gcc
1059
Asp Trp Leu Asn Gly Lys Glu Tyr Lys Cys Lys Val Ser Asn Lys Ala
330                 335                 340                 345

ctc cca gcc ccc atc gag aaa acc atc tcc aaa gcc aaa ggg cag ccc
1107
Leu Pro Ala Pro Ile Glu Lys Thr Ile Ser Lys Ala Lys Gly Gln Pro
                350                 355                 360

cga gaa cca cag gtg tac acc ctg ccc cca tcc cgg gat gag ctg acc
1155

Arg Glu Pro Gln Val Tyr Thr Leu Pro Pro Ser Arg Asp Glu Leu Thr
            365                 370                 375

aag aac cag gtc agc ctg acc tgc ctg gtc aaa ggc ttc tat ccc agc
1203
Lys Asn Gln Val Ser Leu Thr Cys Leu Val Lys Gly Phe Tyr Pro Ser
        380                 385                 390

gac atc gcc gtg gag tgg gag agc aat ggg cag ccg gag aac aac tac
1251
Asp Ile Ala Val Glu Trp Glu Ser Asn Gly Gln Pro Glu Asn Asn Tyr
    395                 400                 405

aag acc acg cct ccc gtg ctg gac tcc gac ggc tcc ttc ttc ctc tac
1299
Lys Thr Thr Pro Pro Val Leu Asp Ser Asp Gly Ser Phe Phe Leu Tyr
410                 415                 420                 425

agc aag ctc acc gtg gac aag agc agg tgg cag cag ggg aac gtc ttc
1347
Ser Lys Leu Thr Val Asp Lys Ser Arg Trp Gln Gln Gly Asn Val Phe
                430                 435                 440

tca tgc tcc gtg atg cat gag gct ctg cac aac cac tac acg cag aag
1395
Ser Cys Ser Val Met His Glu Ala Leu His Asn His Tyr Thr Gln Lys
            445                 450                 455

agc ctc tcc ctg tct ccg ggt aaa tga actagagcgg ccgctacaga t
1443
Ser Leu Ser Leu Ser Pro Gly Lys
        460                 465


<210> 6
<211> 465
<212> PRT
<213> Artificial Sequence

<220>
<223> Description of Artificial Sequence: fusion
      polypeptide

<400> 6
Met Glu Thr Asp Thr Leu Leu Leu Trp Val Leu Leu Leu Trp Val Pro
  1               5                  10                  15
Gly Ser Thr Gly Thr Ser Cys Gly Asn Gly Met Val Glu Gln Gly Glu

                    20                  25                  30
Glu Cys Asp Cys Gly Tyr Ser Asp Gln Cys Lys Asp Glu Cys Cys Phe
            35                  40                  45
Asp Ala Asn Gln Pro Glu Gly Arg Lys Cys Lys Leu Lys Pro Gly Lys
        50                  55                  60
Gln Cys Ser Pro Ser Gln Gly Pro Cys Cys Thr Ala Gln Cys Ala Phe
65                  70                  75                  80
Lys Ser Lys Ser Glu Lys Cys Arg Asp Asp Ser Asp Cys Ala Arg Glu
                85                  90                  95
Gly Ile Cys Asn Gly Phe Thr Ala Leu Cys Pro Ala Ser Asp Pro Lys
            100                 105                 110
Pro Asn Phe Thr Asp Cys Asn Arg His Thr Gln Val Cys Ile Asn Gly
        115                 120                 125
Gln Cys Ala Gly Ser Ile Cys Glu Lys Tyr Gly Leu Glu Glu Cys Thr
    130                 135                 140
Cys Ala Ser Ser Asp Gly Lys Asp Asp Lys Glu Leu Cys His Val Cys
145                 150                 155                 160
Cys Met Lys Lys Met Asp Pro Ser Thr Cys Ala Ser Thr Gly Ser Val
                165                 170                 175
Gln Trp Ser Arg His Phe Ser Gly Arg Thr Ile Thr Leu Gln Pro Gly
            180                 185                 190
Ser Pro Cys Asn Asp Phe Arg Gly Tyr Cys Asp Val Phe Met Arg Cys
        195                 200                 205
Arg Leu Val Asp Ala Asp Gly Pro Leu Ala Arg Leu Lys Lys Ala Ile
    210                 215                 220
Phe Ser Pro Glu Leu Tyr Glu Asn Ile Ala Glu Arg Ser Cys Asp Lys
225                 230                 235                 240
Thr His Thr Cys Pro Pro Cys Pro Ala Pro Glu Ala Glu Gly Ala Pro
                245                 250                 255
Ser Val Phe Leu Phe Pro Pro Lys Pro Lys Asp Thr Leu Met Ile Ser
            260                 265                 270
Arg Thr Pro Glu Val Thr Cys Val Val Val Asp Val Ser His Glu Asp
        275                 280                 285
Pro Glu Val Lys Phe Asn Trp Tyr Val Asp Gly Val Glu Val His Asn
    290                 295                 300
Ala Lys Thr Lys Pro Arg Glu Glu Gln Tyr Asn Ser Thr Tyr Arg Val
305                 310                 315                 320
Val Ser Val Leu Thr Val Leu His Gln Asp Trp Leu Asn Gly Lys Glu
                325                 330                 335
Tyr Lys Cys Lys Val Ser Asn Lys Ala Leu Pro Ala Pro Ile Glu Lys
            340                 345                 350
Thr Ile Ser Lys Ala Lys Gly Gln Pro Arg Glu Pro Gln Val Tyr Thr
        355                 360                 365
Leu Pro Pro Ser Arg Asp Glu Leu Thr Lys Asn Gln Val Ser Leu Thr
    370                 375                 380
Cys Leu Val Lys Gly Phe Tyr Pro Ser Asp Ile Ala Val Glu Trp Glu
385                 390                 395                 400
Ser Asn Gly Gln Pro Glu Asn Asn Tyr Lys Thr Thr Pro Pro Val Leu
                405                 410                 415
Asp Ser Asp Gly Ser Phe Phe Leu Tyr Ser Lys Leu Thr Val Asp Lys
            420                 425                 430
Ser Arg Trp Gln Gln Gly Asn Val Phe Ser Cys Ser Val Met His Glu
        435                 440                 445
Ala Leu His Asn His Tyr Thr Gln Lys Ser Leu Ser Leu Ser Pro Gly
    450                 455                 460
Lys
465

<210> 7

<211> 1638
<212> DNA
<213> Artificial Sequence

<220>
<223> Description of Artificial Sequence: fusion
      polypeptide

<220>
<221> CDS
<222> (41)..(1609)

<400> 7
cgggcccccc ctcgaggtcg acccaagctg gctagccacc atg gag aca gac aca    55
                                            Met Glu Thr Asp Thr
                                             1               5

ctc ctg cta tgg gta ctg ctg ctc tgg gtt cca ggt tcc act ggt act    103
Leu Leu Leu Trp Val Leu Leu Leu Trp Val Pro Gly Ser Thr Gly Thr
                10                  15                  20

agt tgc gga aat atg ttt gtg gag ccg ggc gag cag tgt gac tgt ggc    151
Ser Cys Gly Asn Met Phe Val Glu Pro Gly Glu Gln Cys Asp Cys Gly
            25                  30                  35

ttc ctg gat gac tgc gtc gat ccc tgc tgt gat tct ttg acc tgc cag    199
Phe Leu Asp Asp Cys Val Asp Pro Cys Cys Asp Ser Leu Thr Cys Gln
        40                  45                  50

ctg agg cca ggt gca cag tgt gca tct gac gga ccc tgt tgt caa aat    247
Leu Arg Pro Gly Ala Gln Cys Ala Ser Asp Gly Pro Cys Cys Gln Asn
    55                  60                  65

tgc cag ctg cgc ccg tct ggc tgg cag tgt cgt cct acc aga ggg gat    295
Cys Gln Leu Arg Pro Ser Gly Trp Gln Cys Arg Pro Thr Arg Gly Asp
70                  75                  80                  85

tgt gac ttg cct gaa ttc tgc cca gga gac agc tcc cag tgt ccc cct    343
Cys Asp Leu Pro Glu Phe Cys Pro Gly Asp Ser Ser Gln Cys Pro Pro
                90                  95                  100

gat gtc agc cta ggg gat ggc gag ccc tgc gct ggc ggg caa gct gtg    391
Asp Val Ser Leu Gly Asp Gly Glu Pro Cys Ala Gly Gly Gln Ala Val
            105                 110                 115

tgc atg cac ggg cgt tgt gcc tcc tat gcc cag cag tgc cag tca ctt    439
Cys Met His Gly Arg Cys Ala Ser Tyr Ala Gln Gln Cys Gln Ser Leu
        120                 125                 130

tgg gga cct gga gcc cag ccc gct gcg cca ctt tgc ctc cag aca gct    487
Trp Gly Pro Gly Ala Gln Pro Ala Ala Pro Leu Cys Leu Gln Thr Ala
    135                 140                 145

aat act cgg gga aat gct ttt ggg agc tgt ggg cgc aac ccc agt ggc    535
Asn Thr Arg Gly Asn Ala Phe Gly Ser Cys Gly Arg Asn Pro Ser Gly
150                 155                 160                 165

agt tat gtg tcc tgc acc cct aga gat gcc att tgt ggg cag ctc cag    583
Ser Tyr Val Ser Cys Thr Pro Arg Asp Ala Ile Cys Gly Gln Leu Gln
                170                 175                 180

tgc cag aca ggt agg acc cag cct ctg ctg ggc tcc atc cgg gat cta     631
Cys Gln Thr Gly Arg Thr Gln Pro Leu Leu Gly Ser Ile Arg Asp Leu
            185                 190                 195

ctc tgg gag aca ata gat gtg aat ggg act gag ctg aac tgc agc tgg     679
Leu Trp Glu Thr Ile Asp Val Asn Gly Thr Glu Leu Asn Cys Ser Trp
        200                 205                 210

gtg cac ctg gac ctg ggc agt gat gtg gcc cag ccc ctc ctg act ctg     727
Val His Leu Asp Leu Gly Ser Asp Val Ala Gln Pro Leu Leu Thr Leu
    215                 220                 225

cct ggc aca gcc tgt ggc cct ggc ctg gtg tgt ata gac cat cga tgc     775
Pro Gly Thr Ala Cys Gly Pro Gly Leu Val Cys Ile Asp His Arg Cys
230                 235                 240                 245

cag cgt gtg gat ctc ctg ggg gca cag gaa tgt cga agc aaa tgc cat     823
Gln Arg Val Asp Leu Leu Gly Ala Gln Glu Cys Arg Ser Lys Cys His
                250                 255                 260

gga cat ggg gtc tgt gac agc aac agg cac tgc tac tgt gag gag ggc     871
Gly His Gly Val Cys Asp Ser Asn Arg His Cys Tyr Cys Glu Glu Gly
            265                 270                 275

tgg gca ccc cct gac tgc acc act cag ctc aaa gca acc agc tcc aga     919
Trp Ala Pro Pro Asp Cys Thr Thr Gln Leu Lys Ala Thr Ser Ser Arg
        280                 285                 290

tct tgt gac aaa act cac aca tgc cca ccg tgc cca gca cct gaa gcc     967
Ser Cys Asp Lys Thr His Thr Cys Pro Pro Cys Pro Ala Pro Glu Ala
    295                 300                 305

gag ggc gcg ccg tca gtc ttc ctc ttc ccc cca aaa ccc aag gac acc
1015
Glu Gly Ala Pro Ser Val Phe Leu Phe Pro Pro Lys Pro Lys Asp Thr
310                 315                 320                 325

ctc atg atc tcc cgg acc cct gag gtc aca tgc gtg gtg gtg gac gtg
1063
Leu Met Ile Ser Arg Thr Pro Glu Val Thr Cys Val Val Val Asp Val
                330                 335                 340

agc cac gaa gac cct gag gtc aag ttc aac tgg tac gtg gac ggc gtg
1111
Ser His Glu Asp Pro Glu Val Lys Phe Asn Trp Tyr Val Asp Gly Val
            345                 350                 355

gag gtg cat aat gcc aag aca aag ccg cgg gag gag cag tac aac agc
1159
Glu Val His Asn Ala Lys Thr Lys Pro Arg Glu Glu Gln Tyr Asn Ser
        360                 365                 370

acg tac cgt gtg gtc agc gtc ctc acc gtc ctg cac cag gac tgg ctg
1207
Thr Tyr Arg Val Val Ser Val Leu Thr Val Leu His Gln Asp Trp Leu
    375                 380                 385

aat ggc aag gag tac aag tgc aag gtc tcc aac aaa gcc ctc cca gcc
1255
Asn Gly Lys Glu Tyr Lys Cys Lys Val Ser Asn Lys Ala Leu Pro Ala
390                 395                 400                 405

ccc atc gag aaa acc atc tcc aaa gcc aaa ggg cag ccc cga gaa cca
1303
Pro Ile Glu Lys Thr Ile Ser Lys Ala Lys Gly Gln Pro Arg Glu Pro
                410                 415                 420

cag gtg tac acc ctg ccc cca tcc cgg gag gag atg acc aag aac cag
1351
Gln Val Tyr Thr Leu Pro Pro Ser Arg Glu Glu Met Thr Lys Asn Gln
            425                 430                 435

gtc agc ctg acc tgc ctg gtc aaa ggc ttc tat ccc agc gac atc gcc
1399
Val Ser Leu Thr Cys Leu Val Lys Gly Phe Tyr Pro Ser Asp Ile Ala
        440                 445                 450

gtg gag tgg gag agc aat ggg cag ccg gag aac aac tac aag acc acg
1447
Val Glu Trp Glu Ser Asn Gly Gln Pro Glu Asn Asn Tyr Lys Thr Thr
    455                 460                 465

cct ccc gtg ctg gac tcc gac ggc tcc ttc ttc ctc tat agc aag ctc
1495
Pro Pro Val Leu Asp Ser Asp Gly Ser Phe Phe Leu Tyr Ser Lys Leu
470                 475                 480                 485

acc gtg gac aag agc agg tgg cag cag ggg aac gtc ttc tca tgc tcc
1543
Thr Val Asp Lys Ser Arg Trp Gln Gln Gly Asn Val Phe Ser Cys Ser
                490                 495                 500

gtg atg cat gag gct ctg cac aac cac tac acg cag aag agc ctc tcc
1591
Val Met His Glu Ala Leu His Asn His Tyr Thr Gln Lys Ser Leu Ser
            505                 510                 515

ctg tct ccg ggt aaa tga actagagcgg ccgccaccgc ggtggagct
1638
Leu Ser Pro Gly Lys
        520


<210> 8
<211> 522
<212> PRT
<213> Artificial Sequence

<220>
<223> Description of Artificial Sequence: fusion
      polypeptide

<400> 8
Met Glu Thr Asp Thr Leu Leu Leu Trp Val Leu Leu Leu Trp Val Pro
  1               5                  10                  15
Gly Ser Thr Gly Thr Ser Cys Gly Asn Met Phe Val Glu Pro Gly Glu
             20                  25                  30
Gln Cys Asp Cys Gly Phe Leu Asp Asp Cys Val Asp Pro Cys Cys Asp
         35                  40                  45
Ser Leu Thr Cys Gln Leu Arg Pro Gly Ala Gln Cys Ala Ser Asp Gly
     50                  55                  60
Pro Cys Cys Gln Asn Cys Gln Leu Arg Pro Ser Gly Trp Gln Cys Arg

65                  70                  75                  80
Pro Thr Arg Gly Asp Cys Asp Leu Pro Glu Phe Cys Pro Gly Asp Ser
                85                  90                  95
Ser Gln Cys Pro Pro Asp Val Ser Leu Gly Asp Gly Glu Pro Cys Ala
            100                 105                 110
Gly Gly Gln Ala Val Cys Met His Gly Arg Cys Ala Ser Tyr Ala Gln
        115                 120                 125
Gln Cys Gln Ser Leu Trp Gly Pro Gly Ala Gln Pro Ala Ala Pro Leu
    130                 135                 140
Cys Leu Gln Thr Ala Asn Thr Arg Gly Asn Ala Phe Gly Ser Cys Gly
145                 150                 155                 160
Arg Asn Pro Ser Gly Ser Tyr Val Ser Cys Thr Pro Arg Asp Ala Ile
                165                 170                 175
Cys Gly Gln Leu Gln Cys Gln Thr Gly Arg Thr Gln Pro Leu Leu Gly
            180                 185                 190
Ser Ile Arg Asp Leu Leu Trp Glu Thr Ile Asp Val Asn Gly Thr Glu
        195                 200                 205
Leu Asn Cys Ser Trp Val His Leu Asp Leu Gly Ser Asp Val Ala Gln
    210                 215                 220
Pro Leu Leu Thr Leu Pro Gly Thr Ala Cys Gly Pro Gly Leu Val Cys
225                 230                 235                 240
Ile Asp His Arg Cys Gln Arg Val Asp Leu Leu Gly Ala Gln Glu Cys
                245                 250                 255
Arg Ser Lys Cys His Gly His Gly Val Cys Asp Ser Asn Arg His Cys
            260                 265                 270
Tyr Cys Glu Glu Gly Trp Ala Pro Pro Asp Cys Thr Thr Gln Leu Lys
        275                 280                 285
Ala Thr Ser Ser Arg Ser Cys Asp Lys Thr His Thr Cys Pro Pro Cys
    290                 295                 300
Pro Ala Pro Glu Ala Glu Gly Ala Pro Ser Val Phe Leu Phe Pro Pro
305                 310                 315                 320
Lys Pro Lys Asp Thr Leu Met Ile Ser Arg Thr Pro Glu Val Thr Cys
                325                 330                 335
Val Val Val Asp Val Ser His Glu Asp Pro Glu Val Lys Phe Asn Trp
            340                 345                 350
Tyr Val Asp Gly Val Glu Val His Asn Ala Lys Thr Lys Pro Arg Glu
        355                 360                 365
Glu Gln Tyr Asn Ser Thr Tyr Arg Val Val Ser Val Leu Thr Val Leu
    370                 375                 380
His Gln Asp Trp Leu Asn Gly Lys Glu Tyr Lys Cys Lys Val Ser Asn
385                 390                 395                 400
Lys Ala Leu Pro Ala Pro Ile Glu Lys Thr Ile Ser Lys Ala Lys Gly
                405                 410                 415
Gln Pro Arg Glu Pro Gln Val Tyr Thr Leu Pro Pro Ser Arg Glu Glu
            420                 425                 430
Met Thr Lys Asn Gln Val Ser Leu Thr Cys Leu Val Lys Gly Phe Tyr
        435                 440                 445
Pro Ser Asp Ile Ala Val Glu Trp Glu Ser Asn Gly Gln Pro Glu Asn
    450                 455                 460
Asn Tyr Lys Thr Thr Pro Pro Val Leu Asp Ser Asp Gly Ser Phe Phe
465                 470                 475                 480
Leu Tyr Ser Lys Leu Thr Val Asp Lys Ser Arg Trp Gln Gln Gly Asn
                485                 490                 495
Val Phe Ser Cys Ser Val Met His Glu Ala Leu His Asn His Tyr Thr
            500                 505                 510
Gln Lys Ser Leu Ser Leu Ser Pro Gly Lys
        515                 520

<210> 9
<211> 1386

<212> DNA
<213> Artificial Sequence

<220>
<223> Description of Artificial Sequence: fusion
      polypeptide

<220>
<221> CDS
<222> (25)..(1365)

<400> 9
gtcgacccaa gctggctagc cacc atg gag aca gac aca ctc ctg cta tgg     51
                           Met Glu Thr Asp Thr Leu Leu Leu Trp
                             1               5

gta ctg ctg ctc tgg gtt cca ggt tcc act ggt act agt tgt ggg aac     99
Val Leu Leu Leu Trp Val Pro Gly Ser Thr Gly Thr Ser Cys Gly Asn
 10                  15                  20                  25

tcg agg gtg gat gaa gga gaa gag tgt gat cct ggc atc atg tat ctg    147
Ser Arg Val Asp Glu Gly Glu Glu Cys Asp Pro Gly Ile Met Tyr Leu
                 30                  35                  40

aac aac gac acc tgc tgc aac agc gac tgc acg ttg aag gaa ggt gtc    195
Asn Asn Asp Thr Cys Cys Asn Ser Asp Cys Thr Leu Lys Glu Gly Val
             45                  50                  55

cag tgc agt gac agg aac agt cct tgc tgt aaa aac tgt cag ttt gag    243
Gln Cys Ser Asp Arg Asn Ser Pro Cys Cys Lys Asn Cys Gln Phe Glu
         60                  65                  70

act gcc cag aag aag tgc cag gag gcg att aat gct act tgc aaa ggc    291
Thr Ala Gln Lys Lys Cys Gln Glu Ala Ile Asn Ala Thr Cys Lys Gly
     75                  80                  85

gtg tcc tac tgc aca ggt aat agc agt gag tgc ccg cct cca gga aat    339
Val Ser Tyr Cys Thr Gly Asn Ser Ser Glu Cys Pro Pro Pro Gly Asn
 90                  95                 100                 105

gct gaa gat gac act gtt tgc ttg gat ctt ggc aag tgt aag gat ggg    387
Ala Glu Asp Asp Thr Val Cys Leu Asp Leu Gly Lys Cys Lys Asp Gly
                110                 115                 120

aaa tgc atc cct ttc tgc gag agg gaa cag cag ctg gag tcc tgt gca    435
Lys Cys Ile Pro Phe Cys Glu Arg Glu Gln Gln Leu Glu Ser Cys Ala
            125                 130                 135

tgt aat gaa act gac aac tcc tgc aag gtg tgc tgc agg gac ctt tcc    483
Cys Asn Glu Thr Asp Asn Ser Cys Lys Val Cys Cys Arg Asp Leu Ser
        140                 145                 150

ggc cgc tgt gtg ccc tat gtc gat gct gaa caa aag aac tta ttt ttg    531
Gly Arg Cys Val Pro Tyr Val Asp Ala Glu Gln Lys Asn Leu Phe Leu
    155                 160                 165

agg aaa gga aag ccc tgt aca gta gga ttt tgt gac atg aat ggc aaa    579
Arg Lys Gly Lys Pro Cys Thr Val Gly Phe Cys Asp Met Asn Gly Lys
170                 175                 180                 185

tgt gag aaa cga gta cag gat gta att gaa cga ttt tgg gat ttc att    627

Cys Glu Lys Arg Val Gln Asp Val Ile Glu Arg Phe Trp Asp Phe Ile
                190                 195                 200

gac cag ctg agc atc aat act ttt gga aag ttt tta gca gac aac aga     675
Asp Gln Leu Ser Ile Asn Thr Phe Gly Lys Phe Leu Ala Asp Asn Arg
            205                 210                 215

tct tgt gac aaa act cac aca tgc cca ccg tgc cca gca cct gaa gcc     723
Ser Cys Asp Lys Thr His Thr Cys Pro Pro Cys Pro Ala Pro Glu Ala
        220                 225                 230

gag ggc gcg ccg tca gtc ttc ctc ttc ccc cca aaa ccc aag gac acc     771
Glu Gly Ala Pro Ser Val Phe Leu Phe Pro Pro Lys Pro Lys Asp Thr
    235                 240                 245

ctc atg atc tcc cgg acc cct gag gtc aca tgc gtg gtg gtg gac gtg     819
Leu Met Ile Ser Arg Thr Pro Glu Val Thr Cys Val Val Val Asp Val
250                 255                 260                 265

agc cac gaa gac cct gag gtc aag ttc aac tgg tac gtg gac ggc gtg     867
Ser His Glu Asp Pro Glu Val Lys Phe Asn Trp Tyr Val Asp Gly Val
                270                 275                 280

gag gtg cat aat gcc aag aca aag ccg cgg gag gag cag tac aac agc     915
Glu Val His Asn Ala Lys Thr Lys Pro Arg Glu Glu Gln Tyr Asn Ser
            285                 290                 295

acg tac cgg gtg gtc agc gtc ctc acc gtc ctg cac cag gac tgg ctg     963
Thr Tyr Arg Val Val Ser Val Leu Thr Val Leu His Gln Asp Trp Leu
        300                 305                 310

aat ggc aag gag tac aag tgc aag gtc tcc aac aaa gcc ctc cca gcc
1011
Asn Gly Lys Glu Tyr Lys Cys Lys Val Ser Asn Lys Ala Leu Pro Ala
    315                 320                 325

ccc atc gag aaa acc atc tcc aaa gcc aaa ggg cag ccc cga gaa cca
1059
Pro Ile Glu Lys Thr Ile Ser Lys Ala Lys Gly Gln Pro Arg Glu Pro
330                 335                 340                 345

cag gtg tac acc ctg ccc cca tcc cgg gat gag ctg acc aag aac cag
1107
Gln Val Tyr Thr Leu Pro Pro Ser Arg Asp Glu Leu Thr Lys Asn Gln
                350                 355                 360

gtc agc ctg acc tgc ctg gtc aaa ggc ttc tat ccc agc gac atc gcc
1155
Val Ser Leu Thr Cys Leu Val Lys Gly Phe Tyr Pro Ser Asp Ile Ala
            365                 370                 375

gtg gag tgg gag agc aat ggg cag ccg gag aac aac tac aag acc acg
1203
Val Glu Trp Glu Ser Asn Gly Gln Pro Glu Asn Asn Tyr Lys Thr Thr
        380                 385                 390

cct ccc gtg ctg gac tcc gac ggc tcc ttc ttc ctc tac agc aag ctc
1251
Pro Pro Val Leu Asp Ser Asp Gly Ser Phe Phe Leu Tyr Ser Lys Leu
    395                 400                 405

acc gtg gac aag agc agg tgg cag cag ggg aac gtc ttc tca tgc tcc
1299
Thr Val Asp Lys Ser Arg Trp Gln Gln Gly Asn Val Phe Ser Cys Ser
410                 415                 420                 425

gtg atg cat gag gct ctg cac aac cac tac acg cag aag agc ctc tcc
1347

Val Met His Glu Ala Leu His Asn His Tyr Thr Gln Lys Ser Leu Ser
                430                 435                 440

ctg tct ccg ggt aaa tga actagagcgg ccgctacaga t
1386
Leu Ser Pro Gly Lys
            445



<210> 10
<211> 446
<212> PRT
<213> Artificial Sequence

<220>
<223> Description of Artificial Sequence: fusion
      polypeptide

<400> 10
Met Glu Thr Asp Thr Leu Leu Leu Trp Val Leu Leu Leu Trp Val Pro
  1               5                  10                  15
Gly Ser Thr Gly Thr Ser Cys Gly Asn Ser Arg Val Asp Glu Gly Glu
             20                  25                  30
Glu Cys Asp Pro Gly Ile Met Tyr Leu Asn Asn Asp Thr Cys Cys Asn
         35                  40                  45
Ser Asp Cys Thr Leu Lys Glu Gly Val Gln Cys Ser Asp Arg Asn Ser
     50                  55                  60
Pro Cys Cys Lys Asn Cys Gln Phe Glu Thr Ala Gln Lys Lys Cys Gln
 65                  70                  75                  80
Glu Ala Ile Asn Ala Thr Cys Lys Gly Val Ser Tyr Cys Thr Gly Asn
                 85                  90                  95
Ser Ser Glu Cys Pro Pro Pro Gly Asn Ala Glu Asp Asp Thr Val Cys
            100                 105                 110
Leu Asp Leu Gly Lys Cys Lys Asp Gly Lys Cys Ile Pro Phe Cys Glu
        115                 120                 125
Arg Glu Gln Gln Leu Glu Ser Cys Ala Cys Asn Glu Thr Asp Asn Ser
    130                 135                 140
Cys Lys Val Cys Cys Arg Asp Leu Ser Gly Arg Cys Val Pro Tyr Val
145                 150                 155                 160
Asp Ala Glu Gln Lys Asn Leu Phe Leu Arg Lys Gly Lys Pro Cys Thr
                165                 170                 175
Val Gly Phe Cys Asp Met Asn Gly Lys Cys Glu Lys Arg Val Gln Asp
            180                 185                 190
Val Ile Glu Arg Phe Trp Asp Phe Ile Asp Gln Leu Ser Ile Asn Thr
        195                 200                 205
Phe Gly Lys Phe Leu Ala Asp Asn Arg Ser Cys Asp Lys Thr His Thr
    210                 215                 220
Cys Pro Pro Cys Pro Ala Pro Glu Ala Glu Gly Ala Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210> 11
<211> 1653
<212> DNA
<213> Artificial Sequence

<220>
<223> Description of Artificial Sequence: fusion
      polypeptide

<220>
<221> CDS
<222> (25)..(1632)

<400> 11
gtcgacccaa gctggctagc cacc atg gag aca gac aca ctc ctg cta tgg     51
gta ctg ctg ctc tgg gtt cca ggt tcc act ggt act agt tgt ggg aat     99
cta gtg gtt gaa gaa ggg gag gaa tgt gac tgt gga acc ata cgg cag    147
tgt gca aaa gat ccc tgt tgt ctg tta aac tgt act cta cat cct ggg    195
gct gct tgt gct ttt gga ata tgt tgc aaa gac tgc aaa ttt ctg cca    243
tca gga act tta tgt aga caa caa gtt ggt gaa tgt gac ctt cca gag    291
tgg tgc aat ggg aca tcc cat caa tgc cca gat gat gtg tat gtg cag 339
gac ggg atc tcc tgt aat gtg aat gcc ttc tgc tat gaa aag acg tgt 387
aat aac cat gat ata caa tgt aaa gag att ttt ggc caa gat gca agg 435
agt gca tct cag agt tgc tac caa gaa atc aac acc caa gga aac cgt 483
ttc ggt cac tgt ggt att gta ggc aca aca tat gta aaa tgt tgg acc 531
cct gat atc atg tgt ggg agg gtt cag tgt gaa aat gtg gga gta att 579
ccc aat ctg ata gag cat tct aca gtg cag cag ttt cac ctc aat gac 627
acc act tgc tgg ggc act gat tat cat tta ggg atg gct ata cct gat 675
att ggt gag gtg aaa gat ggc aca gta tgt ggt cca gaa aag atc tgc 723
atc cgt aag aag tgt gcc agt atg gtt cat ctg tca caa gcc tgt cag 771
cct aag acc tgc aac atg agg gga atc tgc aac aac aaa caa cac tgt 819
cac tgc aac cat gaa tgg gca ccc cca tac tgc aag gac aaa ggc tat 867
gga ggt agt gct gat agt ggc cca cct cct aag aac aac atg gaa gga 915
tta aat gtg atg gga aag ttg cgt gga tct tgt gac aaa act cac aca 963
tgc cca ccg tgc cca gca cct gaa gcc gag ggc gcg ccg tca gtc ttc
1011
ctc ttc ccc cca aaa ccc aag gac acc ctc atg atc tcc cgg acc cct
1059
gag gtc aca tgc gtg gtg gtg gac gtg agc cac gaa gac cct gag gtc
1107
aag ttc aac tgg tac gtg gac ggc gtg gag gtg cat aat gcc aag aca
1155
aag ccg cgg gag gag cag tac aac agc acg tac cgg gtg gtc agc gtc
1203
ctc acc gtc ctg cac cag gac tgg ctg aat ggc aag gag tac aag tgc
1251
aag gtc tcc aac aaa gcc ctc cca gcc ccc atc gag aaa acc atc tcc
1299
aaa gcc aaa ggg cag ccc cga gaa cca cag gtg tac acc ctg ccc cca
1347
tcc cgg gat gag ctg acc aag aac cag gtc agc ctg acc tgc ctg gtc
1395
aaa ggc ttc tat ccc agc gac atc gcc gtg gag tgg gag agc aat ggg
1443
cag ccg gag aac aac tac aag acc acg cct ccc gtg ctg gac tcc gac
1491
ggc tcc ttc ttc ctc tac agc aag ctc acc gtg gac aag agc agg tgg
1539
cag cag ggg aac gtc ttc tca tgc tcc gtg atg cat gag gct ctg cac
1587
aac cac tac acg cag aag agc ctc tcc ctg tct ccg ggt aaa tga
1632
actagagcgg ccgctacaga t
1653



<210> 12
<211> 535
<212> PRT
<213> Artificial Sequence

<220>
<223> Description of Artificial Sequence: fusion
      polypeptide

<400> 12

<210> 13
<211> 1617
<212> DNA
<213> Artificial Sequence

<220>
<223> Description of Artificial Sequence: fusion
      polypeptide

<220>
<221> CDS
<222> (25)..(1596)

<400> 13
gtcgacccaa gctggctagc cacc atg gag aca gac aca ctc ctg cta tgg     51
gta ctg ctg ctc tgg gtt cca ggt tcc act ggt act agt tgt ggg aat     99
ggt gtg gtt gaa aga gaa gag cag tgt gac tgt gga tcc gta cag cag    147
tgt gaa caa gac gcc tgt tgt ctg ttg aac tgc act cta agg cct ggg    195
gct gcc tgt gct ttt ggg ctt tgt tgc aaa gac tgc aag ttc atg cca    243
tca ggg gaa ctc tgt aga caa gag gtc aat gaa tgt gac ctt cca gaa 291
tgg tgc aat gga aca tct cat cag tgt cca gaa gat aga tat gtg cag 339
gac ggg atc ccc tgt agt gac agt gcc tac tgc tat caa aag agg tgt 387
aat aac cat gac cag cat tgc agg gag att ttt ggt aaa gat gca aaa 435
agt gca tct cag aat tgc tat aaa gaa atc aac tct cag gga aac cgt 483
ttt ggt cac tgt ggt ata aat ggc aca aca tac cta aaa tgt cat atc 531
tct gat gtc ttt tgt ggg aga gtt caa tgt gag aat gtg aga gac att 579
cct ctt ctc caa gat cat ttt act ttg cag cac act cat atc aat ggt 627
gtc acc tgc tgg ggt att gac tat cat tta agg atg aac ata tct gac 675
att ggt gaa gtg aaa gat ggt act gtg tgt ggc cca gga aag atc tgc 723
atc cat aag aag tgt gtc agt ctg tct gtc ttg tca cat gtc tgc ctt 771
cct gag acc tgc aat atg aag ggg atc tgc aat aac aaa cat cac tgc 819
cac tgt ggc tat ggg tgg tcc cca ccc tac tgc cag cac aga ggc tat 867
ggg ggc agt att gac agt ggc cca gca tct gca aag aga tct tgt gac 915
aaa act cac aca tgc cca ccg tgc cca gca cct gaa gcc gag ggc gcg 963
ccg tca gtc ttc ctc ttc ccc cca aaa ccc aag gac acc ctc atg atc
1011
tcc cgg acc cct gag gtc aca tgc gtg gtg gtg gac gtg agc cac gaa
1059
gac cct gag gtc aag ttc aac tgg tac gtg gac ggc gtg gag gtg cat
1107
aat gcc aag aca aag ccg cgg gag gag cag tac aac agc acg tac cgg
1155
gtg gtc agc gtc ctc acc gtc ctg cac cag gac tgg ctg aat ggc aag
1203
gag tac aag tgc aag gtc tcc aac aaa gcc ctc cca gcc ccc atc gag
1251
aaa acc atc tcc aaa gcc aaa ggg cag ccc cga gaa cca cag gtg tac
1299
acc ctg ccc cca tcc cgg gat gag ctg acc aag aac cag gtc agc ctg
1347
acc tgc ctg gtc aaa ggc ttc tat ccc agc gac atc gcc gtg gag tgg
1395
gag agc aat ggg cag ccg gag aac aac tac aag acc acg cct ccc gtg
1443
ctg gac tcc gac ggc tcc ttc ttc ctc tac agc aag ctc acc gtg gac
1491
aag agc agg tgg cag cag ggg aac gtc ttc tca tgc tcc gtg atg cat
1539
gag gct ctg cac aac cac tac acg cag aag agc ctc tcc ctg tct ccg
1587
ggt aaa tga actagagcgg ccgctacaga t
1617

<210> 14
<211> 523
<212> PRT
<213> Artificial Sequence

<220>
<223> Description of Artificial Sequence: fusion
      polypeptide

<400> 14


<210> 15
<211> 1674
<212> DNA
<213> Artificial Sequence

<220>
<223> Description of Artificial Sequence: fusion
      polypeptide

<220>
<221> CDS
<222> (25)..(1653)

<400> 15
gtcgacccaa gctggctagc cacc atg gag aca gac aca ctc ctg cta tgg      51
gta ctg ctg ctc tgg gtt cca ggt tcc act ggt act agt tgt ggc aat      99
ggc ttc att gaa act gga gag gag tgt gat tgt gga acc ccg gcc gaa     147
tgt gtc ctt gaa gga gca gag tgt tgt aag aaa tgc acc ttg act caa     195
gac tct caa tgc agt gac ggt ctt tgc tgt aaa aag tgc aag ttt cag     243
cct atg ggc act gtg tgc cga gaa gca gta aat gat tgt gat att cgt     291
gaa acg tgc tca gga aat tca agc cag tgt gcc cct aat att cat aaa     339
atg gat gga tat tca tgt gat ggt gtt cag gga att tgc ttt gga gga     387
aga tgc aaa acc aga gat aga caa tgc aaa tac att tgg ggg caa aag     435
gtg aca gca tca gac aaa tat tgc tat gag aaa ctg aat att gaa ggg     483
acg gag aag ggt aac tgt ggg aaa gac aaa gac aca tgg ata cag tgc     531
aac aaa cgg gat gtg ctt tgt ggt tac ctt ttg tgt acc aat att ggc     579
aat atc cca agg ctt gga gaa ctc gat ggt gaa atc aca tct act tta     627
gtt gtg cag caa gga aga aca tta aac tgc agt ggt ggg cat gtt aag     675
ctt gaa gaa gat gta gat ctt ggc tat gtg gaa gat ggg aca cct tgt     723
ggt ccc caa atg atg tgc tta gaa cac agg tgt ctt cct gtg gct tct     771
ttc aac ttt agt act tgc ttg agc agt aaa gaa ggc act att tgc tca     819
gga aat gga gtt tgc agt aat gag ctg aag tgt gtg tgt aac aga cac     867
tgg ata ggt tct gat tgc aac act tac ttc cct cac aat gat gat gca     915
aag act ggt atc act ctg tct ggc aat ggt gtt gct ggc acc aat gga     963
tct tgt gac aaa act cac aca tgc cca ccg tgc cca gca cct gaa gcc
1011
gag ggc gcg ccg tca gtc ttc ctc ttc ccc cca aaa ccc aag gac acc
1059
ctc atg atc tcc cgg acc cct gag gtc aca tgc gtg gtg gtg gac gtg
1107
agc cac gaa gac cct gag gtc aag ttc aac tgg tac gtg gac ggc gtg
1155
gag gtg cat aat gcc aag aca aag ccg cgg gag gag cag tac aac agc
1203
acg tac cgg gtg gtc agc gtc ctc acc gtc ctg cac cag gac tgg ctg
1251
aat ggc aag gag tac aag tgc aag gtc tcc aac aaa gcc ctc cca gcc
1299
ccc atc gag aaa acc atc tcc aaa gcc aaa ggg cag ccc cga gaa cca
1347
cag gtg tac acc ctg ccc cca tcc cgg gat gag ctg acc aag aac cag
1395
gtc agc ctg acc tgc ctg gtc aaa ggc ttc tat ccc agc gac atc gcc
1443
gtg gag tgg gag agc aat ggg cag ccg gag aac aac tac aag acc acg
1491
cct ccc gtg ctg gac tcc gac ggc tcc ttc ttc ctc tac agc aag ctc
1539
acc gtg gac aag agc agg tgg cag cag ggg aac gtc ttc tca tgc tcc
1587
gtg atg cat gag gct ctg cac aac cac tac acg cag aag agc ctc tcc
1635
ctg tct ccg ggt aaa tga actagagcgg ccgctacaga t
1674

<210> 16
<211> 542
<212> PRT
<213> Artificial Sequence

<220>
<223> Description of Artificial Sequence: fusion
      polypeptide

<400> 16


<210> 17
<211> 1668
<212> DNA
<213> Artificial Sequence

<220>
<223> Description of Artificial Sequence: fusion
      polypeptide

<220>
<221> CDS
<222> (25)..(1647)

<400> 17
gtcgacccaa gctggctagc cacc atg gag aca gac aca ctc ctg cta tgg      51
                           Met Glu Thr Asp Thr Leu Leu Leu Trp
                             1               5

gta ctg ctg ctc tgg gtt cca ggt tcc act ggt act agt tgt gga aat      99
Val Leu Leu Leu Trp Val Pro Gly Ser Thr Gly Thr Ser Cys Gly Asn
 10                  15                  20                  25

gga tac gtc gaa gct ggg gag gag tgt gat tgt ggt ttt cat gtg gaa     147
Gly Tyr Val Glu Ala Gly Glu Glu Cys Asp Cys Gly Phe His Val Glu
                 30                  35                  40

tgc tat gga tta tgc tgt aag aaa tgt tcc ctc tcc aac ggg gct cac 195
Cys Tyr Gly Leu Cys Cys Lys Lys Cys Ser Leu Ser Asn Gly Ala His
            45                  50                  55

tgc agc gac ggg ccc tgc tgt aac aat acc tca tgt ctt ttt cag cca 243
Cys Ser Asp Gly Pro Cys Cys Asn Asn Thr Ser Cys Leu Phe Gln Pro
        60                  65                  70

cga ggg tat gaa tgc cgg gat gct gtg aac gag tgt gat att act gaa 291
Arg Gly Tyr Glu Cys Arg Asp Ala Val Asn Glu Cys Asp Ile Thr Glu
    75                  80                  85

tat tgt act gga gac tct ggt cag tgc cca cca aat ctt cat aag caa 339
Tyr Cys Thr Gly Asp Ser Gly Gln Cys Pro Pro Asn Leu His Lys Gln
90                  95                  100                 105

gac gga tat gca tgc aat caa aat cag ggc cgc tgc tac aat ggc gag 387
Asp Gly Tyr Ala Cys Asn Gln Asn Gln Gly Arg Cys Tyr Asn Gly Glu
                110                 115                 120

tgc aag gcc aga gac aac cag tgt cag tac atc tgg gga aca aag gct 435
Cys Lys Ala Arg Asp Asn Gln Cys Gln Tyr Ile Trp Gly Thr Lys Ala
            125                 130                 135

gca ggg tct gac aag ttc tgc tat gaa aag ctg aat aca gaa ggc act 483
Ala Gly Ser Asp Lys Phe Cys Tyr Glu Lys Leu Asn Thr Glu Gly Thr
        140                 145                 150

gag aag gga aac tgc ggg aag gat gga gac cgg tgg att cag tgc agc 531
Glu Lys Gly Asn Cys Gly Lys Asp Gly Asp Arg Trp Ile Gln Cys Ser
    155                 160                 165

aaa cat gat gtg ttc tgt gga ttc tta ctc tgt acc aat ctt act cga 579
Lys His Asp Val Phe Cys Gly Phe Leu Leu Cys Thr Asn Leu Thr Arg
170                 175                 180                 185

gct cca cgt att ggt caa ctt cag ggt gag atc att cca act tcc ttc 627
Ala Pro Arg Ile Gly Gln Leu Gln Gly Glu Ile Ile Pro Thr Ser Phe
                190                 195                 200

tac cat caa ggc cgg gtg att gac tgc agt ggt gcc cat gta gtt tta 675
Tyr His Gln Gly Arg Val Ile Asp Cys Ser Gly Ala His Val Val Leu
            205                 210                 215

gat gat gat acg gat gtg ggc tat gta gaa gat gga acg cca tgt ggc 723
Asp Asp Asp Thr Asp Val Gly Tyr Val Glu Asp Gly Thr Pro Cys Gly
        220                 225                 230

ccg tct atg atg tgt tta gat cgg aag tgc cta caa att caa gcc cta 771

Pro Ser Met Met Cys Leu Asp Arg Lys Cys Leu Gln Ile Gln Ala Leu
    235                 240                 245

aat atg agc agc tgt cca ctc gat tcc aag ggt aaa gtc tgt tcg ggc 819
Asn Met Ser Ser Cys Pro Leu Asp Ser Lys Gly Lys Val Cys Ser Gly
250                 255                 260                 265

cat ggg gtg tgt agt aat gaa gcc acc tgc att tgt gat ttc acc tgg 867
His Gly Val Cys Ser Asn Glu Ala Thr Cys Ile Cys Asp Phe Thr Trp
                270                 275                 280

gca ggg aca gat tgc agt atc cgg gat cca gtt agg aac ctt cac ccc     915
Ala Gly Thr Asp Cys Ser Ile Arg Asp Pro Val Arg Asn Leu His Pro
            285                 290                 295

ccc aag gat gaa gga ccc aag ggt cct agt gcc acc aat aga tct tgt     963
Pro Lys Asp Glu Gly Pro Lys Gly Pro Ser Ala Thr Asn Arg Ser Cys
        300                 305                 310

gac aaa act cac aca tgc cca ccg tgc cca gca cct gaa gcc gag ggc
1011
Asp Lys Thr His Thr Cys Pro Pro Cys Pro Ala Pro Glu Ala Glu Gly
    315                 320                 325

gcg ccg tca gtc ttc ctc ttc ccc cca aaa ccc aag gac acc ctc atg
1059
Ala Pro Ser Val Phe Leu Phe Pro Pro Lys Pro Lys Asp Thr Leu Met
330                 335                 340                 345

atc tcc cgg acc cct gag gtc aca tgc gtg gtg gtg gac gtg agc cac
1107
Ile Ser Arg Thr Pro Glu Val Thr Cys Val Val Val Asp Val Ser His
                350                 355                 360

gaa gac cct gag gtc aag ttc aac tgg tac gtg gac ggc gtg gag gtg
1155
Glu Asp Pro Glu Val Lys Phe Asn Trp Tyr Val Asp Gly Val Glu Val
            365                 370                 375

cat aat gcc aag aca aag ccg cgg gag gag cag tac aac agc acg tac
1203
His Asn Ala Lys Thr Lys Pro Arg Glu Glu Gln Tyr Asn Ser Thr Tyr
        380                 385                 390

cgg gtg gtc agc gtc ctc acc gtc ctg cac cag gac tgg ctg aat ggc
1251
Arg Val Val Ser Val Leu Thr Val Leu His Gln Asp Trp Leu Asn Gly
    395                 400                 405

aag gag tac aag tgc aag gtc tcc aac aaa gcc ctc cca gcc ccc atc
1299
Lys Glu Tyr Lys Cys Lys Val Ser Asn Lys Ala Leu Pro Ala Pro Ile
410                 415                 420                 425

gag aaa acc atc tcc aaa gcc aaa ggg cag ccc cga gaa cca cag gtg
1347
Glu Lys Thr Ile Ser Lys Ala Lys Gly Gln Pro Arg Glu Pro Gln Val
                430                 435                 440

tac acc ctg ccc cca tcc cgg gat gag ctg acc aag aac cag gtc agc
1395
Tyr Thr Leu Pro Pro Ser Arg Asp Glu Leu Thr Lys Asn Gln Val Ser
            445                 450                 455

ctg acc tgc ctg gtc aaa ggc ttc tat ccc agc gac atc gcc gtg gag
1443
Leu Thr Cys Leu Val Lys Gly Phe Tyr Pro Ser Asp Ile Ala Val Glu
        460                 465                 470

tgg gag agc aat ggg cag ccg gag aac aac tac aag acc acg cct ccc
1491
Trp Glu Ser Asn Gly Gln Pro Glu Asn Asn Tyr Lys Thr Thr Pro Pro

475                 480                 485

gtg ctg gac tcc gac ggc tcc ttc ttc ctc tac agc aag ctc acc gtg
1539
Val Leu Asp Ser Asp Gly Ser Phe Phe Leu Tyr Ser Lys Leu Thr Val
490                 495                 500                 505

gac aag agc agg tgg cag cag ggg aac gtc ttc tca tgc tcc gtg atg
1587
Asp Lys Ser Arg Trp Gln Gln Gly Asn Val Phe Ser Cys Ser Val Met
                510                 515                 520

cat gag gct ctg cac aac cac tac acg cag aag agc ctc tcc ctg tct
1635
His Glu Ala Leu His Asn His Tyr Thr Gln Lys Ser Leu Ser Leu Ser
            525                 530                 535

ccg ggt aaa tga actagagcgg ccgctacaga t
1668
Pro Gly Lys
        540


<210> 18
<211> 540
<212> PRT
<213> Artificial Sequence

<220>
<223> Description of Artificial Sequence: fusion
      polypeptide

<400> 18
Met Glu Thr Asp Thr Leu Leu Leu Trp Val Leu Leu Leu Trp Val Pro
  1               5                  10                  15
Gly Ser Thr Gly Thr Ser Cys Gly Asn Gly Tyr Val Glu Ala Gly Glu
             20                  25                  30
Glu Cys Asp Cys Gly Phe His Val Glu Cys Tyr Gly Leu Cys Cys Lys
         35                  40                  45
Lys Cys Ser Leu Ser Asn Gly Ala His Cys Ser Asp Gly Pro Cys Cys
     50                  55                  60
Asn Asn Thr Ser Cys Leu Phe Gln Pro Arg Gly Tyr Glu Cys Arg Asp
 65                  70                  75                  80
Ala Val Asn Glu Cys Asp Ile Thr Glu Tyr Cys Thr Gly Asp Ser Gly
                 85                  90                  95
Gln Cys Pro Pro Asn Leu His Lys Gln Asp Gly Tyr Ala Cys Asn Gln
            100                 105                 110
Asn Gln Gly Arg Cys Tyr Asn Gly Glu Cys Lys Ala Arg Asp Asn Gln
        115                 120                 125
Cys Gln Tyr Ile Trp Gly Thr Lys Ala Ala Gly Ser Asp Lys Phe Cys
    130                 135                 140
Tyr Glu Lys Leu Asn Thr Glu Gly Thr Glu Lys Gly Asn Cys Gly Lys
145                 150                 155                 160
Asp Gly Asp Arg Trp Ile Gln Cys Ser Lys His Asp Val Phe Cys Gly
                165                 170                 175
Phe Leu Leu Cys Thr Asn Leu Thr Arg Ala Pro Arg Ile Gly Gln Leu
            180                 185                 190
Gln Gly Glu Ile Ile Pro Thr Ser Phe Tyr His Gln Gly Arg Val Ile
        195                 200                 205
Asp Cys Ser Gly Ala His Val Val Leu Asp Asp Asp Thr Asp Val Gly
    210                 215                 220

Tyr Val Glu Asp Gly Thr Pro Cys Gly Pro Ser Met Met Cys Leu Asp
225                 230                 235                 240
Arg Lys Cys Leu Gln Ile Gln Ala Leu Asn Met Ser Ser Cys Pro Leu
                245                 250                 255

Asp Ser Lys Gly Lys Val Cys Ser Gly His Gly Val Cys Ser Asn Glu
            260                 265                 270
Ala Thr Cys Ile Cys Asp Phe Thr Trp Ala Gly Thr Asp Cys Ser Ile
        275                 280                 285
Arg Asp Pro Val Arg Asn Leu His Pro Pro Lys Asp Glu Gly Pro Lys
    290                 295                 300
Gly Pro Ser Ala Thr Asn Arg Ser Cys Asp Lys Thr His Thr Cys Pro
305                 310                 315                 320
Pro Cys Pro Ala Pro Glu Ala Glu Gly Ala Pro Ser Val Phe Leu Phe
                325                 330                 335
Pro Pro Lys Pro Lys Asp Thr Leu Met Ile Ser Arg Thr Pro Glu Val
            340                 345                 350
Thr Cys Val Val Val Asp Val Ser His Glu Asp Pro Glu Val Lys Phe
        355                 360                 365
Asn Trp Tyr Val Asp Gly Val Glu Val His Asn Ala Lys Thr Lys Pro
    370                 375                 380
Arg Glu Glu Gln Tyr Asn Ser Thr Tyr Arg Val Val Ser Val Leu Thr
385                 390                 395                 400
Val Leu His Gln Asp Trp Leu Asn Gly Lys Glu Tyr Lys Cys Lys Val
                405                 410                 415
Ser Asn Lys Ala Leu Pro Ala Pro Ile Glu Lys Thr Ile Ser Lys Ala
            420                 425                 430
Lys Gly Gln Pro Arg Glu Pro Gln Val Tyr Thr Leu Pro Pro Ser Arg
        435                 440                 445
Asp Glu Leu Thr Lys Asn Gln Val Ser Leu Thr Cys Leu Val Lys Gly
    450                 455                 460
Phe Tyr Pro Ser Asp Ile Ala Val Glu Trp Glu Ser Asn Gly Gln Pro
465                 470                 475                 480
Glu Asn Asn Tyr Lys Thr Thr Pro Pro Val Leu Asp Ser Asp Gly Ser
                485                 490                 495
Phe Phe Leu Tyr Ser Lys Leu Thr Val Asp Lys Ser Arg Trp Gln Gln
            500                 505                 510
Gly Asn Val Phe Ser Cys Ser Val Met His Glu Ala Leu His Asn His
        515                 520                 525
Tyr Thr Gln Lys Ser Leu Ser Leu Ser Pro Gly Lys
    530                 535                 540


<210> 19
<211> 3
<212> PRT
<213> Artificial Sequence

<220>
<223> Description of Artificial Sequence: Consensus
      binding motif

<400> 19
Arg Gly Asp
  1


<210> 20
<211> 67
<212> PRT
<213> Artificial Sequence

<220>
<223> Description of Artificial Sequence: consensus
      disintegrin domain

<220>
<221> VARIANT
<222> (5)..(9)
<223> 3-5 varying residues in a consensus sequence

<220>
<221> VARIANT
<222> (11)..(16)
<223> 3-6 varying residues in a consensus sequence

<220>
<221> VARIANT
<222> (19)..(22)
<223> 2-4 varying residues in a consensus sequence

<220>
<221> VARIANT
<222> (24)..(30)
<223> 7 varying residues in a consensus sequence

<220>
<221> VARIANT
<222> (32)..(37)
<223> 4-6 varying residues in a consensus sequence

<220>
<221> VARIANT
<222> (40)..(43)
<223> 2-4 varying residues in a consensus sequence

<220>
<221> VARIANT
<222> (45)..(52)
<223> 8 varying residues in a consensus sequence

<220>
<221> VARIANT
<222> (54)..(60)
<223> 5-7 varying residues in a consensus sequence

<220>
<221> VARIANT
<222> (62)..(66)
<223> 3-5 varying residues in a consensus sequence

<400> 20
Cys Asp Cys Gly Xaa Xaa Xaa Xaa Xaa Cys Xaa Xaa Xaa Xaa Xaa Xaa
1               5                   10                  15

Cys Cys Xaa Xaa Xaa Xaa Cys Xaa Xaa Xaa Xaa Xaa Xaa Xaa Cys Xaa
            20                  25                  30

Xaa Xaa Xaa Xaa Xaa Cys Cys Xaa Xaa Xaa Xaa Cys Xaa Xaa Xaa Xaa
        35                  40                  45

Xaa Xaa Xaa Xaa Cys Xaa Xaa Xaa Xaa Xaa Xaa Xaa Cys Xaa Xaa Xaa

50                  55                  60

Xaa Xaa Cys
65

<210> 21
<211> 1725
<212> DNA
<213> Artificial Sequence

<220>
<223> Description of Artificial Sequence: fusion
      polypeptide

<220>
<221> CDS
<222> (118)..(1704)

<400> 21
gggttttccc agtcacgacg ttgtaaaacg acggccagtg aattgtaata cgactcacta 60

tagggcgaat tgggtaccgg gccccccctc gaggtcgacc caagctggct agccacc    117

atg gag aca gac aca ctc ctg cta tgg gta ctg ctg ctc tgg gtt cca    165
Met Glu Thr Asp Thr Leu Leu Leu Trp Val Leu Leu Leu Trp Val Pro
  1               5                  10                  15

ggt tcc act ggt act agt tgt ggg aat ggt gtg gtt gaa gaa gga gaa    213
Gly Ser Thr Gly Thr Ser Cys Gly Asn Gly Val Val Glu Glu Gly Glu
             20                  25                  30

gag tgt gac tgt gga cct tta aag cat tgt gca aaa gat ccc tgc tgt    261
Glu Cys Asp Cys Gly Pro Leu Lys His Cys Ala Lys Asp Pro Cys Cys
         35                  40                  45

ctg tca aat tgc act ctg act gat ggt tct act tgt gct ttt ggg ctt    309
Leu Ser Asn Cys Thr Leu Thr Asp Gly Ser Thr Cys Ala Phe Gly Leu
     50                  55                  60

tgt tgc aaa gac tgc aag ttc cta cca tca ggg aaa gtg tgt aga aag    357
Cys Cys Lys Asp Cys Lys Phe Leu Pro Ser Gly Lys Val Cys Arg Lys
 65                  70                  75                  80

gag gtc aat gaa tgt gat ctt cca gag tgg tgc aat ggt act tcc cat    405
Glu Val Asn Glu Cys Asp Leu Pro Glu Trp Cys Asn Gly Thr Ser His
                 85                  90                  95

aag tgc cca gat gac ttt tat gtg gaa gat gga att ccc tgt aag gag    453
Lys Cys Pro Asp Asp Phe Tyr Val Glu Asp Gly Ile Pro Cys Lys Glu
            100                 105                 110

agg ggc tac tgc tat gaa aag agc tgt cat gac cgc aat gaa cag tgt    501
Arg Gly Tyr Cys Tyr Glu Lys Ser Cys His Asp Arg Asn Glu Gln Cys
        115                 120                 125

agg agg att ttt ggt gca ggc gca aat act gca agt gag act tgc tac    549
Arg Arg Ile Phe Gly Ala Gly Ala Asn Thr Ala Ser Glu Thr Cys Tyr
    130                 135                 140

aaa gaa ttg aac acc tta ggt gac cgt gtt ggt cac tgt ggt atc aaa    597

Lys Glu Leu Asn Thr Leu Gly Asp Arg Val Gly His Cys Gly Ile Lys
145                 150                 155                 160

aat gct aca tat ata aag tgt aat atc tca gat gtc cag tgt gga aga     645
Asn Ala Thr Tyr Ile Lys Cys Asn Ile Ser Asp Val Gln Cys Gly Arg
                165                 170                 175

att cag tgt gag aat gtg aca gaa att ccc aat atg agt gat cat act     693
Ile Gln Cys Glu Asn Val Thr Glu Ile Pro Asn Met Ser Asp His Thr
            180                 185                 190

act gtg cat tgg gct cgc ttc aat gac ata atg tgc tgg agt act gat     741
Thr Val His Trp Ala Arg Phe Asn Asp Ile Met Cys Trp Ser Thr Asp
        195                 200                 205

tac cat ttg ggg atg aag gga cct gat att ggt gaa gtg aaa gat gga     789
Tyr His Leu Gly Met Lys Gly Pro Asp Ile Gly Glu Val Lys Asp Gly
    210                 215                 220

aca gag tgt ggg ata gat cat ata tgc atc cac agg cac tgt gtc cat     837
Thr Glu Cys Gly Ile Asp His Ile Cys Ile His Arg His Cys Val His
225                 230                 235                 240

ata acc atc ttg aat agt aat tgc tca cct gca ttt tgt aac aag agg     885
Ile Thr Ile Leu Asn Ser Asn Cys Ser Pro Ala Phe Cys Asn Lys Arg
                245                 250                 255

ggc atc tgc aac aat aaa cat cac tgc cat tgc aat tat ctg tgg gac     933

Gly Ile Cys Asn Asn Lys His His Cys His Cys Asn Tyr Leu Trp Asp
            260                 265                 270

cct ccc aac tgc ctg ata aaa ggc tat gga ggt agt gtt gac agt ggc     981
Pro Pro Asn Cys Leu Ile Lys Gly Tyr Gly Gly Ser Val Asp Ser Gly
        275                 280                 285

cca ccc cct aag aga aag aag aaa aag aag aga tct tgt gac aaa act
1029
Pro Pro Pro Lys Arg Lys Lys Lys Lys Lys Arg Ser Cys Asp Lys Thr
    290                 295                 300

cac aca tgc cca ccg tgc cca gca cct gaa gcc gag ggc gcg ccg tca
1077
His Thr Cys Pro Pro Cys Pro Ala Pro Glu Ala Glu Gly Ala Pro Ser
305                 310                 315                 320

gtc ttc ctc ttc ccc cca aaa ccc aag gac acc ctc atg atc tcc cgg
1125
Val Phe Leu Phe Pro Pro Lys Pro Lys Asp Thr Leu Met Ile Ser Arg
                325                 330                 335

acc cct gag gtc aca tgc gtg gtg gtg gac gtg agc cac gaa gac cct
1173
Thr Pro Glu Val Thr Cys Val Val Val Asp Val Ser His Glu Asp Pro
            340                 345                 350

gag gtc aag ttc aac tgg tac gtg gac ggc gtg gag gtg cat aat gcc
1221
Glu Val Lys Phe Asn Trp Tyr Val Asp Gly Val Glu Val His Asn Ala
        355                 360                 365

aag aca aag ccg cgg gag gag cag tac aac agc acg tac cgg gtg gtc
1269
Lys Thr Lys Pro Arg Glu Glu Gln Tyr Asn Ser Thr Tyr Arg Val Val
    370                 375                 380

agc gtc ctc acc gtc ctg cac cag gac tgg ctg aat ggc aag gag tac
1317
Ser Val Leu Thr Val Leu His Gln Asp Trp Leu Asn Gly Lys Glu Tyr
385                 390                 395                 400

aag tgc aag gtc tcc aac aaa gcc ctc cca gcc ccc atc gag aaa acc
1365
Lys Cys Lys Val Ser Asn Lys Ala Leu Pro Ala Pro Ile Glu Lys Thr
                405                 410                 415

atc tcc aaa gcc aaa ggg cag ccc cga gaa cca cag gtg tac acc ctg
1413
Ile Ser Lys Ala Lys Gly Gln Pro Arg Glu Pro Gln Val Tyr Thr Leu
            420                 425                 430

ccc cca tcc cgg gat gag ctg acc aag aac cag gtc agc ctg acc tgc
1461
Pro Pro Ser Arg Asp Glu Leu Thr Lys Asn Gln Val Ser Leu Thr Cys
        435                 440                 445

ctg gtc aaa ggc ttc tat ccc agc gac atc gcc gtg gag tgg gag agc
1509
Leu Val Lys Gly Phe Tyr Pro Ser Asp Ile Ala Val Glu Trp Glu Ser
    450                 455                 460

aat ggg cag ccg gag aac aac tac aag acc acg cct ccc gtg ctg gac
1557
Asn Gly Gln Pro Glu Asn Asn Tyr Lys Thr Thr Pro Pro Val Leu Asp
465                 470                 475                 480

tcc gac ggc tcc ttc ttc ctc tac agc aag ctc acc gtg gac aag agc
1605
Ser Asp Gly Ser Phe Phe Leu Tyr Ser Lys Leu Thr Val Asp Lys Ser
                485                 490                 495

agg tgg cag cag ggg aac gtc ttc tca tgc tcc gtg atg cat gag gct
1653
Arg Trp Gln Gln Gly Asn Val Phe Ser Cys Ser Val Met His Glu Ala
            500                 505                 510

ctg cac aac cac tac acg cag aag agc ctc tcc ctg tct ccg ggt aaa
1701
Leu His Asn His Tyr Thr Gln Lys Ser Leu Ser Leu Ser Pro Gly Lys
        515                 520                 525

tga actagagcgg ccgctacaga t
1725


<210> 22
<211> 528
<212> PRT
<213> Artificial Sequence

<220>
<223> Description of Artificial Sequence: fusion

polypeptide

<400> 22
Met Glu Thr Asp Thr Leu Leu Leu Trp Val Leu Leu Leu Trp Val Pro
1               5                   10                  15
Gly Ser Thr Gly Thr Ser Cys Gly Asn Gly Val Val Glu Glu Gly Glu
            20                  25                  30
Glu Cys Asp Cys Gly Pro Leu Lys His Cys Ala Lys Asp Pro Cys Cys
        35                  40                  45
Leu Ser Asn Cys Thr Leu Thr Asp Gly Ser Thr Cys Ala Phe Gly Leu
    50                  55                  60
Cys Cys Lys Asp Cys Lys Phe Leu Pro Ser Gly Lys Val Cys Arg Lys
65                  70                  75                  80
Glu Val Asn Glu Cys Asp Leu Pro Glu Trp Cys Asn Gly Thr Ser His
                85                  90                  95
Lys Cys Pro Asp Asp Phe Tyr Val Glu Asp Gly Ile Pro Cys Lys Glu
            100                 105                 110
Arg Gly Tyr Cys Tyr Glu Lys Ser Cys His Asp Arg Asn Glu Gln Cys
        115                 120                 125
Arg Arg Ile Phe Gly Ala Gly Ala Asn Thr Ala Ser Glu Thr Cys Tyr
    130                 135                 140
Lys Glu Leu Asn Thr Leu Gly Asp Arg Val Gly His Cys Gly Ile Lys
145                 150                 155                 160
Asn Ala Thr Tyr Ile Lys Cys Asn Ile Ser Asp Val Gln Cys Gly Arg
                165                 170                 175
Ile Gln Cys Glu Asn Val Thr Glu Ile Pro Asn Met Ser Asp His Thr
            180                 185                 190
Thr Val His Trp Ala Arg Phe Asn Asp Ile Met Cys Trp Ser Thr Asp
        195                 200                 205
Tyr His Leu Gly Met Lys Gly Pro Asp Ile Gly Glu Val Lys Asp Gly
    210                 215                 220
Thr Glu Cys Gly Ile Asp His Ile Cys Ile His Arg His Cys Val His
225                 230                 235                 240
Ile Thr Ile Leu Asn Ser Asn Cys Ser Pro Ala Phe Cys Asn Lys Arg
                245                 250                 255
Gly Ile Cys Asn Asn Lys His His Cys His Cys Asn Tyr Leu Trp Asp
            260                 265                 270
Pro Pro Asn Cys Leu Ile Lys Gly Tyr Gly Gly Ser Val Asp Ser Gly
        275                 280                 285
Pro Pro Pro Lys Arg Lys Lys Lys Lys Lys Arg Ser Cys Asp Lys Thr
    290                 295                 300
His Thr Cys Pro Pro Cys Pro Ala Pro Glu Ala Glu Gly Ala Pro Ser
305                 310                 315                 320
Val Phe Leu Phe Pro Pro Lys Pro Lys Asp Thr Leu Met Ile Ser Arg
                325                 330                 335
Thr Pro Glu Val Thr Cys Val Val Val Asp Val Ser His Glu Asp Pro
            340                 345                 350
Glu Val Lys Phe Asn Trp Tyr Val Asp Gly Val Glu Val His Asn Ala
        355                 360                 365
Lys Thr Lys Pro Arg Glu Glu Gln Tyr Asn Ser Thr Tyr Arg Val Val
    370                 375                 380
Ser Val Leu Thr Val Leu His Gln Asp Trp Leu Asn Gly Lys Glu Tyr
385                 390                 395                 400
Lys Cys Lys Val Ser Asn Lys Ala Leu Pro Ala Pro Ile Glu Lys Thr
                405                 410                 415
Ile Ser Lys Ala Lys Gly Gln Pro Arg Glu Pro Gln Val Tyr Thr Leu
            420                 425                 430
Pro Pro Ser Arg Asp Glu Leu Thr Lys Asn Gln Val Ser Leu Thr Cys
        435                 440                 445
Leu Val Lys Gly Phe Tyr Pro Ser Asp Ile Ala Val Glu Trp Glu Ser
    450                 455                 460

Asn Gly Gln Pro Glu Asn Asn Tyr Lys Thr Thr Pro Pro Val Leu Asp
465                 470                 475                 480
Ser Asp Gly Ser Phe Phe Leu Tyr Ser Lys Leu Thr Val Asp Lys Ser
                485                 490                 495
Arg Trp Gln Gln Gly Asn Val Phe Ser Cys Ser Val Met His Glu Ala
            500                 505                 510
Leu His Asn His Tyr Thr Gln Lys Ser Leu Ser Leu Ser Pro Gly Lys
        515                 520                 525